# EUROPEAN PATENT SPECIFICATION

(11) **EP 3 256 607 B1**
(45) Date of publication and mention of the grant of the patent: **20.01.2021**
(21) Application number: 16750045.3
(22) Date of filing: 14.02.2016
(51) Int. Cl.: C12Q 1/6851, C12Q 1/6806

(54) **METHODS FOR HIGHLY PARALLEL AND ACCURATE DETECTION OF NUCLEIC ACIDS**
VERFAHREN ZUR HOCHPARALLELEN UND GENAUEN DETEKTION VON NUKLEINSÄUREN
PROCÉDÉS DE DÉTECTION D'ACIDES NUCLÉIQUES HAUTEMENT PARALLÈLE ET PRÉCISE

(30) Priority: 13.02.2015 US 201562116302 P; 20.03.2015 US 201562135923 P
(43) Date of publication of application: 20.12.2017
(73) Proprietor: Patel, Abhijit Ajit, Madison, Connecticut 06443 (US)
(72) Inventor: Patel, Abhijit Ajit, Madison, Connecticut 06443 (US)
(74) Representative: McQueen, Andrew Peter
(86) International application number: PCT/US2016/017920
(87) International publication number: WO 2016/131030

(56) References cited:
- WO-A1-2012/112804
- WO-A1-2013/036929
- WO-A1-2014/152155
- WO-A1-2016/138148
- WO-A2-2012/149042
- US-A1- 2004 067 492
- US-A1- 2006 019 258
- US-B1- 7 270 983
- US-B2- 8 088 580
- US-B2- 8 435 742

## Description

This invention was made with government support under TR000140 and TR000142, awarded by the National Institutes of Health. The government has certain rights in the invention.

### TECHNICAL FIELD

The present document is related to identification and quantitation of nucleic acids in solutions.

### BACKGROUND

Many applications in biomedical research and clinical medicine rely on accurate detection and quantitation of nucleic acids. Some applications rely on measurement of ribonucleic acid ("RNA") levels to provide information about gene activity or gene expression. Other applications rely on measurement of variant deoxyribonucleic acid ("DNA") or RNA sequences that indicate the presence genomic alterations such as point mutations, insertions, deletions, translocations, polymorphisms, or copy-number variations. Several challenges exist in the measurement of nucleic acids, from both technical and practical standpoints. Often, measurements must be made from large numbers of samples. Additionally, if very few copies of a particular nucleic acid sequence of interest are present in a limited sample containing a complex mixture of nucleic acid molecules, it can be challenging to reliably identify and quantify the low-abundance variants.

Analysis of gene expression within diverse clinical and research specimens underpins our understanding of cellular physiology and informs our approaches to disease. Discerning meaningful gene expression patterns within complex biological systems usually involves statistical comparisons in two dimensions: across multiple ribonucleic acids and across multiple samples. While mature technologies exist for highly parallel analysis in the first RNA dimension, throughput efficiency remains limited in a second, sample dimension. A genome-wide picture of RNA expression can be obtained using techniques such as transcriptome sequencing ("RNA-Seq") or microarrays. But because these approaches involve separate multi-step processing of each sample s, they are not designed to facilitate large-scale sample multiplexing. Furthermore, while the falling cost of RNA-Seq has fueled its widespread use, there remains a trade-off between sequence depth and per-sample cost, which can limit the sensitivity for measuring rare transcripts.

Evaluation of targeted RNAs across larger sample sets is often performed using quantitative reverse-transcription polymerase chain reaction ("qRT-PCR"), after a subset of differentially expressed RNAs has been identified by global profiling methods. The accuracy, sensitivity, and broad dynamic range of qRT-PCR make it the method of choice for validation and further testing of such transcripts. However, because real-time monitoring of fluorescence needs to be performed on separate reaction volumes, applying a multi-gene qRT-PCR assay to a large number of samples can be costly and laborious. Although throughput can be improved via automation or microfluidics, separate exponential amplifications remain prone to inter-sample variability.

It can also be challenging to detect and quantify low-abundance variant nucleic acid sequences from complex mixtures of nucleic acid molecules. Achieving high analytical sensitivity for detection of rare variant sequences can be especially challenging in situations where the amount of DNA or RNA in a given sample is limited. An application of such a method is to detect small amounts of tumor-derived DNA or RNA molecules in the blood of individuals that have cancer. It is known that fragmented molecules of DNA and RNA are released into the bloodstream from dying cancer cells in patients with various types of malignancies. Such circulating tumor-derived nucleic acids are showing excellent promise as non-invasive cancer biomarkers. In the bloodstream, tumor-derived nucleic acids can be distinguished from normal background DNA or RNA based on the presence of tumor-specific mutations. However, such mutant nucleic acid copies are usually present in small amounts in a background of relatively abundant normal (wild-type) molecules. Often the mutant tumor-derived copies comprise less than 1% of the total DNA or RNA in plasma, and sometimes the abundance can be as low as 0.01% or lower. Thus, an assay with extremely high analytical sensitivity is involved in detecting such low-abundance DNA or RNA.

The patents WO2012112804A1 and WO 2013036929A1 are considered to describe background art which can be regarded as useful to understand the invention.

The patent WO2012112804A1 provides barcode libraries and methods of making and using them including obtaining a plurality of nucleic acid constructs in which each construct comprises a unique N-mer and a functional N-mer and segregating the constructs into a fluid compartments such that each compartment contains one or more copies of a unique construct. The patent WO 2013036929A1 describes a method that comprises providing at least one sample comprising one or more target polynucleotides; partitioning the sample into a finite plurality of partitions; subjecting the partition to polynucleotide fragmentation thereby generating a set of fragments; obtaining partial o complete sequence information from at least a subset of the fragments and relating the sequence information from the plurality of fragments into a subset of the one o more target polynucleotides.

### SUMMARY

The current document is directed to the method of the invention as defined in the enclosed claims. The current document is also directed to methods and compositions that enable quantitation of a broad panel of microRNAs ("miRNAs"), messenger RNAs ("mRNAs"), and other classes of RNAs simultaneously and in a highly parallel manner from many samples. These methods use far less sequence depth than existing digital profiling approaches. In one implementation, quantitative tags are assigned during reverse-transcription to permit up-front sample pooling before competitive amplification and deep sequencing. This approach is designed to bring large-scale gene expression studies within more practical reach.

The current document is also directed to methods and compositions that enable quantitation of low-abundance variant nucleic acid sequences from a complex mixture of nucleic acid molecules.

### BRIEF DESCRIPTION OF THE DRAWINGS

Figure 1 is a schematic description of the disclosed RNA profiling method.
Figure 2 provides data supporting the accuracy of multiplexed RNA quantitation using the disclosed RNA profiling method.
Figure 3 shows data to validate the quantitative performance of the method with RNA from human tissues and reference samples.
Figure 4 shows results of high-throughput measurements of radiation-induced gene expression changes in human blood.
Figure 5 provides data to compare several miRNA profiling platforms.
Figure 6 shows results of absolute quantitation of miRNAs in human tissues.
Figure 7 shows a schematic of an RNAse H2-activatable primer that is designed to resist digestion of its terminal blocking groups by the 3' to 5' exonuclease activity of proofreading polymerases.
Figure 8 provides a schematic description of Lineage-Traced PCR.
Figure 9 shows results of lineage-traced PCR experiments.
Figure 10 shows an example of how heat-releasable primers containing bead-specific barcodes can be produced on microbeads.
Figure 11 shows a method for producing temporarily immobilized oligonucleotides that can be released by heat-denaturation.
Figure 12 shows an in-solution method for delivering clonally tagged oligonucleotides into micro-compartments, which can function as primers to add compartment-specific tags to PCR products that are co-amplified with the same reaction volume.
Figure 13 shows an example of how different targets might be randomly compartmentalized within droplets or micro-wells for PCR amplification.
Figure 14 shows an example of the contents of a single reaction compartment (such as a micro-well or a droplet).
Figure 15 A and B show two example scenarios of lineage-traced PCR being carried out within a micro-compartment containing a single microbead carrying barcoded primers.
Figure 16 A and B show two additional example scenarios of lineage-traced PCR being carried out within a micro-compartment containing a single microbead carrying barcoded primers.
Figure 17 illustrates how analysis of lineage-traced PCR within micro-compartments would be performed if there were two (or more) differently barcoded primers in a given compartment.

### DETAILED DESCRIPTION

The current document is also directed to methods and compositions that enable quantitation of a broad panel of microRNAs ("miRNAs"), messenger RNAs ("mRNAs"), and other classes of RNAs simultaneously and in a highly parallel manner from many samples. These methods use far less sequence depth than existing digital profiling approaches. In one implementation, quantitative tags are assigned during reverse-transcription to permit up-front sample pooling before competitive amplification and deep sequencing. This approach is designed to bring large-scale gene expression studies within more practical reach.

The current document is also directed to compositions and methods relating to next-generation sequencing and medical diagnostics. Methods include identifying and quantifying nucleic acid variants, particularly those available in low abundance or those obscured by an abundance of wild-type sequences. The current document is also directed to methods related to identifying and quantifying specific sequences from a plurality of sequences amid a plurality of samples. The current document is also directed to detecting and distinguishing true nucleic acid variants from polymerase misincorporation errors, sequencer errors, and sample misclassification errors. In one implementation, methods include early attachment of barcodes and molecular lineage tags (MLTs) to targeted nucleic acids within a sample. Methods also include use of nested pairs of 3'-blocked primers that become unblocked upon highly specific hybridization to target DNA sequences, enabling assignment of MLTs while minimizing spurious amplification products during the polymerase chain reaction (PCR). Methods include raising the annealing temperature after the first few cycles of PCR to avoid participation of MLT-containing primers in later cycles of the reaction. Methods also include clonal overlapping paired-end sequencing to achieve sequence redundancy. Methods also include dividing of PCR amplifications into many small reaction compartments (such as aqueous droplets in oil or microscopic reaction volumes within a microfluidic device) to enable tracking of molecular lineage. Additional methods include amplification and tagging of both strands of a double-stranded DNA fragment within a microscopic reaction volume to improve analytical sensitivity by allowing mutations to be confirmed on both strands of a DNA duplex. Methods also include introduction of multiple copies of clonally tagged oligonucleotides into many small reaction volumes (e.g. micro-compartments) to facilitate compartment-specific tagging of the nucleic acid contents within the reaction volume. In one implementation, such clonally tagged oligonucleotides can be introduced to the compartments without needing to be attached to a surface such as a micro-bead or the compartment walls.

In one implementation, a method includes measuring nucleic acid variants by tagging and amplifying low abundance template nucleic acids in a multiplexed PCR. Low abundance template nucleic acids may be fetal DNA in the maternal circulation, circulating tumor DNA (ctDNA), circulating tumor RNA, exosome-derived RNA, viral RNA, viral DNA, DNA from a transplanted organ, or bacterial DNA. A multiplex PCR may include gene specific primers for a mutation prone genomic region. In one implementation, a mutation prone region may be within a gene that is altered in association with cancer.

In one implementation, primers comprise a barcode and/or a molecular lineage tag (MLT). In one implementation, a MLT can be 2-10 nucleotides. In another implementation, a MLT can be 6, 7, or 8 nucleotides. In one implementation, a barcode can identify the sample of origin of the template nucleic acid. In one implementation, a primer extension reaction employs targeted early barcoding. In targeted early barcoding, a plurality of different primers specific for different nucleic acid regions all have an identical barcode. An identical barcode identifies the nucleic acids from a particular sample. In one implementation, primers used for targeted early barcoding are produced by combining a unique barcode-containing oligonucleotide segment with a uniform mixture of gene-specific primer segments in a modular fashion.

In one implementation, disclosed assays can be used for clinical purposes. In one implementation, nucleic acid variants within blood can be identified and measured before and after treatment. In an example of cancer, a nucleic acid variant (e.g., cancer-related mutation) can be identified and/or measured prior to treatment (e.g., chemotherapy, radiation therapy, surgery, biologic therapy, combinations thereof). Then after treatment, the same nucleic acid variant can be identified or measured. After treatment, a quantitative change in the nucleic acid variant can indicate that the therapy was successful.

### Explanation of the Phrase "Molecular Lineage Tag" ("MLT")

The phrase "molecular lineage tag" ("MLT") is used to refer to a stretch of sequence that is contained within a synthetic oligonucleotide (e.g. a primer) and is used to assign diverse sequence tags to copies of template nucleic acid molecules. Assignment of MLTs enables the lineage of copied (or amplified) DNA sequences to be traced to early copies made from template nucleic acid molecules during the first few cycles of PCR. A molecular lineage tag can contain degenerate and/or predefined DNA sequences, although a diverse population of tags is most easily achieved by incorporating several degenerate positions. A molecular lineage tag is designed to have between two and 14 degenerate base positions, but preferably has between six and eight base positions. The bases need not be consecutive, and can be separated by constant sequences. The number of possible MLT sequences that can be generated in a population of oligonucleotide molecules is generally determined by the length of the MLT sequence and the number of possible bases at each degenerate position. For example, if an MLT is eight bases long, and has an approximately equal probability of having A, C, G, or T at each position, then the number of possible sequences is 4^8 = 65,536. MLTs need not have sufficient diversity to ensure assignment of a completely unique sequence tag to each copied template molecule, but rather there should be a low probability of assigning any given MLT sequence to a particular molecule. The greater the number of possible MLT sequences, the lower the probability of any particular sequence being assigned to a given template molecule. When many template molecules are copied and tagged, it is possible that the same MLT sequence might be assigned to more than one template molecule. MLT sequences are used to track the lineage of molecules from initial copying through amplification, processing and sequencing. They can be used to distinguish sequences that arise from polymerase misincorporations or sequencer errors from sequences that are derived from true mutant template molecules. MLTs can also be used to identify when amplified PCR products were copied from a single DNA strand or more than one DNA strand (e.g. when a single copy of a template nucleic acid fragment is amplified within a small reaction compartment). MLTs can also be used to distinguish sequences that have the wrong barcode assignment as a result of cross-over of barcodes during pooled amplification.

The phrase "molecular lineage tagging" refers to the process of assigning molecular lineage tags to nucleic acid templates molecules. MLTs can be incorporated within primers, and can be attached to copies made from targeted template nucleic acid fragments by specific extension of primers on the templates.

### Methods

### High-throughput RNA quantitation:

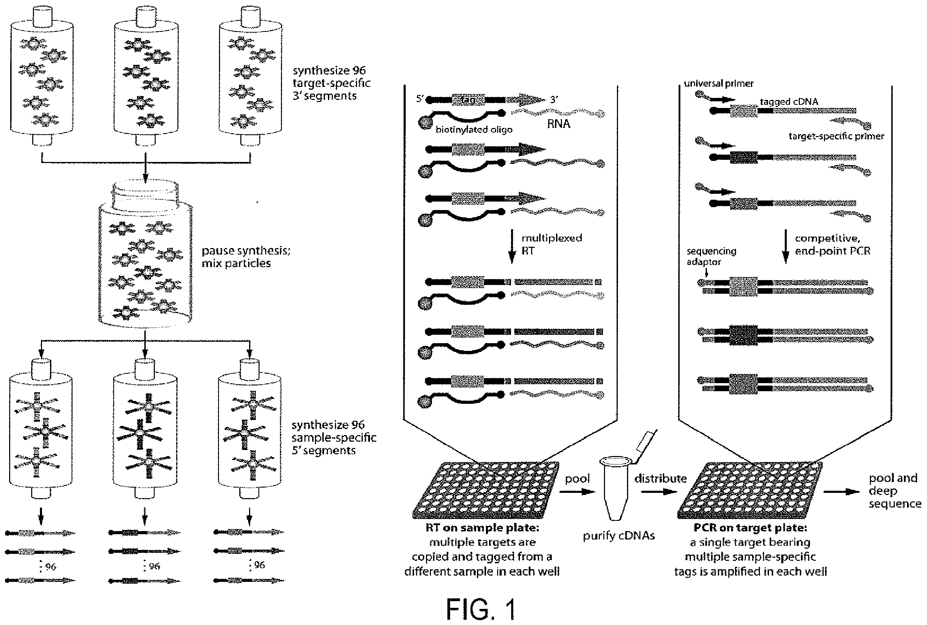

A RNA quantitation strategy is described that retains the quantification advantages of qRT-PCR while leveraging the simplicity, scalability, and uniformity of a single reaction involving pooled samples that is afforded by using a sequencing-based readout. Figure 1 is a schematic description of the disclosed RNA profiling method. The example depicts measurement of 96 miRNAs from 96 samples. Figure 1(A) shows that modular RT primer mixes are synthesized in two stages: 96 partially synthesized 3' primer segments containing target-specific sequences are pooled prior to redistribution for addition of 96 5' tag segments that will be used as sample markers. The 96 resulting primer mixes each have distinct tags. Because the second stage of synthesis begins with the same uniform mixture of 3' segments in each column, the final primer mixes all share similar ratios of target-specific sequences. Figure 1(B) shows that each sample first undergoes multiplexed reverse-transcription (RT) using a sample-specific modular primer mix to assign the sample-specific counting tags to cDNAs in proportion to target RNA abundance. Tagged cDNAs from all samples are combined into a single volume and are purified by in-solution hybrid capture using biotin-labeled oligonucleotides complementary to primer-extended sequences. Pooled cDNAs bearing tags from multiple samples are then co-amplified by competitive, single-plex PCRs of each target taken to plateau phase. Counting of tag/target combinations from deep sequenced amplicons reveals the relative abundance of RNAs across all samples.

In certain implementations, the method is capable of quantifying microRNAs (miRNAs), messenger RNAs (mRNAs), long non-coding RNAs (lncRNAs), or other RNA classes. The method demands far less mean sequence depth per base than other targeted or whole-transcriptome sequencing methods because separate end-point PCRs serve to roughly equalize total copies of low- and high-abundance RNA species. Thus rare transcripts can be adequately sampled without having to oversample abundant ones, yielding a broad dynamic range while maximizing sequence economy. As shown in Table 1, below, the lowest output mode of an Ion Torrent personal bench-top sequencer (fewer than 1 million reads) can be used to rapidly and inexpensively quantify 96 RNAs from 96 samples, providing data equivalent to 9,216 individual qRT-PCR assays. Analysis of even larger sample sets would further underscore the simplicity of this approach compared to qRT-PCR because the number of reaction tubes scales as the sum - not the product - of the number of RNAs and number of samples being evaluated.

**Table 1. Sequence depth for tested samples.**

| **RNA source** | **Target type** | **Sequencing chip(s) used** | **Filtered reads** | **Samples tested x (replicates)** | **Targeted RNAs** | **Avg. reads per target-sample bin per replicate** |
|---|---|---|---|---|---|---|
| Synthetic (Fig. 2, a and c) | miR NA | 318 chip x 2 | 4.53 M + 2.42 M | 96 x (2) | 96 | 377 |
| Synthetic (Fig. 2, b and d) | miR NA | 314 chip x 2 | 411 K + 419 K | 96 x (2) | 96 | 45 |
| Normal human tissues (Fig. 3, a and b) | miR NA | 314 chip x 1 | 446 K | 20 x (3) | 96 | 77 |
| Normal human tissues plus synthetic standard sample (Fig. 6) | miR NA | 314 chip x 1 | 387 K | 21 x (3) | 96 | 64 |
| MAQC reference samples (Fig. 3, c and d) | mR NA | 314 chip x 0.5 (shared with other samples) | -217 K | 4 x (4) | 30 targets + 4 ref. regions (in 2 ref. genes) | 399 |
| Irradiated human blood (Fig. 4) | mR NA | 314 chip x 2 | 318 K + 326 K | 108 x (2) | 23 targets + 2 ref. genes | 119 |

In one implementation, the method enables up-front sample parallelization, which confers several advantages over approaches that combine samples just prior to sequencing. Workflow is greatly simplified, obviating the need for microfluidic devices or automation. Pooled processing at all post-RT steps is expected to reduce quantitative variability across samples. By carrying PCR of each target to completion, sequence depth gets evenly distributed across all targets rather than being mostly consumed by abundant transcripts. Thus, per-sample cost, which is tied to sequence depth, is minimized while preserving ample depth to accurately quantify inter-sample differences among low-abundance transcripts.

The method differs from existing targeted sequencing approaches because it takes advantage of early sample pooling, uses far less sequence depth, and is able to target short miRNAs. It is also better suited for discrimination of sequence variants (particularly for longer mRNA targets) than qRT-PCR or most hybridization-based approaches. The method should be broadly accessible to most laboratories because next-generation sequencers are more commonly available in institutional core facilities than many of the specialized instruments used by other microfluidic or direct molecular counting technologies. The method is also readily adaptable to different sequencing platforms, it can be extended to analyze various functional RNA classes, and it uses minimal computational infrastructure and expertise.

### Quantification of low-abundance nucleic acid variants:

Methods and compositions are disclosed that identify and quantify nucleic acid sequence variants. Methods are disclosed that identify and quantify low-abundance sequence variants from complex mixtures of DNA or RNA. The methods can measure small amounts of tumor-derived DNA that can be found in the circulation of patients with various types of cancer.

Assessment of rare variant DNA sequences is important in many areas of biology and medicine. Small amounts of fetal DNA can be found in the circulation of pregnant women. One implementation includes analyzing rare fetal DNA that can be used to assess disease-associated genetic features or the sex of the fetus. An organ that is undergoing rejection by the recipient can release small amounts of DNA into the blood, and this donor-derived DNA can be distinguished based on genetic differences between the donor and the recipient. One implementation includes measuring donor-derived DNA to provide information about organ rejection and efficacy of treatment. In another implementation, nucleic acids can be detected from an infectious agent (e.g., bacteria, virus, fungus, parasite, etc.) in a patient sample. Genetic information about variations in pathogen-derived nucleic acids can help to better characterize the infection and to guide treatment decisions. For instance, detection of antibiotic resistance genes in the bacterial genome infecting a patient can direct antibiotic treatments.

Detection and measurement of low-abundance mutations has many important applications in the field of oncology. Tumors are known to acquire somatic mutations, some of which promote the unregulated proliferation of cancer cells. Identifying and quantifying such mutations has become a key diagnostic goal in the field of oncology. Companion diagnostics have become an important tool in identifying the mutational cause of cancer and then administering effective therapy for that particular mutation. Furthermore, some tumors acquire new mutations that confer resistance to targeted therapies. Thus, accurate determination of a tumor's mutation status can be a critical factor in determining the appropriateness of particular therapies for a given patient. However, detecting tumor-specific somatic mutations can be difficult, especially if tumor tissue obtained from a biopsy or a resection specimen has few tumor cells in a large background of stromal cells. Tumor-derived mutant DNA can be even more challenging to measure when it is found in very small amounts in blood, sputum, urine, stool, pleural fluid, or other biological samples.

Tumor-derived DNA is released into the bloodstream from dying cancer cells in patients with various types of malignancies. Detection of circulating tumor DNA (ctDNA) has several applications including, but not limited to, detecting presence of a malignancy, informing a prognosis, assessing treatment efficacy, tracking changes in tumor mutation status, and monitoring for disease recurrence or progression. Since unique somatic mutations can be used to distinguish tumor-derived DNA from normal background DNA in plasma, such circulating tumor-derived DNA represents a new class of highly specific cancer biomarkers with clinical applications that may complement those of conventional serum protein markers. In one implementation, methods include screening ctDNA for presence of tumor-specific, somatic mutations. In such implementations, false-positive results are very rare since it would be very unlikely to find cancer-related mutations in the plasma DNA of a healthy individual. Disclosed methods include methods that measure rare mutant DNA molecules that are shed into blood from cancer cells with high analytical sensitivity and specificity. Achieving extremely high detection sensitivity is especially important for detection of a small tumor at an early (and more curable) stage.

Since somatic mutations can occur at many possible locations within various cancer-related genes, a clinically useful test for analyzing ctDNA would need to be able to evaluate mutations in many genes simultaneously, and preferably from many samples simultaneously. Analysis of a plurality of mutation-prone regions from a plurality of samples allows more efficient use of large volumes of sequence data that can be obtained using massively parallel sequencing technologies. In one implementation, labeling molecules arising from a given sample with a sample-specific DNA sequence tag, also known as a barcode or index, facilitates simultaneous analysis of more than one sample. By using distinct barcode sequences to label molecules derived from different samples, it is possible to combine molecules and to carry out massively parallel sequencing on a mixture. Resultant sequences can then be sorted based on barcode identity to determine which sequences were derived from which samples. To minimize chances of misclassification, barcodes are designed so that any given barcode can be reliably distinguished from all other barcodes in the set by having distinct bases at a minimum of two positions.

In most protocols that are currently used to prepare samples for massively parallel sequencing, barcodes are attached after several steps of sample processing (e.g. purification, amplification, end repair, etc). Barcodes can be attached either by ligation of barcoded sequencing adapters or by incorporation of barcodes within primers that are used to make copies of nucleic acids of interest. Both approaches typically use several processing steps to be performed separately on nucleic acids derived from each sample before barcodes can be attached. Only after barcodes are attached can samples be mixed.

In one implementation, barcodes are assigned to targeted molecules at a very early step of sample processing. Targeted early barcode attachment not only permits sequencing of multiple samples to be performed in batch, it also enables most processing steps to be performed in a combined reaction volume. Once barcodes are attached to nucleic acid molecules in a sample-specific manner, molecules can be mixed, and all subsequent steps can be carried out in a single tube. If a large number of samples are analyzed, targeted early barcoding can greatly simplify the workflow. Since all molecules can be processed under identical conditions in a single tube, the molecules would experience uniform experimental conditions, and inter-sample variations would be minimized. In one implementation, tagging of nucleic acids from different samples can be achieved in consistent proportions and then used to enable quantitative comparisons of nucleic acid concentrations across samples. Thus, early barcoding can be used to quantify a total amount of various targeted nucleic acids, and not just variants, across many samples.

In one implementation, well-defined mixtures of primers are produced containing combinations of sample-specific barcodes and consistent ratios of gene-specific segments. Such primers can be used for targeted early barcoding and subsequent batched sample processing. These primers can also be used for quantitation of DNA or RNA in different samples. In one implementation, such primers allow parallel processing and analysis of multiple mutation-prone genomic target regions from multiple samples in a simplified and uniform manner.

Currently disclosed methods include methods that accurately quantify mutant DNA rather than simply determining its presence or absence. In one implementation, an amount of mutant DNA provides information about tumor burden and prognosis. Currently disclosed methods are capable of analyzing DNA that is highly fragmented due to degradation by blood-borne nucleases as well as due to degradation upon release from cells undergoing apoptotic death. Since somatic mutations can occur at many possible locations within various cancer-related genes, One implementation can evaluate mutations in many genes simultaneously from a given sample. Currently disclosed methods are capable of finding mutations in ctDNA without knowing beforehand which mutations are present in a patient's tumor. One implementation is able to screen for many different types of cancer by evaluating multiple regions of genomic DNA that are prone to developing tumor-specific somatic mutations. One implementation includes multiple samples combined together in the same reaction tube to minimize inter-sample variations.

Although the currently described methods have been optimized for measurement of small amounts of mutant circulating tumor DNA (ctDNA) in a background of normal (wild-type) cell-free DNA in the plasma or serum of a patient having cancer, it is understood that they could be applied more broadly to the analysis of nucleic acid variants from a variety of sources. Examples of such sources include, but are not limited to lymph nodes, tumor margins, pleural fluid, urine, stool, serum, bone marrow, peripheral white blood cells, cheek swabs, circulating tumor cells, cerebrospinal fluid, peritoneal fluid, amniotic fluid, cystic fluid, frozen tumor specimens, and tumor specimens that have been formalin-fixed and paraffin-embedded.

### Methods

### High-throughput RNA quantitation:

### Up-front sample parallelization for simplified and accurate RNA measurement:

In one implementation, the high-throughput RNA quantitation method can be carried out via the following fundamental steps.

In one implementation, to enable early parallelization of workflow, sample-specific counting tags (barcodes) are assigned to a panel of RNA molecules being targeted within each sample during reverse transcription (RT). In one implementation, gene-specific primers are used to target the RNAs of interest for reverse-transcription. In one implementation, the RNAs of interest can be microRNAs, messenger RNAs, long-non-coding RNAs (IncRNAs), or any other RNA type. In one implementation, the gene specific primers are labeled with sample-specific barcodes. In one implementation, sample specific barcodes are assigned to complementary DNAs (cDNAs) during reverse transcription. In one implementation, the quantity of a given sample-specific tag that is assigned to a cDNA is proportional to the abundance of the corresponding RNA in the sample. In one implementation, the gene-specific hybridization region of the primers used for reverse-transcription can be as short as 6 nucleotides, and as long as 40 nucleotides. In certain implementations, gene-specific hybridization sequences are 6 nucleotides long when used to reverse-transcribe short microRNA targets. In one implementation, to enhance the specificity and stability of the 6-base-pair RNA/DNA interaction, the primer bases not binding to the microRNA can be masked by annealing a biotinylated oligonucleotide complementary to the common primer sequences. In certain implementations, gene-specific hybridization sequences are 15 to 25 nucleotides long when used to reverse-transcribe longer messenger RNA or lncRNA targets. In one implementation, multiple gene-specific primers can be used in the same reaction volume to perform targeted reverse-transcription (RT) of multiple RNA sequences. In one implementation, all primers used to reverse-transcribe RNAs from a given sample contain the same sample-specific barcode (tag). In one implementation, multiple samples can be simultaneously reverse-transcribed in separate reaction volumes. In one implementation, upon completion of reverse transcription, all tagged cDNA copies from all samples can be combined into a single volume and purified. In one implementation, pooled cDNAs can be purified by biotin-capture using streptavidin or its analogs immobilized on a surface.

In one implementation, a modular oligonucleotide synthesis scheme is used to ensure that RNAs from different samples are copied to complementary DNAs (cDNAs) in consistent proportions. In one implementation, to enable multiplexed targeted labeling of *i* RNAs during reverse transcription from j samples, it was necessary to create RT primers having i x j combinations of target-specific sequences attached to sample-specific tags. In one implementation, to ensure quantitative consistency, it was critical to reverse-transcribe different samples using uniquely tagged primer mixes having identical ratios of all target-specific sequences. Because simply mixing thousands of individually made primers was impractical and would yield imprecise ratios, a two-stage modular oligonucleotide synthesis strategy was used. In one implementation, oligonucleotide synthesis can be paused after making several different target-specific primer sequences. In one implementation, the synthesizer can be paused, and the particles harboring partially synthesized oligonucleotides can be mixed and dispensed into several fresh synthesis columns. In one implementation, synthesis can then be resumed, adding a sequence to each column that includes a unique sample-specific tag and a universal PCR primer-binding site. In one implementation, several primer mixes are produced, each having a unique sample-specific tag in the 5'-segment and a uniform composition of several target-specific primer sequences in the 3'-segment.

In one implementation, tagged cDNAs derived from all samples are pooled and purified. In one implementation, the cDNA pool is distributed into separate reaction volumes for amplification of each target by separate, single-plex, end-point PCRs (taken to plateau phase). Because all sample-specific tags associated with a given cDNA species are amplified competitively in a single volume, tag ratios encoding RNA abundance are preserved. In one implementation, incorporation of sequencing adapters at the 5'-ends of the PCR primers enables the resulting amplicons to be pooled, gel-purified, and directly used as templates for massively parallel sequencing without additional library preparation steps.

In one implementation, the relative amounts of RNAs in various samples can be deduced by enumerating the sample-specific tags associated with each cDNA sequence obtained by massively parallel sequencing of the PCR products.

### Utility and composition of modular primer mixes:

For the RNA profiling method, modular primer mixes were used to assign sample-specific tags to targeted nucleic acid molecules (in particular, cDNA copied from RNA templates). However, such modular primer mixes can have a broad range of uses. They can be used, more generally, to assign tags that could aid in identifying, categorizing, classifying, sorting, counting, or determining the distribution or frequency of targeted nucleic acid molecules (RNA or DNA). A modular primer mix is a mixture of primers having multiple distinct target-specific sequences in the 3' segment, and having a unique tag sequence in the 5' segment. Often, several modular primer mixes are made as a set, such that each primer mix has a distinct tag, and all mixes have the same composition of target-specific sequences. When the numbers of targets and tags become large, it can be impractical to individually synthesize primers and then mix them.

The tags (also referred to as barcodes or labels) that are incorporated into modular primer mixes may consist of arbitrary sequences, but typically include pre-defined sequences that can be reliably differentiated from each other. For example, in the RNA profiling method, each tag was designed to differ from all other tags in the set by at least two nucleotide positions so that sequencing errors would rarely lead to misclassification of tags. Tags need not be contained within a single, contiguous stretch of bases. In certain implementations, nucleotide positions comprising tag sequences can be distributed across non-contiguous regions of the 5' segments of modular primer mixes. Tags can also contain random or degenerate positions (A degenerate position is one at which, for example, the four nucleotides A, T, C, and G are incorporated with equal probability during oligonucleotide synthesis). However, tags within modular primer mixes must contain at least some positions having pre-defined (not degenerate) sequences.

Within modular primer mixes, tags need not be sample-specific. For example, a tag can be assigned to a sample, a molecule, a location, or a compartment. A tag can also be assigned to a set of samples, a set of molecules, a set of locations, or a set of compartments. Depending on the application, the assignment of tags could be random (e.g. any tag is randomly assigned to any sample, molecule, location, or compartment), or it could be pre-determined (e.g. one can decide to assign a particular tag to a particular sample, molecule, location, or compartment). Unique assignment of tags is not always necessary. For some applications each sample, molecule, location, or compartment must be assigned a unique tag. For some other applications it is acceptable for a given tag to be assigned to more than one sample, molecule, location, or compartment.

In some applications, more than one modular primer mix can be used to label a target or set of targets. For example, modular primer mixes could be used as both forward and reverse primer sets in a PCR amplification reaction, permitting assignment of two distinct tags to a target. A large diversity of labels can be achieved by using various combinations of tagged forward and reverse primer mixes.

### Quantitation of low-abundance mutant DNA from complex mixtures

### Isolation of Template DNA:

Methods for purification or isolation of DNA or RNA from various clinical or experimental specimens are disclosed. Many kits and reagents are commercially available to facilitate nucleic acid purification. Depending on the type of sample to be analyzed, appropriate nucleic acid isolation techniques can be selected. Substances that might inhibit subsequent enzymatic reaction steps (such as polymerization) should be removed or reduced to non-inhibitory concentrations in purified DNA or RNA samples. Yield of nucleic should be maximized whenever possible. It would be disadvantageous to lose DNA during purification, since the lost DNA might include rare variant DNA. When isolating DNA from plasma, about 1 ng to 100 ng of cell-free DNA can be purified from 1 mL of plasma, which corresponds to about 350 to 35,000 genome copies. DNA yields can vary dramatically, especially in patients with an ongoing disease process such as cancer.

In one implementation, DNA can also be analyzed from other sample types, including but not limited to the following: pleural fluid, urine, stool, serum, bone marrow, peripheral white blood cells, circulating tumor cells, cerebrospinal fluid, peritoneal fluid, amniotic fluid, cystic fluid, lymph nodes, frozen tumor specimens, and tumor specimens that have been formalin-fixed and paraffin-embedded.

### Lineage-Traced PCR

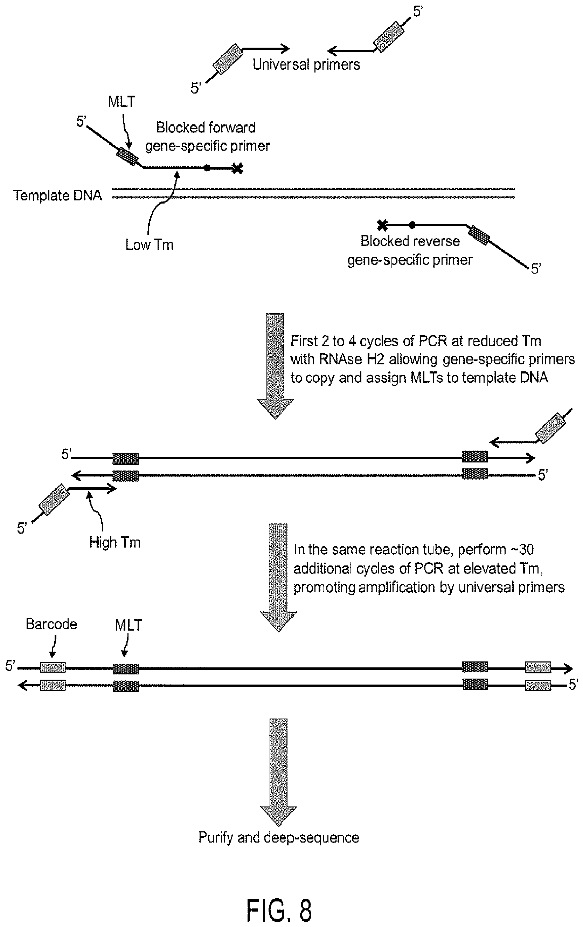

In one implementation, methods are provided that enable targeted template DNA molecules to be labeled with "molecular lineage tags" (MLTs) using gene-specific primers, and that enable these tagged copies to then be further copied (amplified) using universal primers. In one implementation, this reaction is performed in a single reaction volume without transferring reagents, which offers a significant advantage of procedural simplicity. As illustrated in Figure 8, several gene-specific primers containing MLT sequences are used to simultaneously copy and label multiple targeted genomic regions of interest (e.g., regions that are prone to somatic mutations in cancer). The gene-specific primers have a melting temperature (for hybridization to the target gene sequence) that is lower than the melting temperature of the universal primers. Copying of targeted template DNA fragments and assignment of MLT sequences is promoted by using a lower annealing temperature during the first few (two to four) cycles of PCR. In subsequent PCR cycles, the annealing temperature is raised to discourage further participation of the MLT-containing gene-specific primers in the reaction. The 5' portion of the forward gene-specific primers contains a common sequence that is identical to the 3' portion of the forward universal primer sequence. The 5' portion of the reverse gene-specific primers contains a second (different) common sequence that is identical to the 3' portion of the reverse universal primer sequence.

The universal primer sequences are designed to have a higher melting temperature than the gene-specific primers. In one implementation, universal primers can be modified with nucleotide analogs at some positions to increase the stability of hybridization, such as locked nucleic acid (LNA) residues. Alternatively, universal primers can simply have a longer sequence and/or greater G/C content to increase the melting temperature. During the later cycles of PCR (after the first two to four cycles) the annealing temperature of thermal cycling can be raised to a level at which universal primers can efficiently hybridize, but gene-specific primers cannot. Thus, the MLT labeled copies which are generated in the first few PCR cycles become amplified and should comprise a large portion of the amplicon sequences.

In one implementation, the gene-specific primers would be present in the PCR cocktail in relatively low concentration (∼10 to ∼50 nM each), whereas the barcoded universal primers would be present in higher concentration (∼200 to ∼500 nM each). In one implementation, short universal primers lacking a barcode and adapter sequence could also be added to the cocktail in a relatively high concentration (∼100 nM to 500 nM each). To allow sufficient time for hybridization and extension of the low-concentration gene-specific primers, a longer annealing time can be used for the first few PCR cycles, with optional slow cooling to the annealing temperature. During subsequent PCR cycles, a faster annealing time can be used because of the higher concentration of the universal primers.

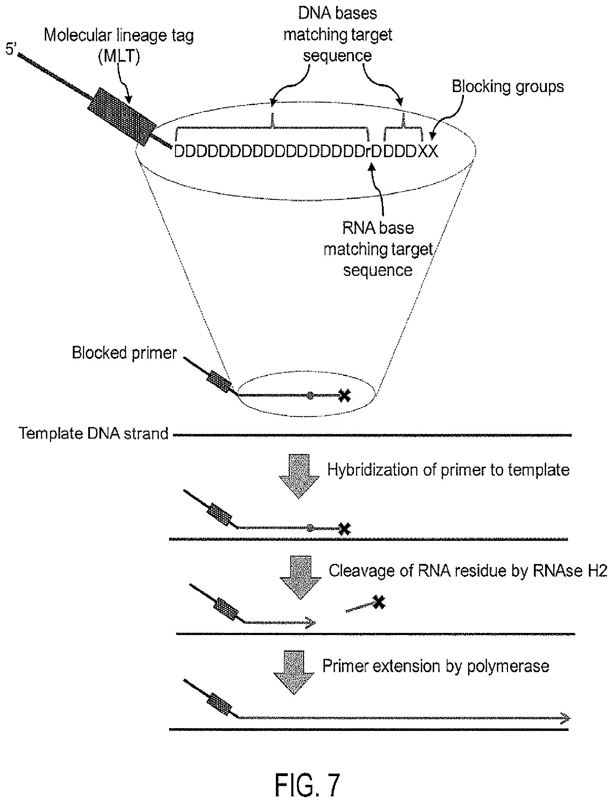

Minimizing off-target hybridization and extension of gene-specific primers is critical to the success of this method. Because of the presence of universal primers within the same reaction cocktail, it is especially important to minimize hybridization and extension of gene specific primers with each other (i.e., formation of primer dimers). Even very small amounts of dimer formation among gene-specific primers can be catastrophic to the reaction, because those dimers can be exponentially copied and amplified by the universal primers. If the amplification of dimers dominates the reaction, the targeted gene regions may not be sufficiently amplified. To minimize off-target hybridization and extension of gene-specific primers, In one implementation, blocked gene-specific primers are used. The 3'-end of such primers is blocked with one or more residues that cannot be extended by a PCR polymerase. It is also important that the blocking group should not be digestible by the 3'-5' exonuclease activity of the polymerase. For this purpose, In one implementation, two nucleotides can be attached in the reverse orientation at the end of the primer (so that the penultimate linkage is 3'-3'). As illustrated in Figure 7 a single RNA residue can be introduced into the DNA oligonucleotide, so that the blocking group can be cleaved off by thermostable RNAse H2 enzyme upon target-specific hybridization of the primer. Upon cleavage of the blocking group, the primer can be extended on its intended target. While some spurious hybridization and extension may still occur, such measures can minimize its impact on the reaction.

Figure 7 shows a schematic of an RNAse H2-activatable primer that is designed to resist digestion of its terminal blocking groups by the 3' to 5' exonuclease activity of proofreading polymerases. Blocking groups are added to the 3'-end of the primer to prevent non-specific extension of the primer, especially to avoid formation of primer dimers. Upon specific hybridization of the primer to its target DNA sequence, a thermostable RNAse H2 enzyme can cleave the primer at its single RNA nucleotide, producing a 3' hydroxyl end that can then be extended by a polymerase. The positions indicated with a "D" represent DNA nucleotides that are complementary to the target sequence. The position indicated with an "r" represents an RNA nucleotide that is complementary to the target sequence. The blocking groups indicated by "XX" represent two nucleotides that are attached in reverse orientation (the penultimate linkage is a 3'-3' linkage, and the terminal "X" has a free 5' hydroxyl). The XX positions are synthesized using 5'-CE (beta-cyanoethyl) phosporamidites. A dA-5' phosphoramidite was used, but one could also use dC-5', dT-5', or dG-5'. A polymerase will not extend from a 5' terminus, nor will its proofreading 3'-5' exonuclease activity digest such a terminus. In this example, the 5' region of the primer is depicted as having a degenerate molecular lineage tag and a universal primer sequence, but these features are optional and other features such as a sample-specific barcode could be included.

Figure 8 provides a schematic description of Lineage-Traced PCR. The goal of Lineage-Traced PCR is to assign molecular lineage tags (MLTs) to template molecules during the first few cycles of PCR, and then to amplify these tagged copies using universal primers during subsequent PCR cycles (while minimizing incorporation of additional MLTs). This strategy can be used to differentiate true template-derived mutations from polymerase misincorporation errors and sequencer errors. The strategy can also be used to confirm that both strands of a double-stranded DNA template were tagged and amplified within a small reaction volume such as a droplet or micro-well. Lineage-traced PCR can be carried out in a single reaction volume or in multiple microscopic reaction volumes using a continuous thermal cycling program without transferring or adding reagents. The method uses gene-specific primers that have a low melting temperature (for example, 60° C), and universal primers that have a higher melting temperature (for example, 72° C). The gene-specific primers contain an MLT sequence as well as a universal primer sequence in their 5' region. At least the first two (but as many as the first four) cycles of PCR are carried out at a low Tm (e.g. 60° C) to permit hybridization and extension of the MLT-containing gene-specific primers. For the subsequent ∼30 cycles of PCR, a higher Tm is used (e.g. 72° C) to promote preferential use of universal primers, and to minimize incorporation of additional MLTs. To avoid amplification of spurious products by the universal primers, it is imperative to minimize primer-dimer formation from the gene-specific primers. Thus scheme to enhance primer specificity must be employed, such as use of RNAse H2 activatable gene-specific primers. Universal primers could also be RNAse H2 activatable, although that is optional. Here the universal primers are shown to contain a sample-specific barcode, but this portion of the primer could be omitted, or other features could be incorporated depending on the intended application. Tm = melting temperature. MLT = molecular lineage tag.

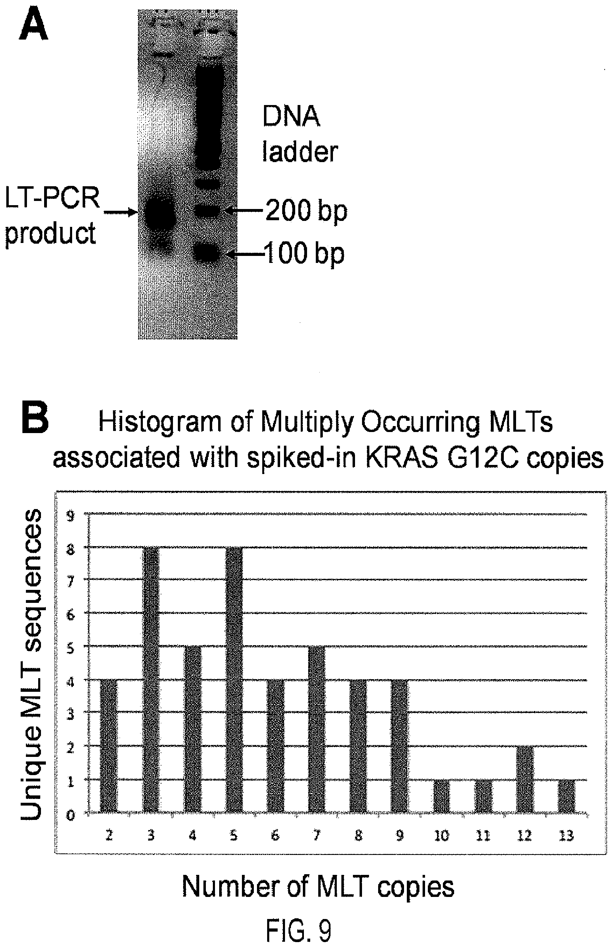

Figure 9 shows results of lineage-traced PCR experiments. Figure 9 (A) shows that amplification products from a single-tube lineage-traced PCR experiment produce a band migrating at the expected size on a 2% agarose gel. Figure 9 (B) shows analysis of next-generation sequencing data generated from lineage-traced PCR amplification products shows an expected distribution pattern of MLT copies on a histogram. The analyzed sample consisted of ∼20 genome equivalents of double-stranded DNA containing a known KRAS G12C mutation spiked into ∼6000 genome equivalents of double-stranded wild-type DNA derived from healthy volunteer human plasma. The X-axis indicates the number of KRAS G12C mutant reads in which a given MLT sequence pair was found. The Y axis indicates the number of unique MLT sequence pairs (different tags) having a given number of read copies. Since approximately 20 double-stranded mutant DNA copies were added to the reaction, ∼40 different MLT sequence pairs would be expected to have multiple read counts, as was observed.

In one implementation, the specificity of universal primers can also be enhanced by incorporating an RNAse H2-cleavable blocking group into the primers. In one implementation, universal primers can also be labeled with sample-specific barcodes, so that use of different barcoded primers for different samples would allow the PCR products to be pooled and subjected to next-generation sequencing in batch. The sequence data could then be sorted into sample-specific bins based on barcode identity. In one implementation, universal primers can also contain adapter sequences, which facilitate sequencing on a next-generation sequencing (NGS) platform of choice. In one implementation, a mixture of long (containing sample-specific barcode and adapter sequence) and short (lacking barcode and adapter) universal primers can be used. Because the short primers would have faster hybridization kinetics, they can enhance the efficiency of amplification during the early cycles of PCR.

In certain implementations, the DNA products are gel-purified to select products of the desired size and to eliminate unused primers before subjecting to massively parallel sequencing. In certain implementations, other approaches to purification could be used, including but not limited to hybrid capture using biotin-tagged complementary oligonucleotides, high-performance liquid chromatography, capillary electrophoresis, silica membrane partitioning, or binding to magnetic Solid Phase Reversible Immobilization (SPRI) beads.

In one implementation, a next-generation sequencer is used to obtain large numbers of sequences from the tagged, amplified, and purified PCR products. Clonal sequences (each sequence arising from a single nucleic acid molecule) produced by such a sequencer can be used to identify and quantify variant molecules using an approach known as ultra-deep sequencing. In principle, because large numbers of sequences can be obtained for each target site and for each sample, rare variants can be detected and measured. However, the error rate of the sequencer can limit the sensitivity of detection because such errors might be mistaken as true variants. To minimize the contribution of sequencer errors, One implementation uses clonal overlapping paired-end sequences. By separately sequencing opposite strands of DNA from each clonal population, and comparing the overlapping regions of the sequences, the vast majority of variants arising from sequencer errors can be eliminated. In one implementation, the region of sequence overlap is designed to be in the mutation-prone area. In one implementation, only read-pairs that perfectly match in the overlapping region are retained for further analysis. For such analysis, sequencers that produce clonal paired-end reads are useful. In certain implementations, other massively parallel sequencing platforms can also be utilized.

In one implementation, errors introduced during PCR amplification, processing, or sequencing can be distinguished from true template-derived mutant sequences by analyzing the distribution of molecular lineage tags (MLTs) associated with variant sequences. If the number of acquired NGS reads for a given target-sample bin is several-fold greater than the number of targeted template DNA copies within that sample, then an originally-assigned MLT would be expected to be present in multiple copies. Thus, if a mutant template DNA fragment were labeled with an MLT sequence during an early cycle of PCR, then the sequence data would be expected to contain multiple reads having that MLT sequence and the mutation. Conversely, variants arising from PCR errors or sequencer errors would be expected to contain fewer reads having the same MLT sequence (typically each MLT sequence would occur only once). In one implementation, MLTs can also be used to distinguish sequences bearing incorrect sample-specific barcodes due to cross-over events during pooled amplification.

### Compartmentalized PCR followed by NGS to identify matching mutations on both strands of a DNA duplex

Although the lineage-traced PCR method described above can distinguish true template-derived mutations from most PCR errors and sequencer errors, it has difficulty identifying misincorporations that occur during the first few PCR cycles. Variant sequences arising from such an early misincorporation error can be associated with a relatively high number of MLT copies, similar to the multiple MLT copies expected for a true template-derived mutation. To improve upon this limitation, an alternative strategy for identifying template-derived mutations is to confirm that the same mutation exists on both strands of a given double-stranded template DNA fragment. Errors arising from PCR or from base damage of the template DNA would be very unlikely to produce complementary alterations on copies of both strands of the same template fragment.

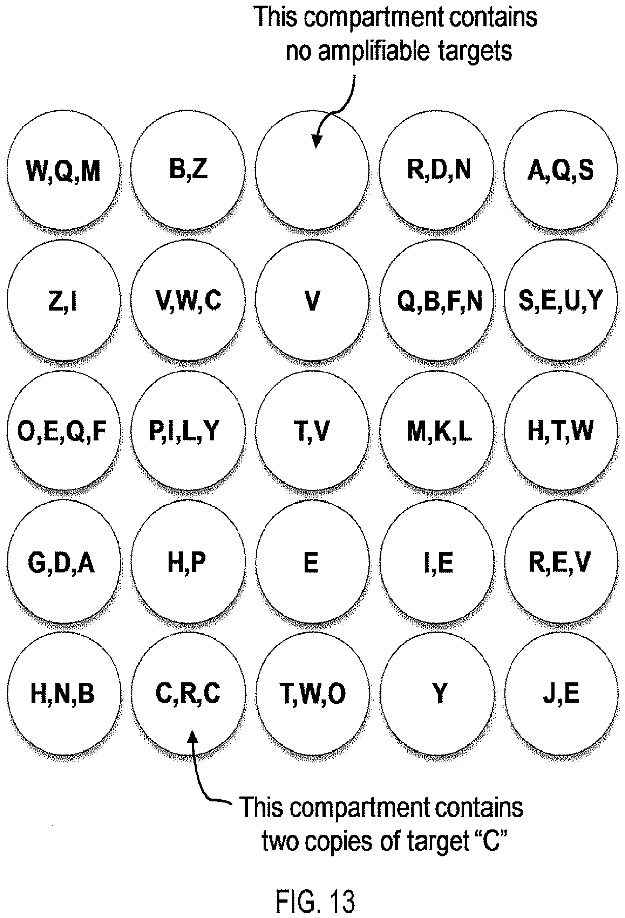

In one implementation, a compartmentalization, tagging, amplification, and sequencing strategy is used to verify that a mutation is present on both strands of a double-stranded template DNA fragment. In one implementation, the PCR reaction cocktail is similar to that used for lineage-traced PCR above (it contains universal primers and a mixture of RNAse H2-activatable gene-specific primers that contain MLT sequences). However, an important difference is that one of the long universal barcoded primers (either forward or reverse) is omitted from the cocktail so that primers containing a compartment-specific barcode can be used instead. In one implementation, the PCR reaction cocktail (including template DNA fragments) is divided into many microfluidic compartments so that any given compartment has a very low probability of containing more than one copy of a particular targeted template DNA fragment. As illustrated in Figure 13, a compartment can have multiple amplifiable targeted fragments (different targets), but it should rarely have more than one copy of the same target. For example, if a copy of a given target is only found in approximately 1 out of 10 compartments, then the probability of finding two copies of that target in the same compartment would be ∼1/100. All compartments contain universal primers and the full panel of gene-specific primers, so that all amplifiable targets within a compartment would be tagged, copied, and amplified. In one implementation, all compartments are simultaneously subjected to the same thermal cycling protocol (similar to that used for lineage-traced PCR).

Figure 13 shows an example of how different targets might be randomly compartmentalized within droplets or micro-wells for PCR amplification. Each letter represents a targeted template DNA fragment, and each occurrence of a letter represents a single copy of that target. Compartmentalization of the amplification reaction is carried out such that typically zero or one (and occasionally two or more) copies of a given amplifiable, targeted template DNA fragment is present within a compartment. However, since multiple genomic regions are simultaneously targeted, several different targeted DNA fragments (usually in single copy each, occasionally in more than one copy) can be present within a compartment.

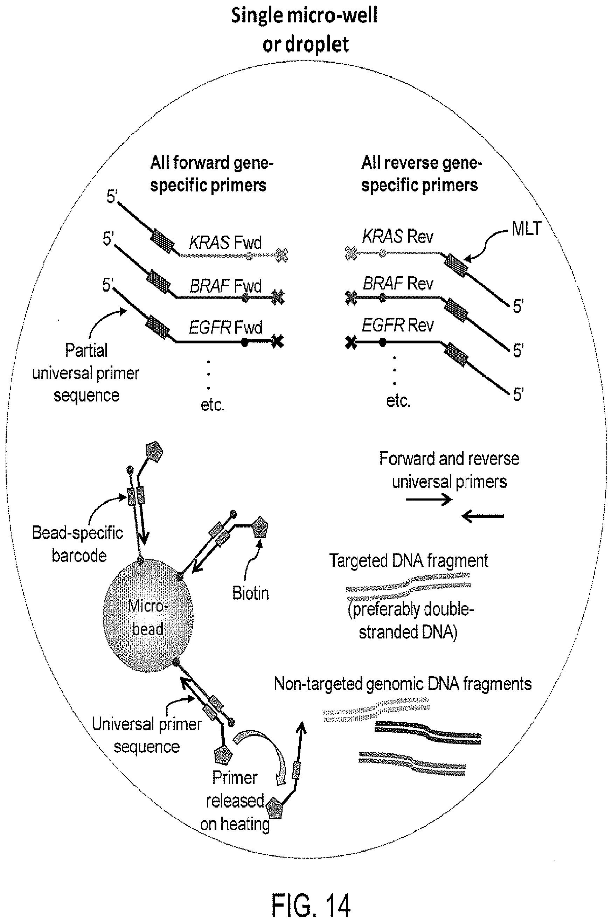

Figure 14 shows an example of the contents of a single reaction compartment (such as a micro-well or a droplet). Shown are MLT-containing gene-specific primers, universal primers, targeted template DNA fragments (and other non-targeted DNA fragments), and a bead carrying heat-releasable primers having a bead-specific barcode. In addition to this, the reaction compartment would contain reaction buffer, dNTPs, RNAse H2 enzyme, and polymerase (such as Phusion Hot Start). All compartments would contain the full panel of gene-specific primers. Each gene-specific primer contains an MLT sequence and it also has a portion of the universal primer sequence. Each gene-specific primer is present in relatively low concentration such as 5 to 50 nM. Universal primers are in high concentration (e.g. 200 to 500 nM). Barcoded primers released from the bead would be expected to have a relatively low concentration in the compartment (∼5 to 50 nM). Double stranded DNA template fragments would allow the most robust error suppression, but single stranded templates could also be used. Any given micro-bead carries multiple copies of primers having the same bead-specific barcode. Since bead distribution within compartments is approximately random, many compartments would contain more than one micro-bead, and a minority of compartments would contain none (determined by Poisson statistics). In this example, biotin labeled amplification products would then be captured and isolated using streptavidin coated beads.

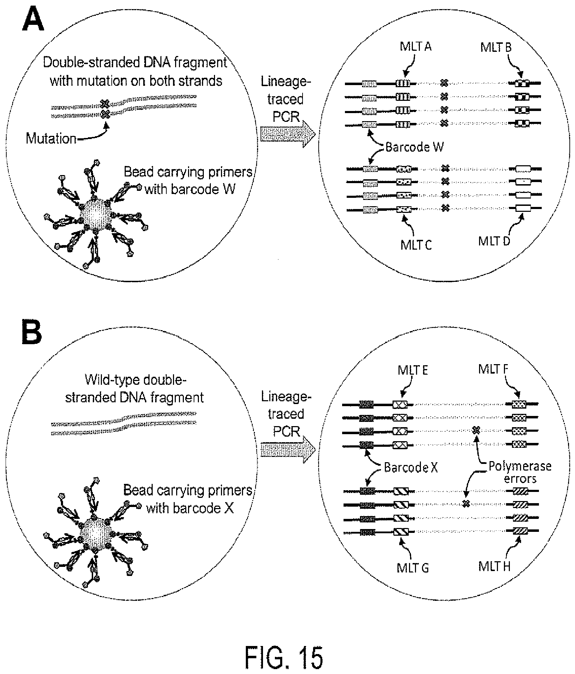

Figure 15 A and B show two example scenarios of lineage-traced PCR being carried out within a micro-compartment containing a single microbead carrying barcoded primers. Panel A depicts tagging and amplification of a double-stranded targeted DNA fragment that contains a true mutation on both strands of the duplex (the two strands of the duplex are perfectly complementary). In this case, the same bead-specific barcode is assigned to all amplification products. The presence of mutations in multiple reads containing two distinct MLT pairs (i.e. A-B, and C-D) indicates that the mutation was present on both strands of the template DNA. Panel B depicts similar tagging and amplification of a wild-type double-stranded DNA fragment. In this case, the amplification products contain a few polymerase errors, but when sequences are grouped by bead-specific barcode, no consistent mutation is seen. MLTs and barcodes labeled with different letters (e.g. MLT G or Barcode W) represent different nucleotide sequence tags. For simplicity, each tag or barcode is identified by a single letter of the alphabet, whereas in reality each tag typically consists of a stretch of six to ten bases.

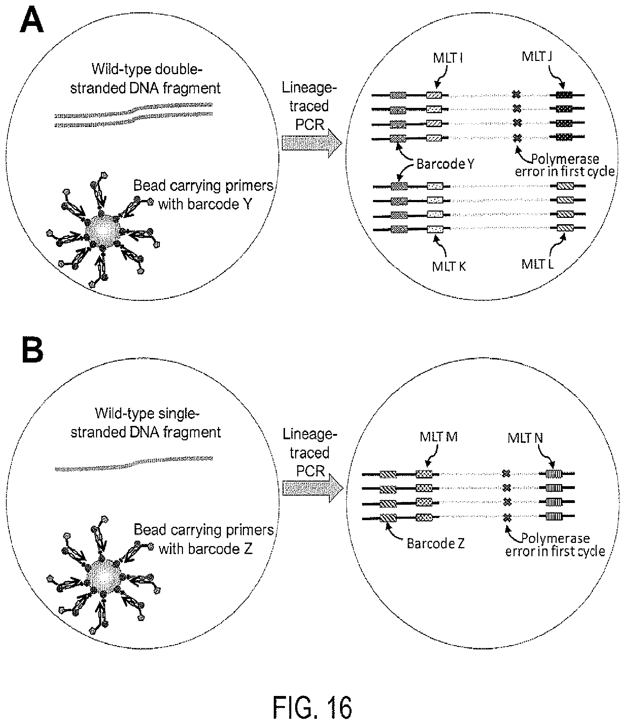

Figure 16 A and B show two additional example scenarios of lineage-traced PCR being carried out within a micro-compartment containing a single microbead carrying barcoded primers. Panel A depicts tagging and amplification of a wild-type double- stranded DNA fragment in which a polymerase misincorporation error occurred during the first cycle of PCR, when copying one of the two DNA template strands. This is shown as an extreme example of how an error could be distinguished even if it occurred during the first cycle of PCR. In this case, the amplification products show the error associated with only one of the two MLT pairs (i.e. I-J), not with both MLT pairs (i.e. I-J and K-L) as would be expected if a true mutation were copied from both strands of a template DNA duplex. Panel B depicts tagging and amplification of a wild-type single-stranded DNA fragment in which a polymerase misincorporation error occurred during the first cycle of PCR. In this case, although the error may be found in the entire population of amplified copies within that compartment (tagged with barcode Z), the copies all have a single MLT pair (i.e. M-N), not two (or more) MLT pairs as would be expected for a true mutation copied from both strands of a template DNA duplex.

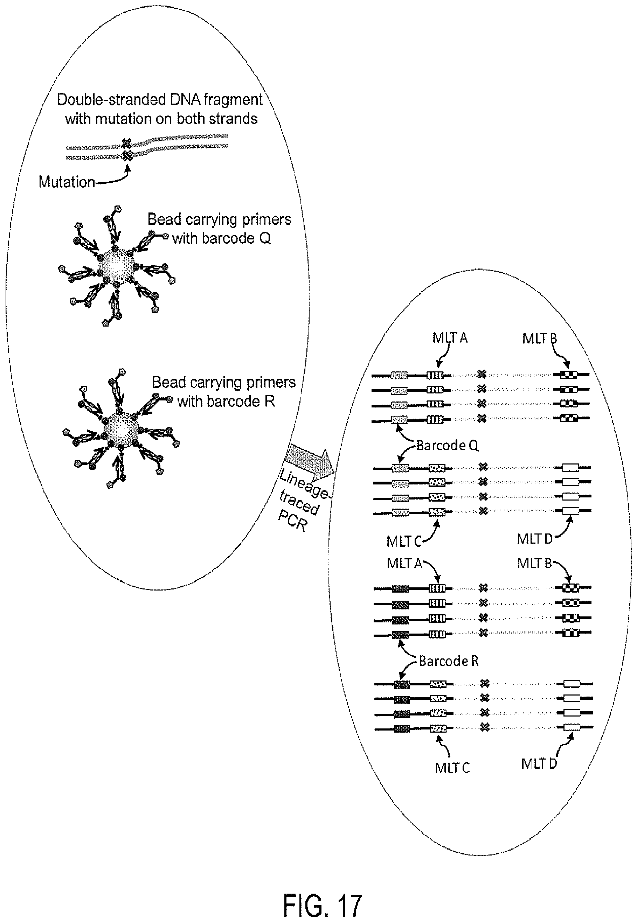

Figure 17 illustrates how the analysis would be performed if there were two (or more) differently barcoded primers on two (or more) beads in a given compartment. Beads are expected to be distributed within different compartments according to a Poisson distribution, with some compartments containing zero beads, some compartments containing a single bead, and some compartments containing two or more. In order to reduce the number of compartments containing zero beads, one could aim to achieve a median of two or three beads per compartment. Alternatively, methods exist to overcome Poisson statistics to distribute a single bead into a single compartment, but these approaches involve complex microfluidic manipulations or pre-dispensing of primers into defined reaction chambers. Compartments in which more than one barcoded primer set is present can be identified during subsequent computational analysis of sequence data. Because a given MLT pair would have an extremely low probability of being found in sequences derived from more than one compartment, all compartment-specific barcodes associated with such a pair can be inferred to be derived from a single compartment.

In one implementation, molecular lineage tags (MLTs) are assigned to template molecules via gene-specific primers, and then these tagged copies are amplified by universal primers as was described for lineage-traced PCR. Within a compartment, if there is generally not more than one copy of a given targeted double-stranded template DNA fragment, then MLTs can be used to identify amplified sequences arising from copies of the two different strands (illustrated in Figure 15). In one implementation, primers containing one or a few compartment-specific tags would be used to identify the amplicons produced within a given reaction compartment. Thus, using such a tagging scheme, it is possible to confirm that the same variant sequence was copied from two different strands of DNA within the same compartment.

The PCR cocktail can be divided into microfluidic compartments in various ways. In one implementation, the compartments can be as small at 10 picoliters and as large as 10 nanoliters. In certain implementations, the compartments are between ∼0.1 to 1 nanoliter in volume. Ideally, the volume of the compartments for a given experiment should be uniform. The number of compartments can range from a few thousand to several million, depending on the application and the expected concentration of template DNA molecules. In one implementation, PCR compartments can be produced as droplets of PCR cocktail in oil using a microfluidic droplet generator device. Mineral oil can be used for this purpose or fluorinated oils can also be used. Surfactant can be used to stabilize the droplets and prevent coalescence of droplets before or during PCR. In one implementation, an emulsion of PCR cocktail in oil can also be made simply by vigorously agitating the mixture (but this approach has the disadvantage of creating non-uniform droplet sizes). In another implementation, the PCR cocktail can be compartmentalized into micro-wells on a microfluidic device. In one implementation, a slide containing patterned polydimethylsiloxane (PDMS) with thousands of nanoliter-sized wells can be used. In one implementation, a microfluidic device containing a narrow serpentine channel can be used in which reaction volumes are separated by oil or air. In one implementation, a similar microfluidic device can be used in which a PCR cocktail can be introduced into channels and then the channels can be divided into separate reaction chambers by simultaneously closing thousands of micro-valves. PCR can be carried out by thermal cycling the micro-compartments simultaneously.

In one implementation, clonal primers containing a compartment-specific tag (or barcode) can be introduced to the compartments via a micro-bead. It is possible to produce a large population of micro-beads that each carry many copies of uniformly tagged primers, but a large diversity of tags exists on different beads. A given bead would carry a clonal population of tagged primers on its surface (all having the same tag), but different beads would carry primers having different tags. In one implementation, microbeads can be mixed with the PCR cocktail and can be compartmentalized with the cocktail. In one implementation, the concentration of beads would be adjusted so that an average of two or three beads would be delivered to each compartment (such that few compartments would have zero beads). The distribution of beads into compartments would be expected to follow Poisson statistics. In one implementation, primers can be released into the compartmentalized solution from the bead surface by heating (by melting the primer off from a complementary DNA strand attached to the bead). In another implementation, primers can be released into the compartmentalized solution from the bead surface by photocleavage (a photocleavable phosphoramidite can be used to link the oligonucleotide to the bead surface). In another implementation, the primers can remain attached to the beads and the hybridization and polymerization reactions can be performed on the bead surface. In one implementation, super-paramagnetic beads can be used (coated with cross-linked polystyrene and surface activated with amine or hydroxyl groups). In other implementations, beads can be used that are composed of materials including but not limited to agarose, polyacrylamide, polystyrene, or polymethyl methacrylate. In one implementation, beads can be coated with streptavidin to bind to biotin-labeled oligonucleotides. In certain implementations, beads can be between 0.5 micrometers and 100 micrometers in size. In certain implementations, beads are between 1 micrometer and 5 micrometers in size. In certain implementations, beads used in a given experiment are a relatively uniform size and carry a relatively uniform number of primer copies on each bead.

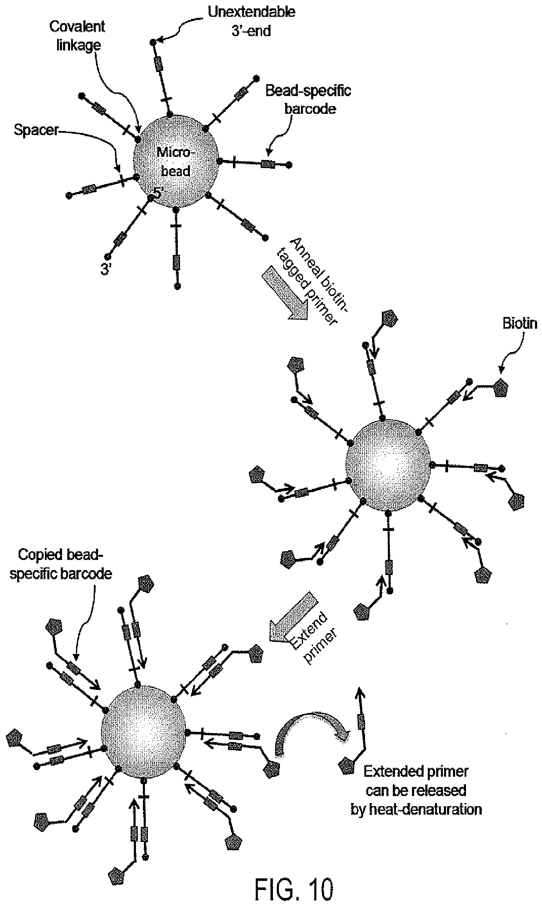

Figure 10 shows an example of how heat-releasable primers containing bead-specific barcodes can be produced on microbeads. First, oligonucleotides can be synthesized on the surface of microbeads using standard phosphoramidite chemistry on an automated oligonucleotide synthesizer. The microbead surface can be functionalized with, for example, amine or hydroxyl groups, which will form a covalent linkage with phosphoramidite monomers. Additional phosphoramidite monomers can then be added sequentially using standard synthesis protocols. Depending on the desired orientation of the bead-bound oligonucleotide, either standard or 5'-beta-cyanoethyl phosphoramidite monomers can be used. To introduce some distance between the oligonucleotide and the bead surface, one or multiple spacer phosphoramidites can be added to the bead surface before adding nucleotide monomers. Split and pool synthesis, as described in the methods section, can be used to incorporate bead-specific barcodes in the oligonucleotides. If microbeads are too small to be retained by the frits used in the columns of automated oligonucleotide synthesizers, one can use super-paramagnetic microbeads held in place by a magnet. A second oligonucleotide containing a common priming sequence (and an optional biotin group) can be used to copy the bead-bound oligonucleotide using a DNA polymerase. In this way, the extended primers would contain the bead-specific barcode sequences as well as the universal primer sequence. After the beads are compartmentalized into smaller reaction volumes such as droplets or micro-wells, the extended primer containing the bead-specific barcode can be released from the bead by heat-denaturation (e.g. during PCR). Other modes of primer release could also be used, such as photocleavage and chemical decoupling.

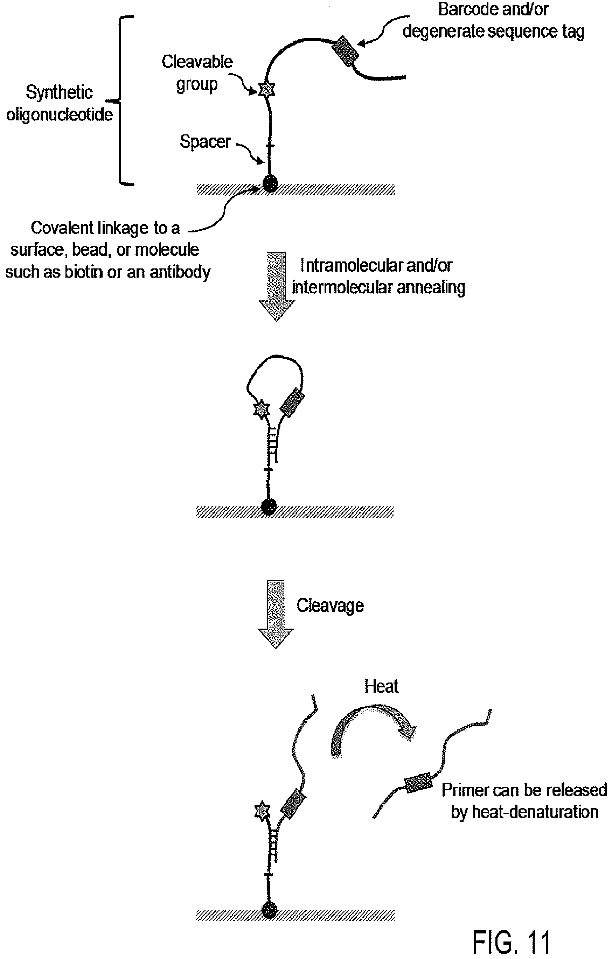

Figure 11 shows an alternative method for producing temporarily immobilized oligonucleotides that can be released by heat-denaturation. Oligonucleotides containing a cleavable group (for example, a photo-cleavable linker) can either be directly synthesized on a surface (such as a micro-bead) or can be coupled post-synthesis to a surface, particle, or molecule via a covalent bond or biotin affinity capture. A set of defined barcode sequences or degenerate tag sequences (such as MLTs) could be incorporated into the oligonucleotide. The tags could also be synthesized via split-and-pool synthesis to produce a large diversity of tags with multiple copies of the same tag on a given bead (or particle). The oligonucleotide is designed to have a region of self-complementarity, such that the cleaved oligonucleotide would remain attached via base-pairing interactions (hybridization). The oligonucleotide can be released into solution at a later time by heat-denaturation. The oligonucleotide can be synthesized in either the 5' to 3' or the 3' to 5' direction, depending on the downstream application.

In one implementation, a population of beads carrying a diverse set of clonally tagged primers (one bead, one tag) can be synthesized using a split-and-pool oligonucleotide synthesis approach. Common primer sequences can be synthesized using standard phosphoramidite chemistry on an automated oligonucleotide synthesizer. Primers can be synthesized in the 5' to 3' or the 3' to 5' direction, using the appropriate phosphoramidites. In one implementation, phosphoramidites can be covalently linked to the beads by using beads whose surface is modified with amine or hydroxyl groups. In one implementation, a permanent magnet or electromagnet can be used to retain magnetic microbeads within a synthesis column on an automated oligonucleotide synthesizer (since beads may be too small to be retained by a frit). In one implementation, a split-and-pool synthesis approach is used to produce a diversity of clonal tags on the beads. The common region of the primer is made, and then the synthesizer is paused at the beginning of the tag sequence. In one implementation, the beads are pooled and then split into four different fresh columns, and a different phosphoramidite (dA, dT, dC, or dG) is added to the four columns (one phosphoramidite per column). In another implementation, more or less than four columns and four phosphoramidites can be used (to increase or decrease the number of possible residues at a given position). After each coupling cycle within the tag region, the beads are pooled and re-distributed into fresh columns for the next cycle. In this way, the oligonucleotides coupled to a given bead receive the same base in a given cycle, but which base is added at a given position is randomly chosen. In one implementation, a bead-specific tag sequence can be between 1 and 15 bases in length. In certain implementations, a bead-specific tag sequence can be 8 to 12 bases in length. In one implementation, a complementary primer can be hybridized to the bead-bound oligonucleotide and extended using a polymerase to copy the tag sequence and additional primer sequence as schematized in Figure 10. The extended primer would serve as a heat-releasable primer having a bead-specific barcode. In one implementation, this heat-releasable barcoded primer can be used to hybridize and extend on the PCR amplified targets within the compartment (the 3'-end of the heat-releasable primers would contain a portion of the universal primer sequence to facilitate hybridization with the targeted amplicons).

In another implementation, primers containing compartment-specific tags can be pre-distributed within compartments. For example, if a PCR cocktail is to be divided into micro-wells on a microfluidic device, primers containing compartment-specific tags can be added to each micro-well before adding the PCR cocktail. In one implementation, primers could be chemically coupled to the surface or the wall of a micro-well, or coupled via a biotin-streptavidin interaction. In one implementation, primers could be released from the microwell by heating (by melting off of an immobilized complementary oligonucleotide as described above), by photocleavage, or other means. In one implementation, primers could remain attached to the surface of the well, and polymerization could be carried out on the surface.

In one implementation, tagged amplification products would be pooled after PCR by combining the contents of the many small reaction volumes. In one implementation, this can be achieved by adding a reagent that causes aqueous droplets in oil to coalesce (e.g. chloroform). In one implementation, reaction volumes can be combined by harvesting reaction products from micro-wells on a microfluidic device. In one implementation, the pooled, amplified DNA products are gel-purified to select products of the desired size and to eliminate unused primers before subjecting to massively parallel sequencing. In certain implementations, other approaches to purification could be used, including but not limited to hybrid capture using biotin-tagged complementary oligonucleotides, high-performance liquid chromatography, capillary electrophoresis, silica membrane partitioning, or binding to magnetic Solid Phase Reversible Immobilization (SPRI) beads.

In one implementation, next-generation sequencing (NGS) is used to obtain large numbers of sequences from the tagged, amplified, and purified PCR products. In one implementation, a clonal overlapping paired-end sequencing approach (as described above) can be used to filter out reads containing sequencer-derived errors. In one implementation, sequence data is analyzed to identify true mutations derived from copying both strands of a targeted double-stranded template DNA fragment. The strategy used to identify such true mutations can be understood by referring to Figures 15-17. The following logic is used:
1. In one implementation, MLT patterns can be used to determine whether amplified PCR products within a micro-compartment were derived from copying one template strand or two template strands. In one implementation, if a single MLT sequence-pair is seen in the amplified sequences from a given compartment, then it can be inferred that the amplified sequences were derived from a single strand of DNA that was amplified within that compartment. In one implementation, if two (or more) MLT sequence-pairs are seen in the amplified sequences from a given compartment, then it can be inferred that the amplified sequences were derived from two (or more) strands of DNA that were amplified within that compartment.
2. In one implementation, PCR amplified sequences can be identified as being derived from a given compartment based on analysis of compartment-specific barcodes. In one implementation, there can be a single barcode assigned to a compartment. In another implementation, there can be more than one barcode assigned to a compartment. If there is more than one barcode, the combination of barcodes can be used to identify the PCR products as having been derived from the same compartment.
3. In one implementation, a mutation would be considered to be an authentic template-derived mutation if the (a) the majority of amplified sequences derived from a given compartment contain the mutation, and (b) the observed MLT pattern confirms that the amplified sequences are derived from more than one template strand. Since a compartment would be very unlikely to contain more than one DNA fragment, it can be inferred with high certainty that sequences derived from more than one template strand are derived from complementary strands of a duplex DNA fragment.

### Method for delivering clonally tagged oligonucleotides to different compartments:

Using beads to deliver clonally tagged primers to different compartments has several disadvantages. Synthesis of such bead populations can be complex, especially because split-and-pool steps are used. It can also be difficult to ensure random distribution of beads into compartments, because the beads can settle or aggregate, leading to a distribution that does not follow Poisson statistics. To achieve a more random distribution of beads, a bead slurry may need to be continuously stirred, or compartmentalization may be performed quickly to minimize settling of beads.

Pre-dispensing clonally tagged primers to into micro-compartments has a disadvantage of procedural complexity. Primers must be separately synthesized with different tags, and copies of differently tagged primers would have to be dispensed into different micro-wells. This would involve use of a special robotic device. It may be feasible to distribute tagged primers into hundreds or thousands of micro-wells, but it would be difficult to achieve this for larger numbers of compartments (e.g. millions).

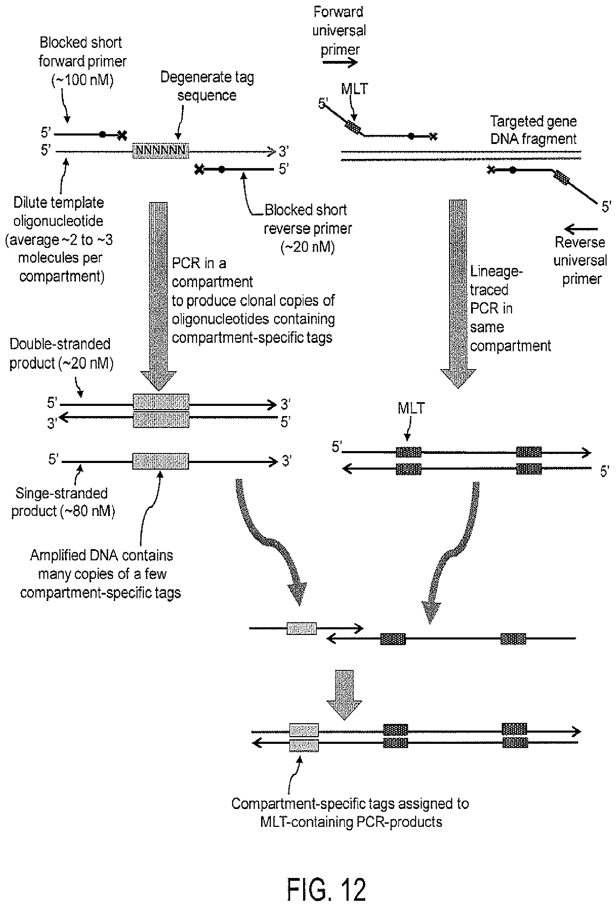

Methods and compositions are disclosed that deliver clonally tagged oligonucleotides to micro-compartments without requiring attachment of the oligonucleotides to a surface (such as beads or a micro-well wall). Use of oligonucleotides in solution is advantageous because it ensures more even distribution of tags into compartments and is very simple to implement. The scheme is outlined in Figure 12.

Figure 12 shows an in-solution method for delivering clonally tagged oligonucleotides into micro-compartments, which can function as primers to add compartment-specific tags to PCR products that are co-amplified with the same reaction volume. A template oligonucleotide containing a degenerate tag sequence can be added to a PCR cocktail such that when the PCR cocktail is compartmentalized, a small number of individual template oligonucleotide molecules (for example, an average of ∼2 to ∼3 molecules) are partitioned into each compartment. Primers capable of amplifying the template oligonucleotide are also included in the reaction cocktail. Thus, when PCR is carried out, a small number of template oligonucleotides within each compartment are amplified to produce many copies containing a few clonal compartment-specific tags. These clonally tagged oligonucleotides can be used as primers to assign compartment- specific tags to other PCR products that are co-amplified within the same reaction volume (for example, via lineage-traced PCR of multiple genomic regions).

In one implementation, many copies of a uniformly tagged oligonucleotide sequence can be produced in a compartment by introducing a single molecule of that tagged DNA sequence into the compartment and then copying and amplifying it within the compartment using short primers (via PCR). By starting with a single tagged DNA molecule as a template, the amplified copies within the compartment would be clonal, harboring the same tag as the template molecule. In one implementation, the tagged template DNA can be double stranded. In another implementation, the template DNA can be single-stranded, consisting of either the top or bottom complementary strand. In one implementation, tag (or barcode) sequences within a population of template molecules can be generated by incorporating degenerate positions during oligonucleotide synthesis (e.g., by incorporating multiple "N" positions, were N denotes an approximately equal probability of coupling a T, C, G, or A base). In one implementation, pre-defined barcodes can also be incorporated into the template molecules. In one implementation, more than one differently tagged molecule can be used as a template within a compartment, in which case the amplified oligonucleotides within a compartment would contain more than one tag sequence. In certain implementations, to minimize the number of compartments containing no tagged template molecule, an average of two or three differently tagged template molecules can be introduced into a compartment (distributed according to Poisson statistics). In one implementation, the resulting amplified clonally tagged oligonucleotide copies within a compartment can function as primers by hybridizing to and copying other DNA sequences within the compartment. In one implementation, such primers can be used to assign compartment-specific tags to the amplification products within a compartment. If primers containing more than one compartment-specific tag (barcode) are present within a compartment, the combination of tags can be used to identify the amplification products as being derived from a given compartment. In one implementation, an unequal concentration of forward and reverse short primers can be used to amplify a tagged template molecule within a compartment. In one implementation, a forward primer can be two-fold to 20-fold more concentrated than a reverse primer (or vice versa). Use of primers of unequal concentration leads to "asymmetric PCR", producing more copies of one amplified strand than its complement. In one implementation, such asymmetric amplification can promote hybridization of the amplified clonally tagged oligonucleotides with other DNA sequences in the compartment (thus allowing the amplified oligonucleotides to function as tagged primers). Figure 12 illustrates this approach.

This method to introduce many copies of a clonally tagged oligonucleotide sequence into a reaction compartment has many potential applications. In one implementation, it can be used to aid in measurement of low-abundance mutant DNA molecules as described above. In another implementation, it can be used to tag amplified DNA products from single cells in different compartments to generate single-cell genomic data. In another implementation, the method can be used to label copies of complementary DNA (cDNA) from single cells in different compartments to facilitate high-throughput RNA profiling of single cells. In another implementation, the method can be used to assign the same tag to multiple amplicons derived from a larger chromosomal fragment within a compartment, in order to facilitate genomic sequence assembly.

In another implementation, the compartment-specific DNA tagging method can be used to facilitate highly multiplexed single cell proteomics. In this approach, antibodies targeting different proteins can be labeled with oligonucleotides containing an antibody-specific barcode sequence flanked by common primer binding sequences. A multiplexed panel of antibodies can be bound to proteins on the surface of intact cells or inside fixed and permeabilized cells. Each antibody in the panel is labeled with an oligonucleotide containing a different antibody-specific tag. After washing away excess antibodies, cells can be compartmentalized (for example into aqueous droplets in oil or into micro-wells on a microfluidic device) such that each compartment is unlikely to contain more than one cell. Common PCR primers within the compartments could be used to simultaneously amplify all antibody-bound barcoded oligonucleotides via common primer binding sequences. The relative abundance of an amplified tag within a compartment would reflect the relative abundance of the corresponding antibody bound to its protein target within the cell. Compartment-specific barcodes could then be introduced to enable quantitation of proteins in different single cells. Since a large variety of antibody-specific tags can be created, the multiplexing capacity for different antibodies is virtually limitless.
More generally, the described method can be used for any application in which nucleic acid molecules within a compartment need to be labeled with a compartment-specific tag.

### EXAMPLES

The present technology may be better understood by reference to the following examples. These examples are intended to be representative of specific implementations.

### Example 1

This example describes application of a high-throughput RNA quantification method. The method enables up-front parallelization of multiple RNA-containing samples in order to simplify and reduce the cost of downstream sample processing and analysis.

### Materials and Methods

### Modular synthesis of RT primer mixes:

A two-stage modular oligonucleotide synthesis strategy was employed to create mixtures of primers, with each mixture having a distinct sample-specific barcode in the 5'-segment and uniform proportions of multiple target-specific sequences in the 3'-segment (Figure 1a). First, several target-specific 3'-segments were made on separate oligonucleotide synthesis columns. Synthesis was carried out using standard phosphoramidite chemistry in the 3' to 5' direction on 40 nanomole polystyrene support columns (Prime Synthesis, Aston, PA) using a Dr. Oligo 192 automated synthesizer. The synthesis was paused after oligomerization of the 3'-segments was complete, and partially synthesized oligonucleotides were left on the polystyrene supports in the protected state with the dimethoxytrityl (DMT) group still on.

Argon gas was blown through the columns to dry the polystyrene supports, and then the columns were cut open and the polystyrene powder was poured into a common glass vial. The particles were suspended in a 2:1 to 3:1 mixture of dichloromethane:acetonitrile that was titrated to make the polystyrene neutrally buoyant. The slurry was constantly agitated to ensure uniform mixing while a pipette was used to dispense equal volumes of the slurry into fresh synthesis columns (with the bottom frit in place). The columns were then flushed with acetonitrile, allowing all polystyrene particles to settle to the bottom. After the acetonitrile had fully drained out by gravity, the top frits were put in place to secure the powder into the columns. One column was made for each sample-specific barcode.

The new columns were placed back on the automated synthesizer for continuation of synthesis. A distinct barcode sequence, as shown in Table 6, below, was assigned to each column for incorporation into the 5'-segment of the primer mix. Barcodes were designed to be eight nucleotides in length, with each barcode differing from all other barcodes in the set at a minimum of two positions (to minimize the probability of misclassification caused by sequencer errors). A universal PCR primer binding sequence was also added to the 5'-segment of each oligonucleotide mixture. The synthesizer was programmed with an additional "dummy base" at the 3'-terminus to account for the partially synthesized oligonucleotides already present on the polystyrene supports.

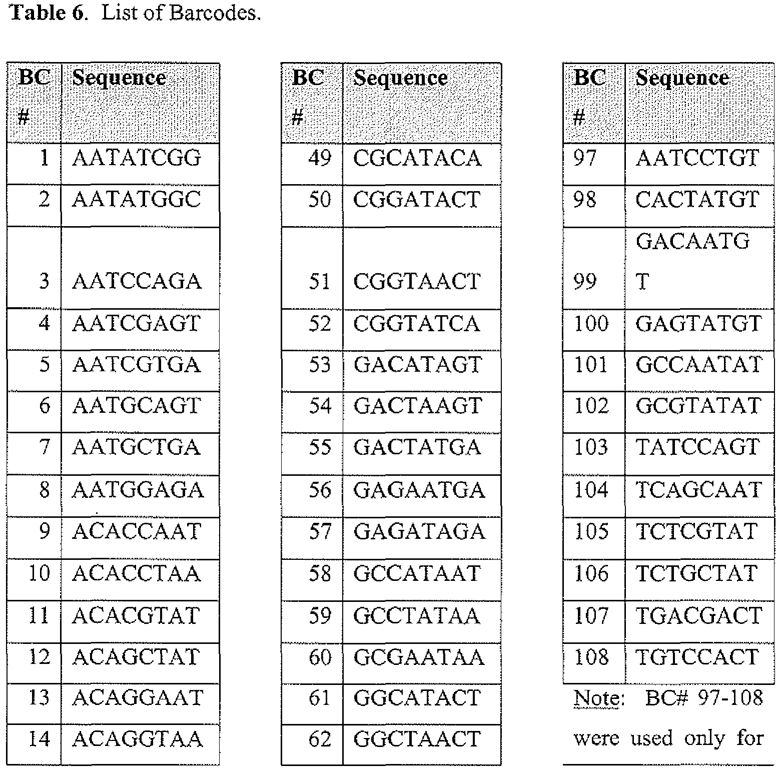

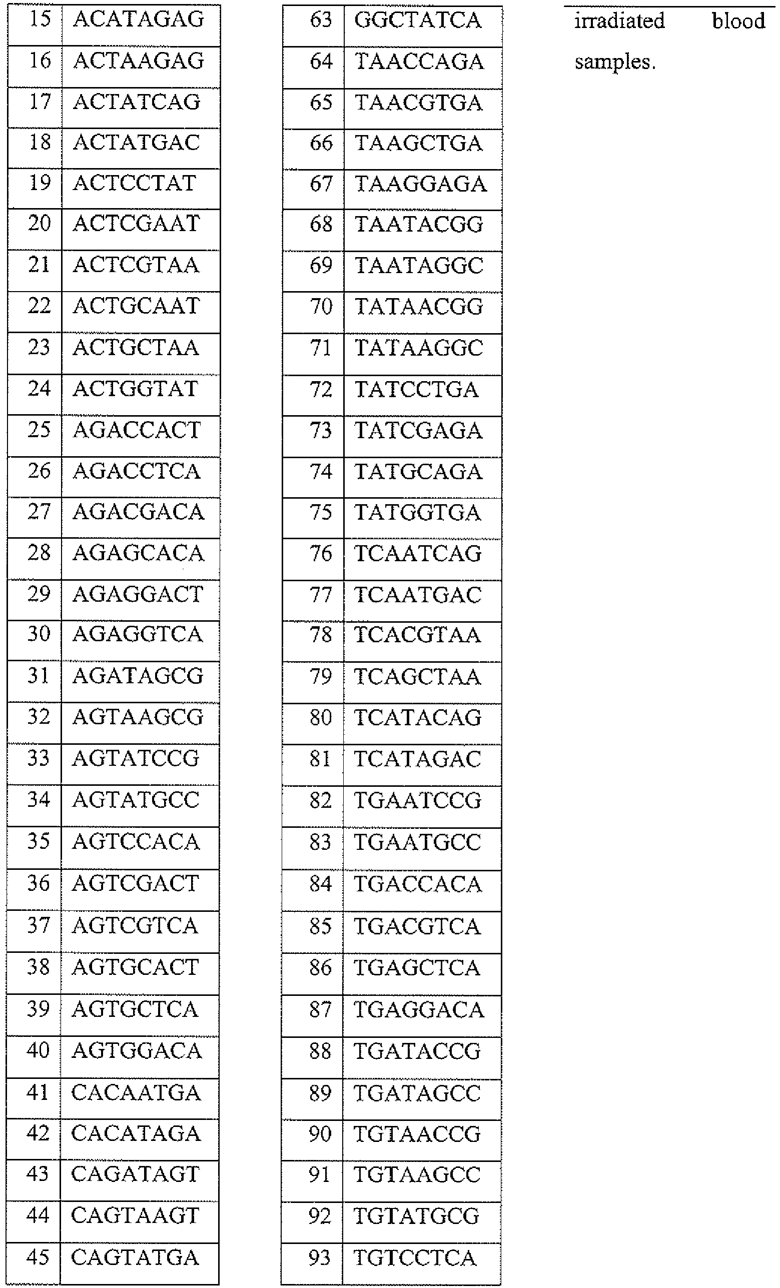

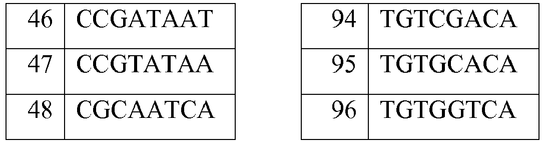

Upon completion of the second stage of the modular synthesis, the oligonucleotide mixtures were cleaved from the polystyrene supports with the DMT group left on. Each mixture was subjected to rapid deprotection followed by purification on a separate Glen-Pak DNA reverse-phase cartridge (Glen Research, Sterling, VA). The cartridge selectively retained the hydrophobic DMT group at the 5'-end of the completed oligonucleotides, enriching for full-length products. The DMT group was removed upon completion of purification. The purified oligonucleotide mixtures were then dried and resuspended in 10 mM Tris (pH 7.6) to create 10x working stocks. Sequences of miRNA and mRNA modular primer segments are listed in Tables 3, 5, and 8, below.

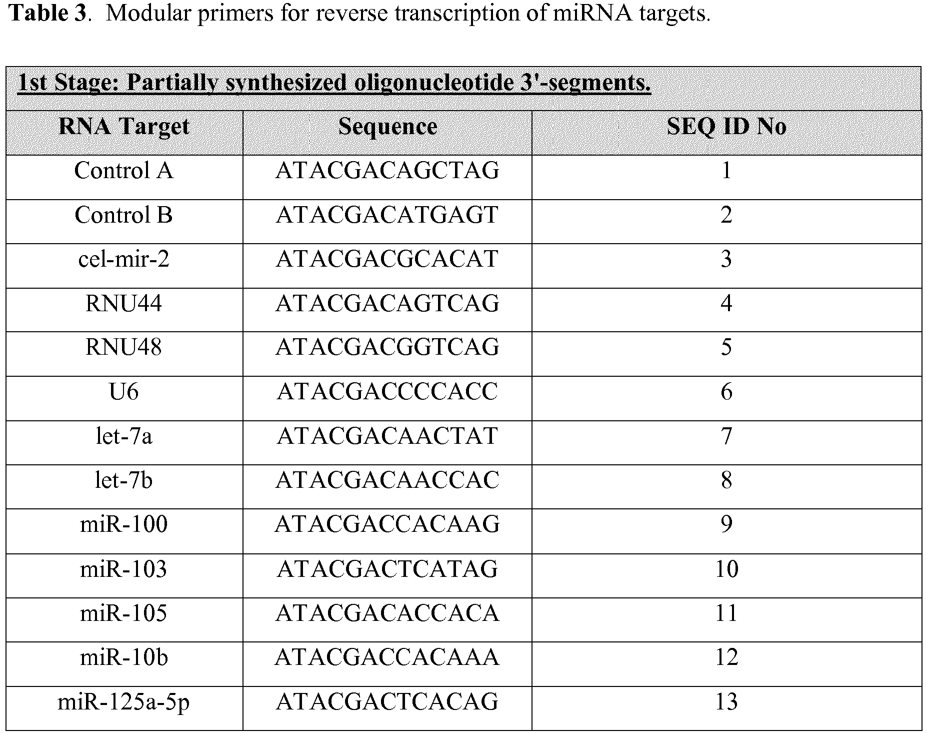

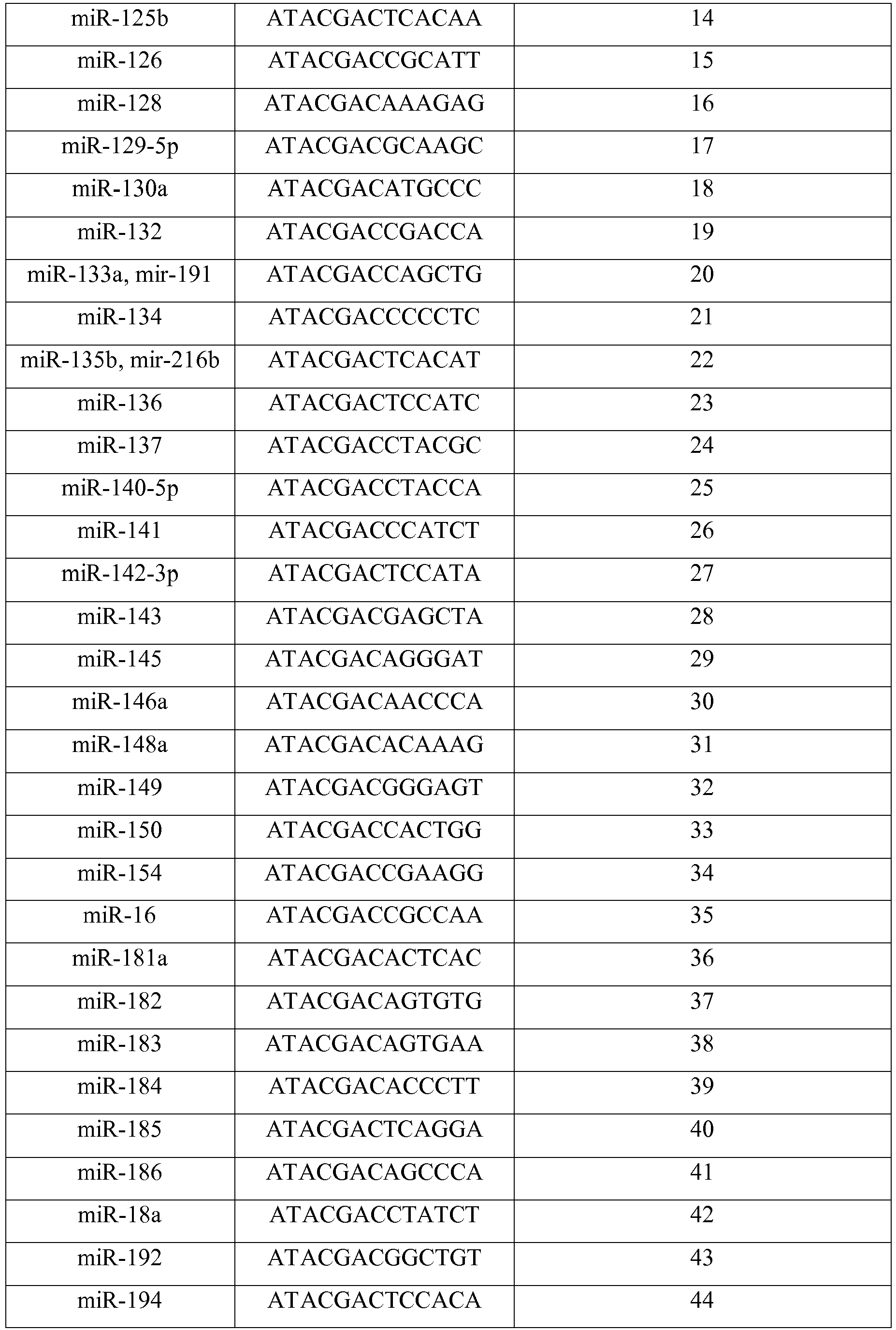

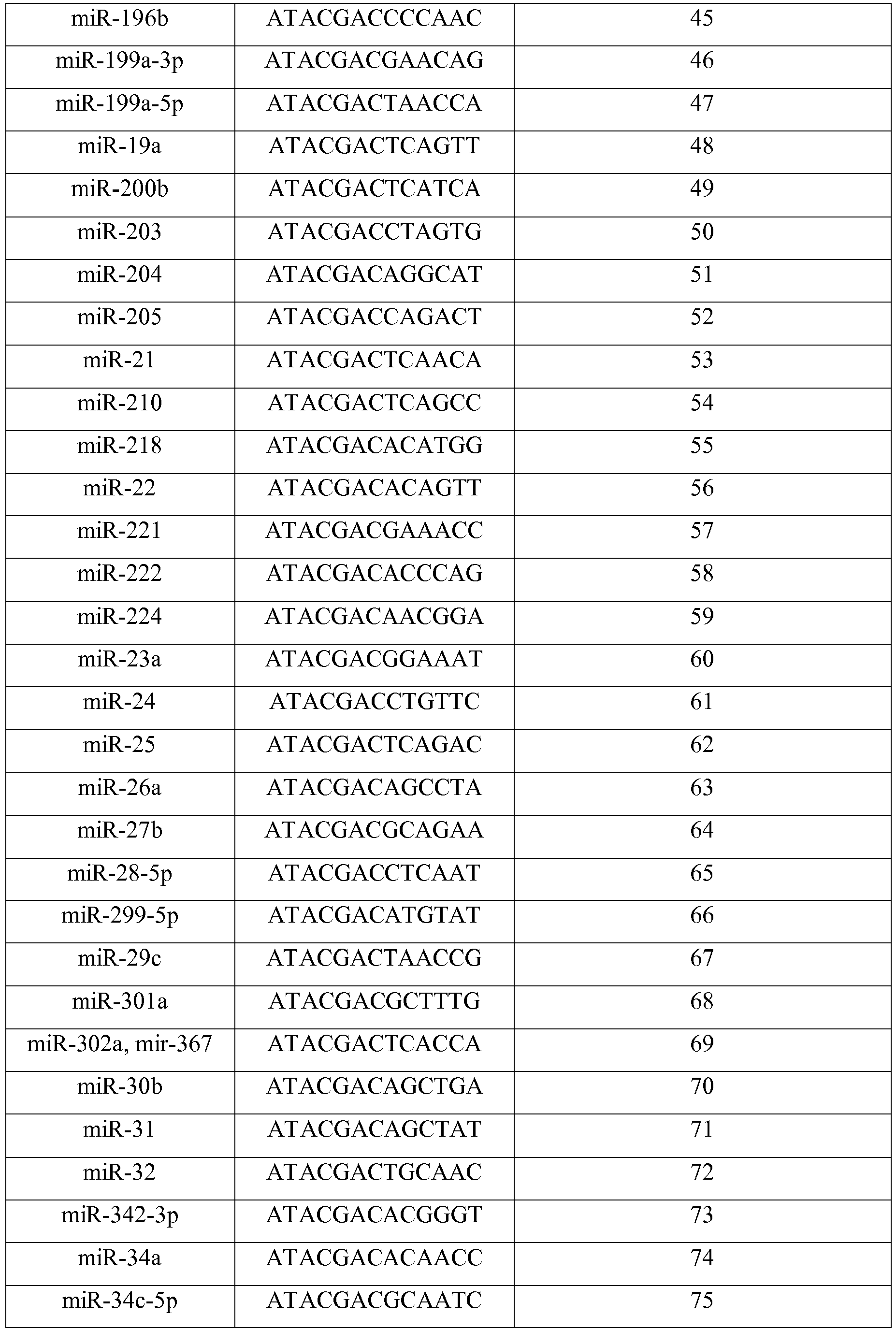

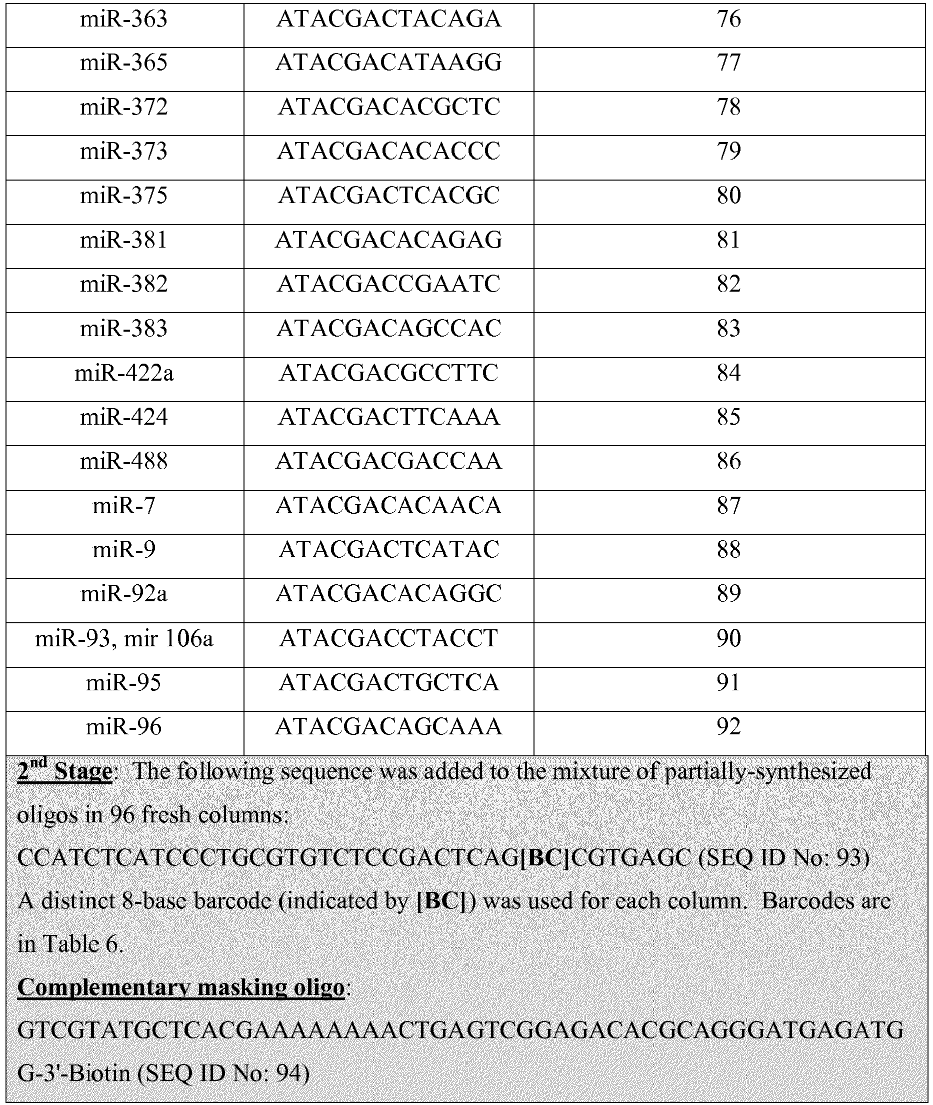

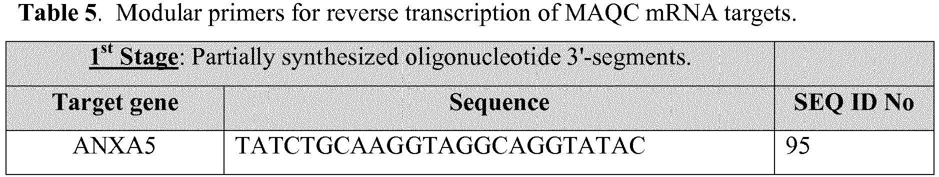

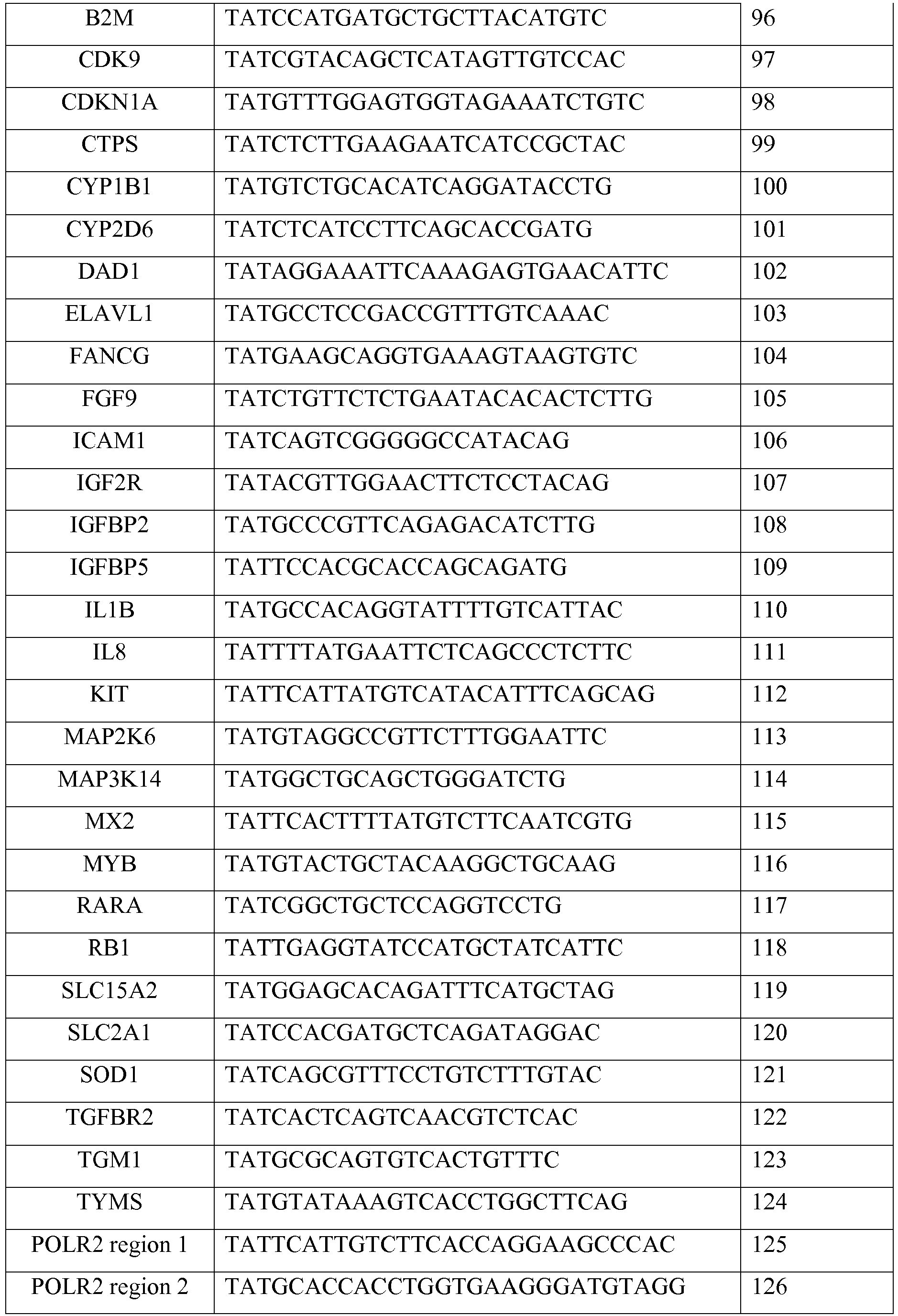

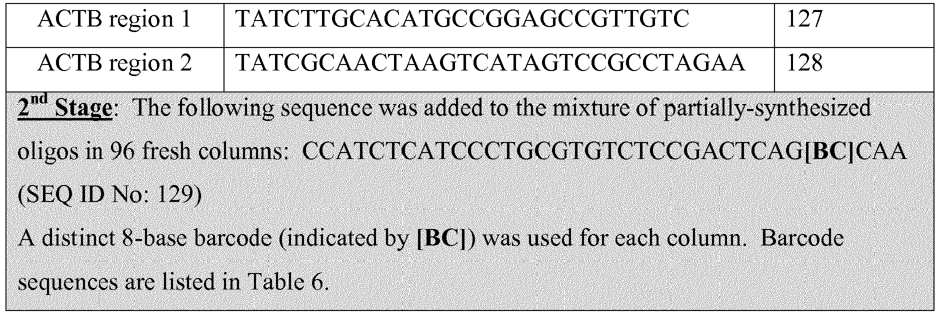

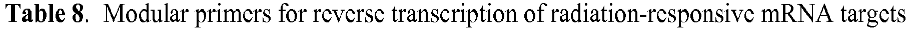

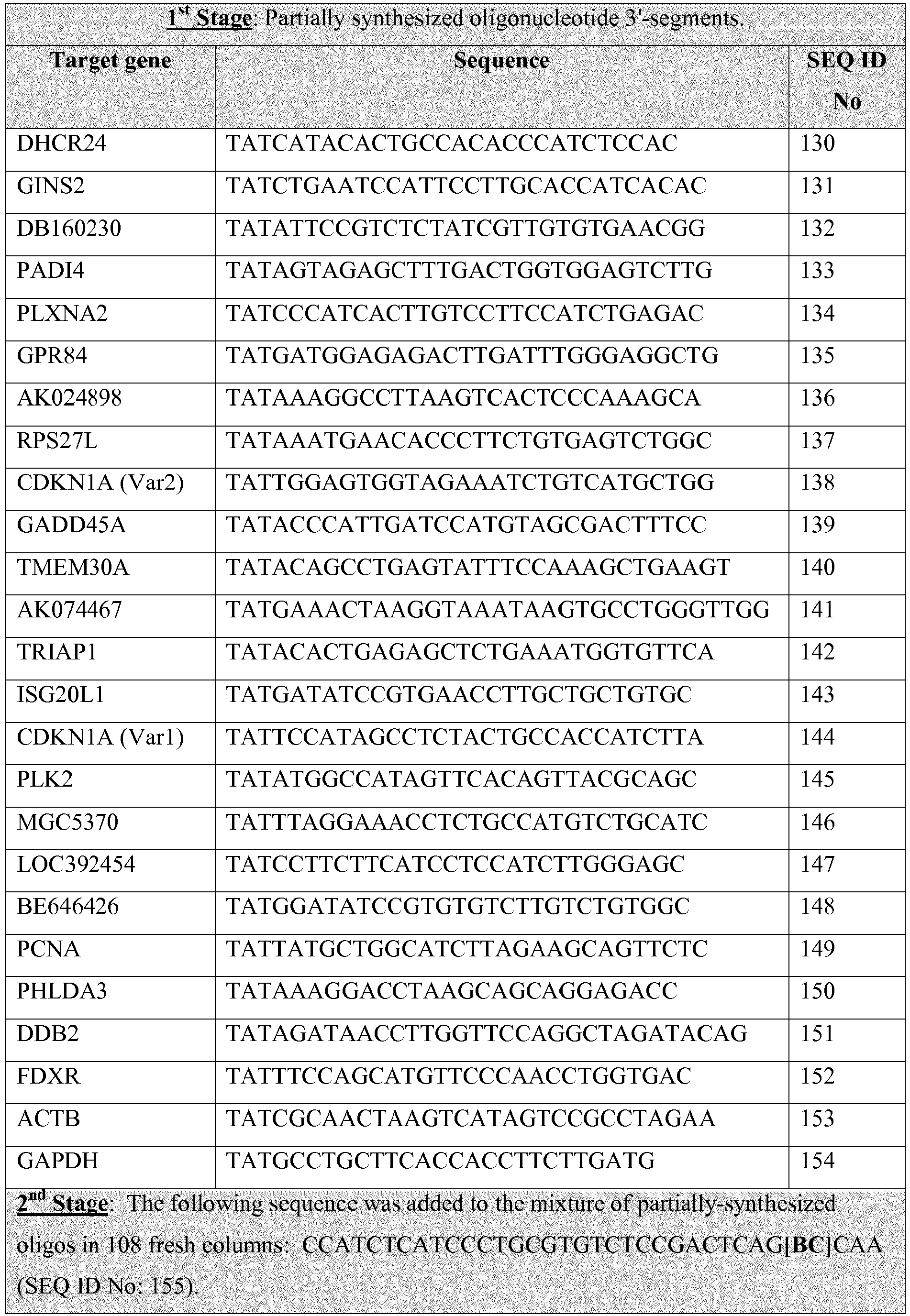

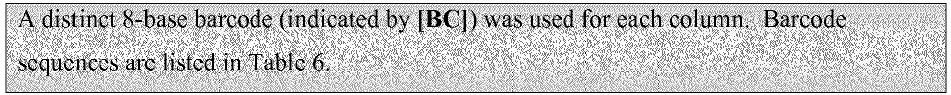

### Preparation of synthetic RNA samples:

RNA oligonucleotides comprised of 90 microRNA and 6 control RNA sequences, shown in Table 2, below, were synthesized at a 40 nmole scale with 2'-deprotection and purification at the Yale Keck oligonucleotide synthesis core facility. A Tecan Freedom Evo 200 robotic liquid handler was programmed to dispense pre-defined amounts of each RNA into the wells of a 96-well plate to achieve final concentrations ranging from 4 to 0.08 nM in a pattern designed to produce an image of a rose on a heat map. The RNAs were dissolved in a buffer containing 10mM Tris (pH 7.6), 0.1 mM EDTA, and 300 ng/mL carrier RNA (Qiagen) in RNAse-free water. The synthetic RNA solutions were stored at -80°C until needed for RT.

**Table 2. Synthetic RNA oligonucleotides.**

| **Synthetic RNA** | **Sequence** | **SEQ ID No** |
|---|---|---|
| Control A | GUUACUUAUGAGAGUGGCUAGCU | 156 |
| Control B | UGAUCAUAUCCUGUGCACUCAU | 157 |
| cel-mir-2 | UAUCACAGCCAGCUUUGAUGUGC | 158 |
| RNU44 | GAAGGUCUUAAUUAGCUCUAACUGACU | 159 |
| RNU48 | CCAUCACCGCAGCGCUCUGACC | 160 |
| U6 | GGCAUCUCGAGCUAAUCUGGUGGG | 161 |
| let-7a | UGAGGUAGUAGGUUGUAUAGUU | 162 |
| let-7b | UGAGGUAGUAGGUUGUGUGGUU | 163 |
| miR-100 | AACCCGUAGAUCCGAACUUGUG | 164 |
| miR-103 | AGCAGCAUUGUACAGGGCUAUGA | 165 |
| miR-105 | UCAAAUGCUCAGACUCCUGUGGU | 166 |
| miR-106a | AAAAGUGCUUACAGUGCAGGUAG | 167 |
| miR-10b | UACCCUGUAGAACCGAAUUUGUG | 168 |
| miR-125a-5p | UCCCUGAGACCCUUUAACCUGUGA | 169 |
| miR-125b | UCCCUGAGACCCUAACUUGUGA | 170 |
| miR-126 | UCGUACCGUGAGUAAUAAUGCG | 171 |
| miR-128 | UCACAGUGAACCGGUCUCUUU | 172 |
| miR-129-5p | CUUUUUGCGGUCUGGGCUUGC | 173 |
| miR-130a | CAGUGCAAUGUUAAAAGGGCAU | 174 |
| miR-132 | UAACAGUCUACAGCCAUGGUCG | 175 |
| miR-133a | UUUGGUCCCCUUCAACCAGCUG | 176 |
| miR-134 | UGUGACUGGUUGACCAGAGGGG | 177 |
| miR-135b | UAUGGCUUUUCAUUCCUAUGUGA | 178 |
| miR-136 | ACUCCAUUUGUUUUGAUGAUGGA | 179 |
| miR-137 | UUAUUGCUUAAGAAUACGCGUAG | 180 |
| miR-140-5p | CAGUGGUUUUACCCUAUGGUAG | 181 |
| miR-141 | UAACACUGUCUGGUAAAGAUGG | 182 |
| miR-142-3p | UGUAGUGUUUCCUACUUUAUGGA | 183 |
| miR-143 | UGAGAUGAAGCACUGUAGCUC | 184 |
| miR-145 | GUCCAGUUUUCCCAGGAAUCCCU | 185 |
| miR-146a | UGAGAACUGAAUUCCAUGGGUU | 186 |
| miR-148a | UCAGUGCACUACAGAACUUUGU | 187 |
| miR-149 | UCUGGCUCCGUGUCUUCACUCCC | 188 |
| miR-150 | UCUCCCAACCCUUGUACCAGUG | 189 |
| miR-154 | UAGGUUAUCCGUGUUGCCUUCG | 190 |
| miR-16 | UAGCAGCACGUAAAUAUUGGCG | 191 |
| miR-181a | AACAUUCAACGCUGUCGGUGAGU | 192 |
| miR-182 | UUUGGCAAUGGUAGAACUCACACU | 193 |
| miR-183 | UAUGGCACUGGUAGAAUUCACU | 194 |
| miR-184 | UGGACGGAGAACUGAUAAGGGU | 195 |
| miR-185 | UGGAGAGAAAGGCAGUUCCUGA | 196 |
| miR-186 | CAAAGAAUUCUCCUUUUGGGCU | 197 |
| miR-18a | UAAGGUGCAUCUAGUGCAGAUAG | 198 |
| miR-191 | CAACGGAAUCCCAAAAGCAGCUG | 199 |
| miR-192 | CUGACCUAUGAAUUGACAGCC | 200 |
| miR-194 | UGUAACAGCAACUCCAUGUGGA | 201 |
| miR-196b | UAGGUAGUUUCCUGUUGUUGGG | 202 |
| miR-199a-3p | CCCAGUGUUCAGACUACCUGUUC | 203 |
| miR-199a-5p | ACAGUAGUCUGCACAUUGGUUA | 204 |
| miR-19a | UGUGCAAAUCUAUGCAAAACUGA | 205 |
| miR-200b | UAAUACUGCCUGGUAAUGAUGA | 206 |
| miR-203 | GUGAAAUGUUUAGGACCACUAG | 207 |
| miR-204 | UUCCCUUUGUCAUCCUAUGCCU | 208 |
| miR-205 | UCCUUCAUUCCACCGGAGUCUG | 209 |
| miR-21 | UAGCUUAUCAGACUGAUGUUGA | 210 |
| miR-210 | CUGUGCGUGUGACAGCGGCUGA | 211 |
| miR-216b | AAAUCUCUGCAGGCAAAUGUGA | 212 |
| miR-218 | UUGUGCUUGAUCUAACCAUGU | 213 |
| miR-22 | AAGCUGCCAGUUGAAGAACUGU | 214 |
| miR-221 | AGCUACAUUGUCUGCUGGGUUUC | 215 |
| miR-222 | AGCUACAUCUGGCUACUGGGU | 216 |
| miR-224 | CAAGUCACUAGUGGUUCCGUU | 217 |
| miR-23 a | AUCACAUUGCCAGGGAUUUCC | 218 |
| miR-24 | UGGCUCAGUUCAGCAGGAACAG | 219 |
| miR-25 | CAUUGCACUUGUCUCGGUCUGA | 220 |
| miR-26a | UUCAAGUAAUCCAGGAUAGGCU | 221 |
| miR-27b | UUCACAGUGGCUAAGUUCUGC | 222 |
| miR-28-5p | AAGGAGCUCACAGUCUAUUGAG | 223 |
| miR-299-5p | UGGUUUACCGUCCCACAUACAU | 224 |
| miR-29c | UAGCACCAUUUGAAAUCGGUUA | 225 |
| miR-301a | CAGUGCAAUAGUAUUGUCAAAGC | 226 |
| miR-302a | UAAGUGCUUCCAUGUUUUGGUGA | 227 |
| miR-30b | UGUAAACAUCCUACACUCAGCU | 228 |
| miR-31 | AGGCAAGAUGCUGGCAUAGCU | 229 |
| miR-32 | UAUUGCACAUUACUAAGUUGCA | 230 |
| miR-342-3p | UCUCACACAGAAAUCGCACCCGU | 231 |
| miR-34a | UGGCAGUGUCUUAGCUGGUUGU | 232 |
| miR-34c-5p | AGGCAGUGUAGUUAGCUGAUUGC | 233 |
| miR-363 | AAUUGCACGGUAUCCAUCUGUA | 234 |
| miR-365 | UAAUGCCCCUAAAAAUCCUUAU | 235 |
| miR-367 | AAUUGCACUUUAGCAAUGGUGA | 236 |
| miR-372 | AAAGUGCUGCGACAUUUGAGCGU | 237 |
| miR-373 | GAAGUGCUUCGAUUUUGGGGUGU | 238 |
| miR-375 | UUUGUUCGUUCGGCUCGCGUGA | 239 |
| miR-381 | UAUACAAGGGCAAGCUCUCUGU | 240 |
| miR-382 | GAAGUUGUUCGUGGUGGAUUCG | 241 |
| miR-383 | AGAUCAGAAGGUGAUUGUGGCU | 242 |
| miR-422a | ACUGGACUUAGGGUCAGAAGGC | 243 |
| miR-424 | CAGCAGCAAUUCAUGUUUUGAA | 244 |
| miR-488 | UUGAAAGGCUAUUUCUUGGUC | 245 |
| miR-7 | UGGAAGACUAGUGAUUUUGUUGU | 246 |
| miR-9 | UCUUUGGUUAUCUAGCUGUAUGA | 247 |
| miR-92a | UAUUGCACUUGUCCCGGCCUGU | 248 |
| miR-93 | CAAAGUGCUGUUCGUGCAGGUAG | 249 |
| miR-95 | UUCAACGGGUAUUUAUUGAGCA | 250 |
| miR-96 | UUUGGCACUAGCACAUUUUUGCU | 251 |

### Tissue and cell line RNA samples:

The First Choice Human Total RNA Survey Panel (Ambion) was used as the source of total RNA from 20 normal human tissues. MAQC reference samples consisted of the Stratagene Universal Human Reference RNA (composed of total RNA from 10 human cell lines), and the Ambion First Choice Human Brain Reference RNA.

### RNA from irradiated blood samples:

Peripheral blood was collected in tubes containing sodium citrate after obtaining informed consent from 18 healthy volunteers under approval of the Human Investigation Committee at Yale University. Blood was divided into 2 mL aliquots and subjected to 0, 0.1, 0.5, 2, 4, or 8 Gy of X-irradiation at a dose rate of 1.79 Gy per minute within 1 hour of blood draw. Blood was then incubated for 24 hours at 37°C after addition of an equal volume of RPMI 1640 medium containing 10% fetal bovine serum. Peripheral blood mononuclear cells were isolated using ficoll gradient centrifugation, and total RNA was prepared from these cells using an RNeasy Mini Kit (Qiagen).

### Processing of miRNA samples:

In the first step of the method, multiple RNA targets were reverse-transcribed in a single tube for each sample. The RT primer mix used for a given sample had a sample-specific tag in the 5'-segment, and consistent ratios of multiple target-specific primer sequences in the 3'-segment, shown in Table 3. Primers were designed to hybridize to six nucleotides at the 3'-end of the short miRNA (and control RNA) targets. A 5'-biotin labeled oligonucleotide was annealed to adjacent complementary common primer sequences to stabilize the short RNA/primer heteroduplex by extending base stacking.

Each reverse transcription cocktail consisted of 5 µM tagged primer mix (∼50 nM of each target-specific primer), 7.5 µM biotin-labeled oligonucleotide, 1 x RT buffer, 3 mM MgCl₂, 250 µM each dNTP, 5 mM dithiothreitol (DTT), 30 ng/µL carrier RNA (Qiagen), template RNA, and 5 units/µL Multiscribe reverse transcriptase (Life Technologies) in RNAse-free water. Each RT was carried out in a final volume of 10 µL. Prior to addition of template RNA, DTT, and reverse transcriptase, the biotin-labeled oligonucleotide was annealed to the primer mix by heating the cocktail to 95° C for 2 minutes and then cooling to room temperature. The final assembled RT cocktail was subjected to 40 cycles of 16° C for 2 minutes, 42° C for 1 minute, and 50° C for 1 second. Reactions were terminated by heating to 65° C for 20 minutes and adding EDTA at a final concentration of 10 mM. Products of all separate RT reactions were then combined into a single volume.

Pooled cDNAs were purified by capture of the complementary biotin-labeled oligonucleotide using high capacity streptavidin-coated agarose resin (Thermo Scientific) (5µL resin slurry added per 10 µL RT reaction). Resin particles were kept suspended in the solution by slowly turning the tubes end-over-end at room temperature for at least two hours to promote biotin binding. Particles were then washed in buffer containing 10 mM Tris pH 7.6 and 50 mM NaCl. cDNAs were released from the resin-bound oligos into a fresh volume of the same buffer (twice the volume of resin slurry) by heat-denaturation at 95° C for two minutes. To remove un-extended RT primers, a second round of selective annealing, capture, washing, and elution was performed using a mix of biotin-labeled oligonucleotides complementary to primer-extended sequences (100 nM each), as shown in Table 4, below.

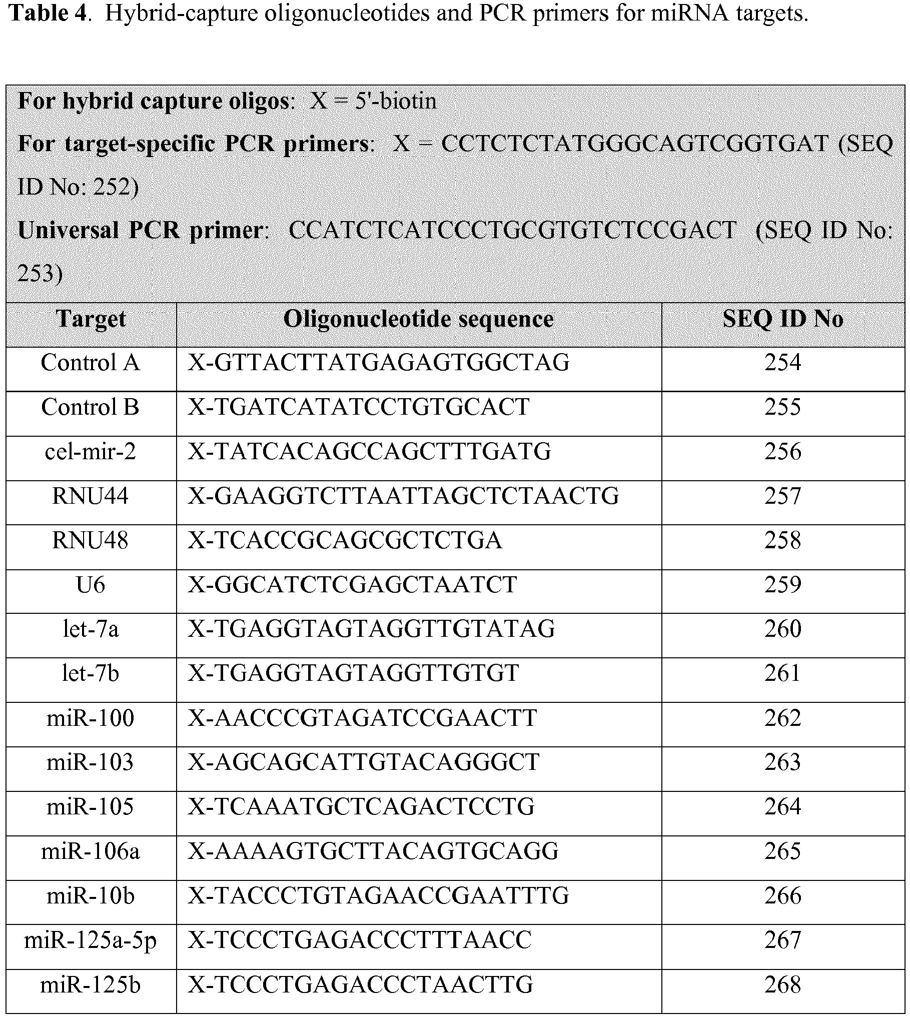

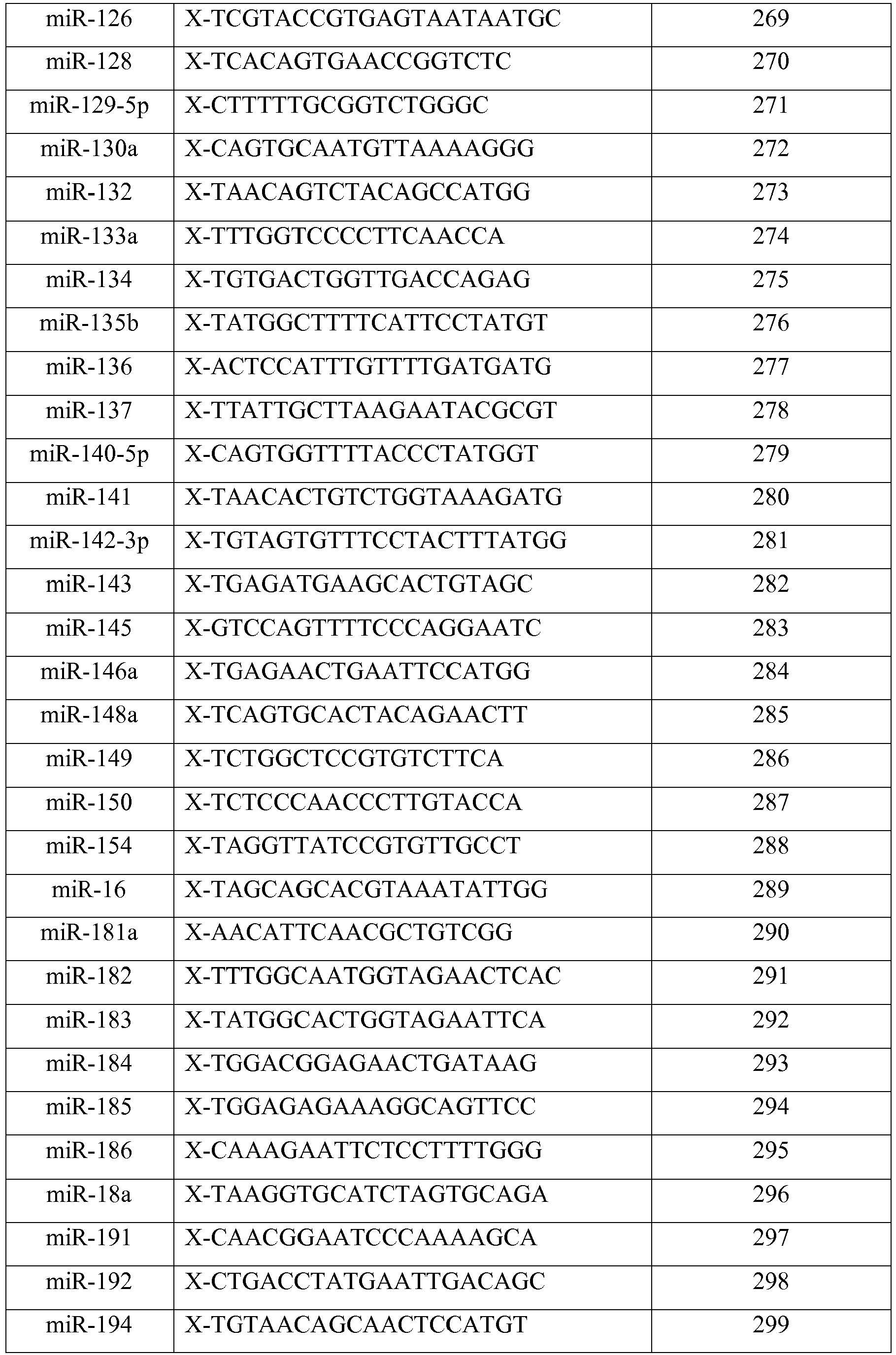

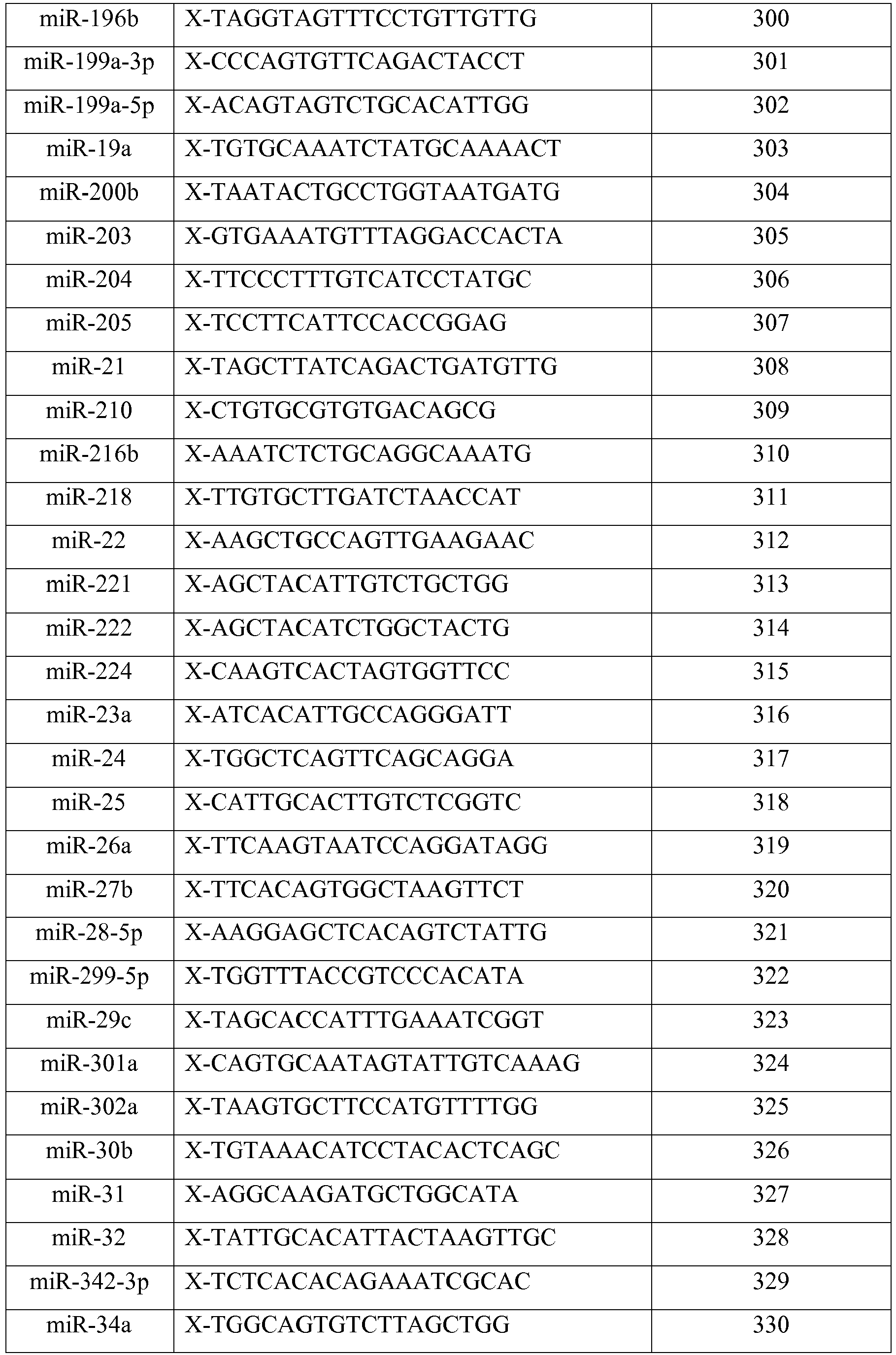

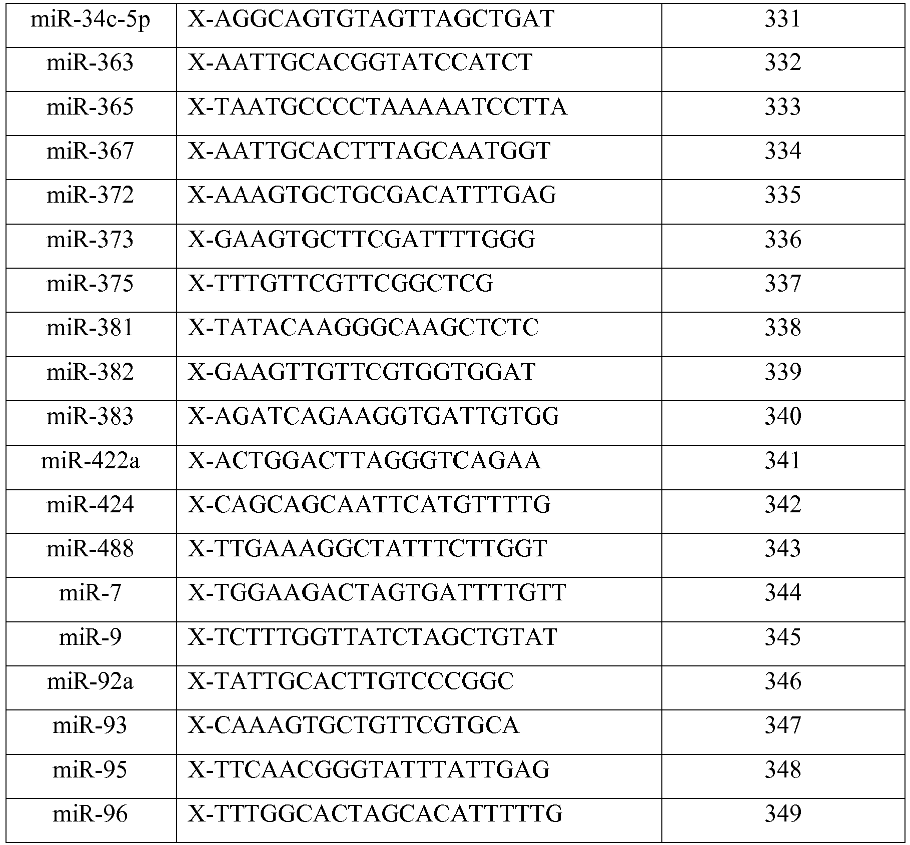

The purified cDNA pool was distributed into 96 separate tubes for single-plex end-point PCR of each cDNA target. Because all sample-specific tags associated with a given target underwent competitive amplification in a single reaction volume, the tag proportions were maintained. The primer pair used in each PCR consisted of a universal forward primer and a distinct target-specific reverse primer as depicted in Figure 1b (Table 4). Sequencing adaptors were incorporated into the 5'-ends of the primers to enable direct sequencing of the PCR products. Each PCR cocktail consisted of a 10 µL volume of 1x AccuPrime PCR Buffer I (which included dNTPs and MgCl₂), 100 nM universal forward primer, 100 nM target-specific reverse primer, 2 µL pooled cDNA template, and 0.2 µL AccuPrime Taq DNA polymerase (Invitrogen). Mineral oil was added to minimize evaporation. Thermal cycling parameters were 94° C for 2 minutes, 60° C for 30 seconds, 72° C for 20 seconds, followed by 40 cycles of 94° C for 20 seconds, 65° C for 30 seconds, and 72° C for 20 seconds. A final extension step was performed at 72° C for 2 minutes followed by cooling to 4° C and addition of EDTA (10 mM final) to terminate polymerase activity.

All PCR volumes were combined, and a 20 µL aliquot of the pooled reaction products was purified on a 2% low-melting point agarose gel. DNA was extracted from the excised gel slice using a QIAquick Gel Extraction Kit (Qiagen). Concentration was estimated using a Bioanalyzer 2100 (Agilent) and adjusted to levels recommended for Ion Torrent emulsion PCR.

### Processing of mRNA samples:

The overall scheme for processing of mRNA samples was the same as that described above for miRNA samples, with a few notable modifications. Because mRNAs were much larger than miRNAs, primers could be designed to amplify ∼100 nucleotide target regions. Accordingly, longer gene-specific RT primers were used (Tables 5 and 8). This enabled RT to be performed at higher temperature with a thermostable polymerase without requiring a complementary biotinylated oligonucleotide to enhance stability via extended base stacking. Each RT reaction was carried out in a 10 µL volume consisting of tagged primer mix (∼50 nM each target-specific primer), 1 x First-Strand buffer, 500 µM each dNTP, 5 mM DTT, template RNA, and 10 units/µL SuperScript III reverse transcriptase (Invitrogen) in RNAse-free water. Primers were annealed to RNA targets by combining at room temperature and then heating to 65° C for five minutes in the absence of buffer, DTT, and polymerase, which were added upon incubation at 55° C for one hour. Reactions were pooled after inactivating the polymerase by heating to 75° C for 20 minutes, 95° C for 1 minute, and adding EDTA (10 mM final).

The absence of a biotin-labeled oligonucleotide during RT enabled capture of cDNAs in a single step using biotinylated oligonucleotides complementary to primer extended sequences (Tables 7 and 9). Pooled and purified cDNA templates were distributed into separate tubes for single-plex end-point PCR of each target using primers listed in Tables 7 and 9. Thermal cycling parameters were identical to those described for miRNAs above, except for use of an annealing temperature of 63° C instead of 60° C for the first cycle.

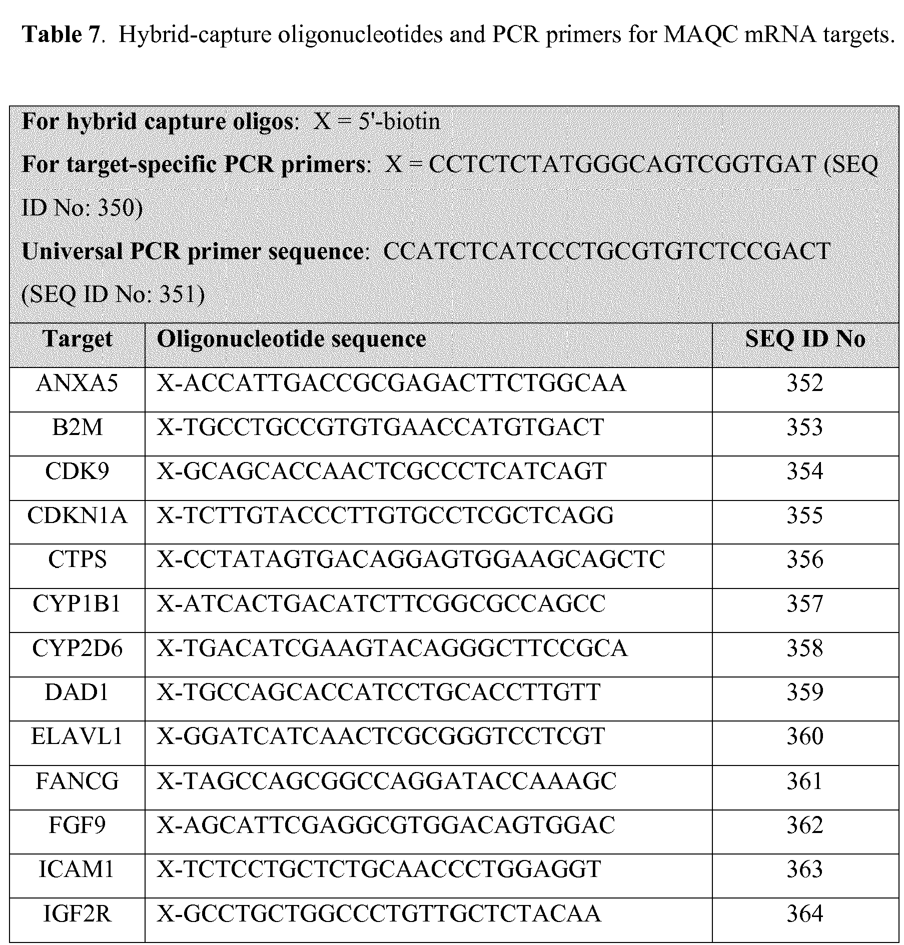

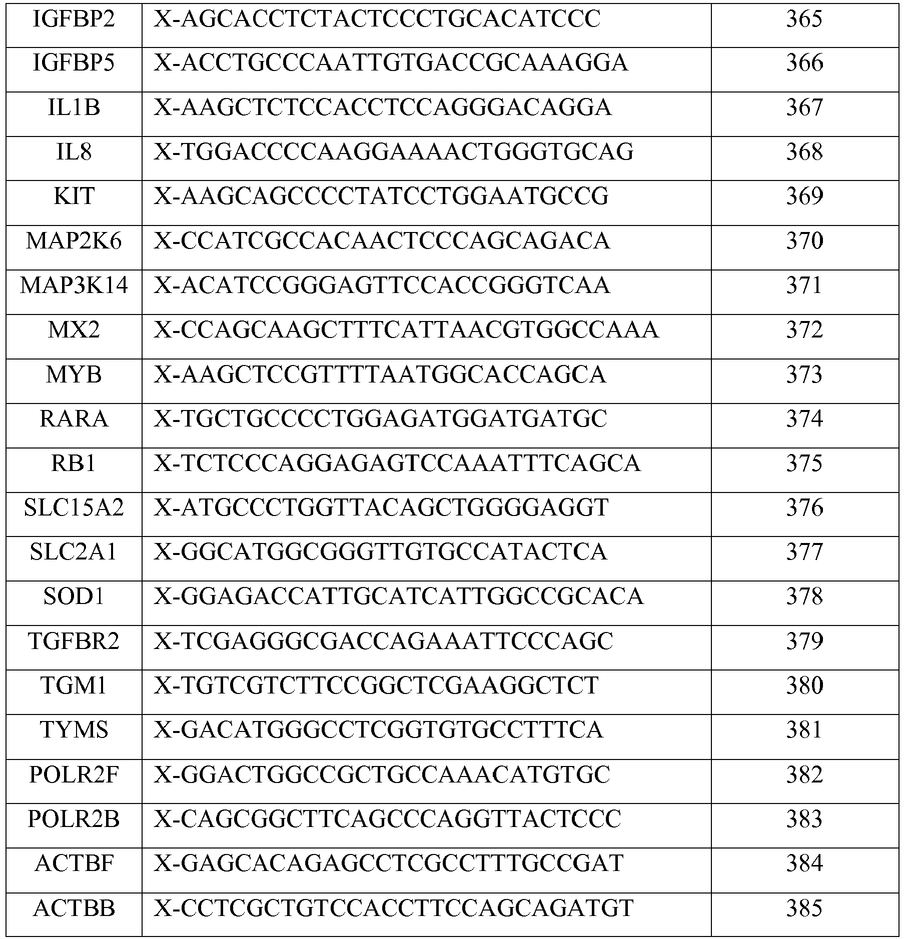

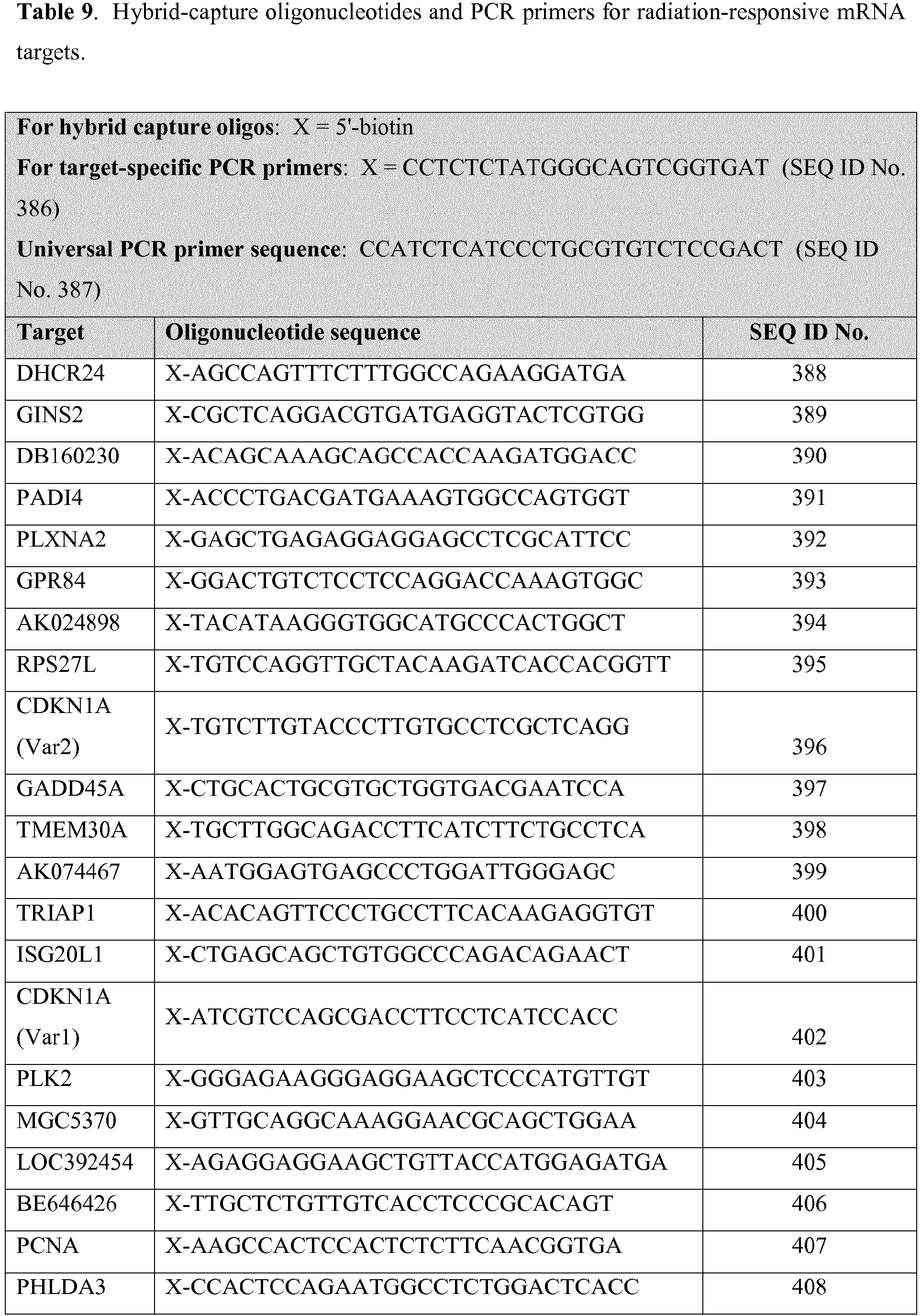

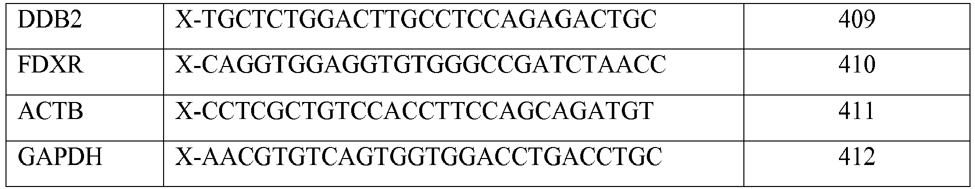

### Next-generation sequencing:

Templates were prepared for Ion Torrent sequencing using the automated Ion OneTouch System (Life Technologies). Gel-purified amplicons were diluted to the concentration recommended by the manufacturer prior to loading on the instrument. Automated emulsion PCR enabled massively parallel clonal amplification onto Ion Sphere Particles (ISPs). To minimize polyclonal ISPs, template dilution was adjusted to achieve between 10% and 30% template-positive ISPs. The OneTouch Enrichment System was used to isolate template-positive ISPs, which were then loaded onto a semiconductor chip for sequencing. Depending on the desired sequence depth, either a 314 low-capacity chip or a 318 high-capacity chip was used. Sequencing was carried out on an Ion Torrent PGM (Life Technologies) using a 200 bp reagent kit.

### Binning and counting of sequences:

To determine the number of reads belonging to each target/barcode bin, the Torrent Mapping Alignment Program (TMAP) provided as part of the TorrentSuite Software (version 4.0) was used. Uploading of three files was necessary for analysis of a given data set: a text file containing user-defined barcodes and adapter sequences, a FASTA format file listing miRNA or mRNA reference sequences, and a BED file defining target regions. After performing alignment of reads to target reference sequences, the coverage analysis plug-in module was run, and the resulting barcode/amplicon coverage matrix was downloaded. This matrix contained read counts for each bin, and could be opened and further manipulated in Microsoft Excel.

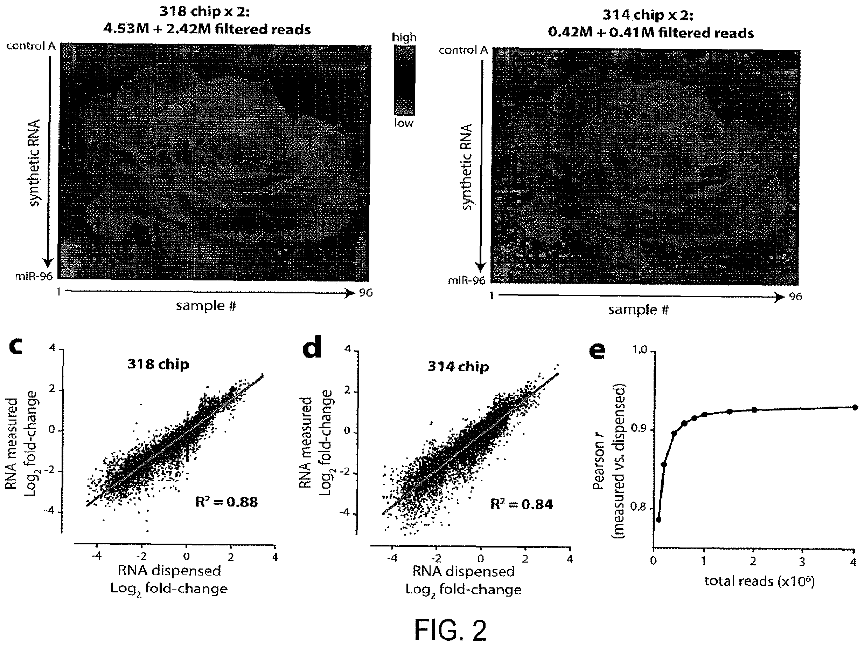

Since down-sampling of sequence data was not possible within the TorrentSuite software, an alternative approach was used to obtain binned counts from defined subsets of reads for Figure 2e. The "countifs" function in Microsoft Excel was exploited for this purpose. An important difference with this approach compared to the TMAP analysis was that only perfect sequence matches were counted. Thus, to minimize the probability of an imperfect match due to sequencer error, short reference sequences of ∼10-12 nucleotides were used. Reference sequences were chosen to extend beyond the sequence contained in any single primer to avoid counting of spurious PCR products (e.g. primer dimers). Care was also taken to ensure that each reference sequence matched only a single target.

### Normalization and standardization of binned sequence counts:

To generate heat maps displaying the rose image in Figures 2a and 2b, counts from two replicate experiments were averaged for each of the 9,216 data bins. Counts were then normalized across rows and columns relative to the known total amounts of dispensed synthetic RNAs. First, counts in a given row were multiplied by the ratio of the sum of counts to the total amount of RNA dispensed in that row. Second, the resulting values in a given column were multiplied by the ratio of the sum of values to the total amount of RNA dispensed in that column. Finally, the binary logarithms of these normalized values were calculated and plotted on a heat map.

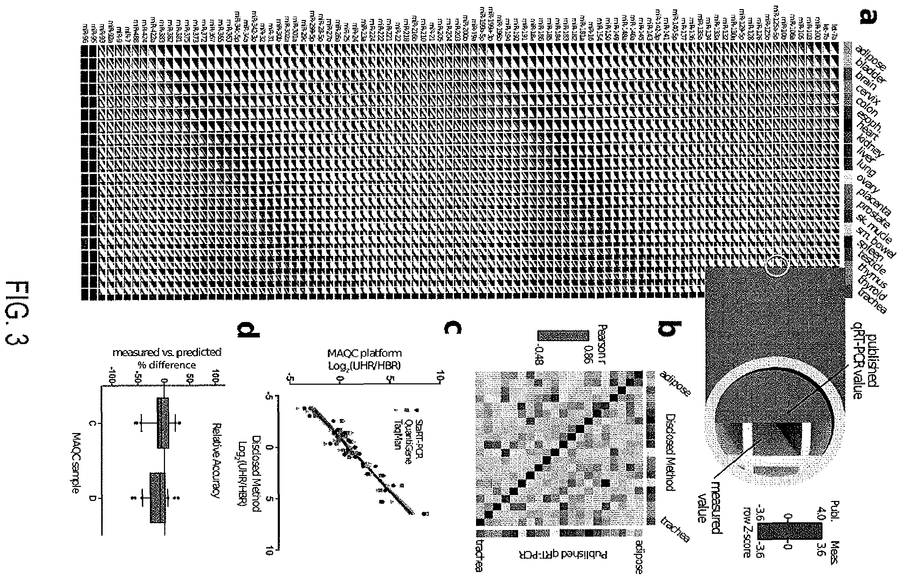

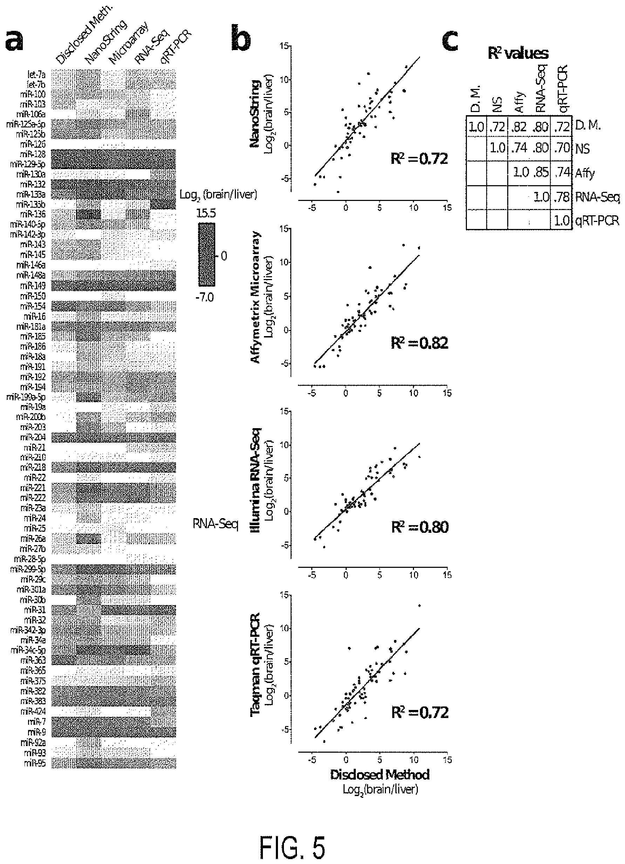

The normalization and standardization of miRNA and mRNA measurements from human tissues and blood samples (Figure 3a, 3b, 5) was performed as described below. First, replicate values were averaged for each data bin. Second, to equalize the total counts produced by different singleplex PCRs for each target, the values across a given row were multiplied by a common factor to make the sum of values in that row equal to 1000. Third, flooring of the data was achieved by adding 0.01 to all bins (thus eliminating 0 values). This was analogous to the common practice in qRT-PCR experiments of transforming Cq values greater than 35 to 35. Fourth, to normalize miRNA levels, the mean expression value for all miRNAs in a given sample was used as the normalization factor. mRNAs from irradiated blood samples were normalized relative to the mean expression values of two housekeeping genes, *ACTB* and *GAPDH.* Fifth, log₁₀(fold-change) values were calculated for all data bins. Sixth, mean centering was performed by subtracting the row average from each value. Finally, values were autoscaled by dividing each value by the standard deviation across the row.

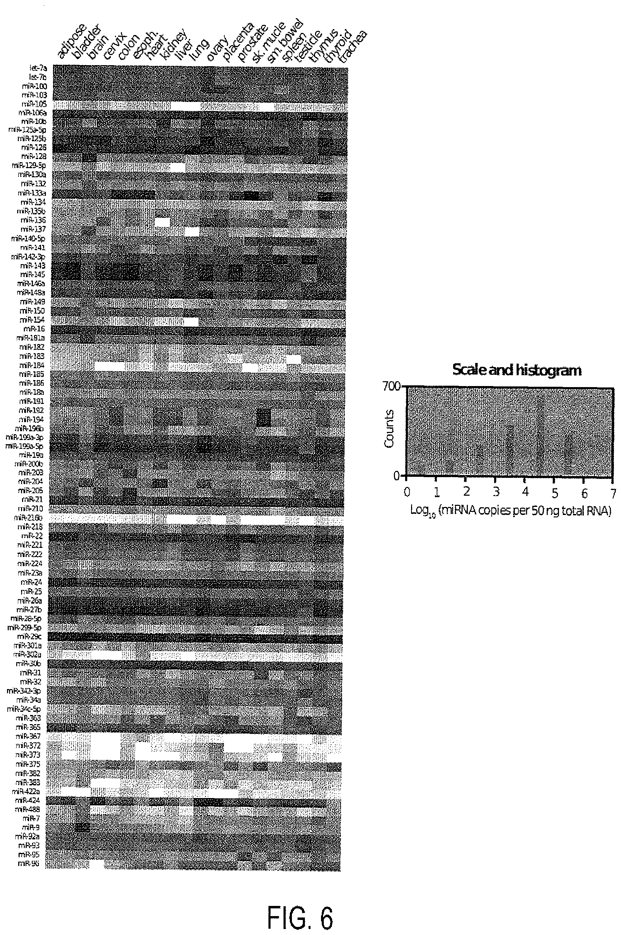

To determine the absolute quantity of miRNAs in normal human tissues (Figure 6), a quantitative reference standard sample containing ∼15,000 copies of each synthetic miRNA was reverse-transcribed and competitively amplified with 50 ng tissue-derived total RNA samples. All samples were analyzed in three technical replicates. Read counts were averaged for the replicates. The average counts for a target in a given tissue sample were divided by the average counts for the same target in the control sample. The resulting value was then multiplied by 15,000, yielding an estimate of the number of miRNA copies per 50 ng total RNA in that tissue sample. Log₁₀-transformed values were plotted on a heat map.

### Plotting of heat maps:

All heat maps were generated without clustering, using Tree View software (downloaded from the website: http://rana.lbl.gov/EisenSoftware.htm). Raw Cq values from published qRT-PCR studies were obtained from the miRNA body map website (www.mirnabodymap.org). The values were floored at 35 and were subjected to the same normalization and standardization steps as outlined above, beginning at the fourth step. Standardized values of published and measured data were plotted on separate heat maps using identical color scale and contrast parameters. Split-pixel maps were created by erasing half of each pixel on one map, and then overlaying it on the second map using Adobe Illustrator and Photoshop.

### Analysis of mRNAs in MAQC samples:

Target genes for mRNA analysis were chosen from among the 48 genes that were commonly tested across all three quantitative (non-microarray) platforms reported in the MAQC data sets. Among these 48 genes, 30 were chosen whose expression was measured at consistent levels (having a low coefficient of variance) across the three platforms. The targeted genes are listed in Table 5.

Binned sequence counts from quadruplicate experiments were averaged for each of the four MAQC samples (A, B, C, and D). The mean counts for a given gene were multiplied by a common factor to make the sum of values for that gene equal to 1000. No flooring was applied. Since only 30 targets were analyzed, normalization relative to the global mean expression level across a sample would not be recommended. Expression values for a given sample were thus normalized relative to average measurements of *POLR2* and *ACTB* reference genes for that sample.

Normalized expression values were used to calculate the fold-change for all 30 genes between the Human Universal Reference RNA (sample A) and the Human Brain Reference RNA (sample B). Relative accuracy was calculated as described in the main text, based on measurements of samples C and D.

### RESULTS

### Assessing accuracy with synthetic RNA mixtures:

The performance of the disclosed RNA profiling method was first tested on mixtures of known amounts of synthetic miRNAs. A representative panel of 90 human miRNAs was chosen from the miRBase registry, with a preference for those discovered earlier and having better-defined biological functions. An additional 6 RNAs were included as controls: three human small nuclear/nucleolar RNA fragments, a *C*. *elegans* miRNA, and two arbitrary sequences not found in nature (Table 2). Each of these synthetic RNA oligonucleotides was dispensed into 96 separate tubes in varying amounts using a robotic liquid handler to achieve final concentrations ranging from four to 0.08 nM in a background of 300 ng/mL poly-A carrier RNA. The RNAs were distributed in a pattern designed to provide a simple visual assessment of the multiplexing capacity and accuracy of the method; when quantified and plotted on a heat map, the RNA mixtures would reproduce an image of a rose.

To enable multiplexed targeted labeling of i RNAs during reverse transcription from *j* samples, it was necessary to create RT primers having *i* x *j* combinations of target-specific sequences attached to sample-specific tags. Moreover, to ensure quantitative consistency, it was critical to reverse-transcribe different samples using uniquely tagged primer mixes having identical ratios of all target-specific sequences. Because simply mixing thousands of individually made primers was impractical and would yield imprecise ratios, a two-stage modular oligonucleotide synthesis strategy was devised (Figure 1a). Synthesis was paused after making 96 partial oligonucleotides, each containing a different target-specific primer sequence at its 3'-end. All polystyrene particles harboring partially synthesized oligonucleotides were thoroughly mixed and dispensed into 96 fresh columns. Synthesis was then resumed, adding a sequence to each column that included a unique sample-specific tag and a universal PCR primer-binding site. Finally, the oligonucleotides were cleaved from their solid supports, deprotected, and cartridge-purified to enrich for full-length products. This approach produced 96 primer mixes (Table 3), each having a unique sample-specific tag in the 5'-segment and a uniform composition of 96 target-specific primer sequences in the 3'-segment. Once made, the primer sets could be used for hundreds of reactions.

In the first step of the disclosed RNA profiling method, all 96 targeted RNAs were simultaneously reverse transcribed in a single well for each sample (Figure 1b). RT primers were designed to hybridize to six nucleotides at the 3'-end of each short miRNA target. Since the ratios of target-specific primer sequences are believed to be similar in all reactions, the proportions of tagged cDNA copies should faithfully reflect the abundance of RNAs in the respective samples. To enhance the specificity and stability of the RNA/DNA interaction, the primer bases not binding to the RNA were masked by annealing a biotinylated oligonucleotide complementary to the common primer sequences; this is also predicted to extend the region of base stacking. Upon completion of RT, tagged cDNAs from all 96 samples were pooled into a single tube and were purified by pull-down of the hybridized biotinylated oligonucleotides using streptavidin-agarose resin. After heat-eluting the cDNAs, a second round of selective hybridization and capture was performed using biotinylated oligonucleotides that were complementary to primer-extended sequences (Table 4).

The cDNA pool was then distributed into the wells of a 96-well plate for amplification of each target by separate end-point PCRs (taken to plateau phase). Importantly, because all tags associated with a given cDNA species were amplified competitively in a single volume, tag ratios encoding RNA abundance were preserved. Incorporation of sequencing adapters at the 5'-ends of the PCR primers (Table 4) enabled the resulting amplicons to be pooled, gel-purified, and directly used as templates for massively parallel sequencing without additional library preparation steps.

The pooled amplicons from all 96 reactions were sequenced on an Ion Torrent PGM using either a low capacity (314) or high capacity (318) chip, yielding an average of 0.42M or 3.48M filtered reads per run, respectively (Table 1). Reads were binned based on their target and tag sequences. The Ion Torrent TMAP coverage analysis module was used to generate a table of read counts for all 9,216 bins. For each chip size, mean counts of two replicate experiments were used to generate a heat map after normalization and log-transformation of the values (detailed in Methods).

The resulting plots reproduced the intended image of the rose (Figure 2, a and b), confirming accurate, highly parallel quantitation of complex synthetic RNA mixtures across a large number of samples. Consistent rendering of subtle differences in pixel shades demonstrated the assay's ability to discriminate relatively small variations in RNA quantity. The image generated with the lower capacity chip, like a photograph taken in low light, appeared more grainy but still exhibited a strong quantitative signal above noise. To evaluate the concordance between the amount of synthetic RNA added to a sample and its measured level, a comparison was performed of the fold-change of known and measured values relative to the mean for each RNA (Figure 2, c and d). Regression analysis yielded a slope and R² of 0.82 and 0.88 for 318 chip data, and 0.89 and 0.84 for 314 chip data, respectively. To then explore the effect of sequence depth on accuracy of measurement, the Pearson correlation coefficient was calculated between known and measured values while varying the total number of reads used (Figure 2e). This analysis showed only modest improvement in accuracy above approximately 500,000 total reads (corresponding to an average of only ∼54 reads per target/sample bin).

Figure 2 provides data supporting the accuracy of multiplexed RNA quantitation using the disclosed RNA profiling method. Panel A shows a heat map that displays a 9,216 pixel image of a rose based on measurements of 96 synthetic miRNAs and control RNAs mixed in specified proportions within 96 samples. Mean values of two experimental replicates are shown, each sequenced using a high-capacity 318 chip. Normalization is described in Methods. RNAs are in the same order as listed in Table 2. Panel B shows a similar heat map generated from two replicates using lower-capacity 314 chips. Panel C and D show concordance between the amount of synthetic RNA added to a sample and its measured level using 318 (panel C) or 314 chips (panel D). Fold-change is relative to the mean for each RNA. Panel E shows the effect of sequence depth on quantitative accuracy, defined by the Pearson correlation coefficient between known and measured RNA levels.

Multiplexed analysis of miRNAs in human tissues:Moving beyond artificial RNA samples, the performance of the assay was then tested on miRNAs derived from 20 normal human tissues. These samples were chosen based on availability of independently published qRT-PCR data against which measurements made using the disclosed RNA profiling method could be validated. Input consisted of 50 ng total RNA from each sample, and resulting read counts were subjected to global mean normalization, mean-centering, and autoscaling as previously described. Results are presented using modified heat maps in which the measurement made using the disclosed RNA profiling method is compared to the published value in the two halves of a diagonally split pixel (Figure 3a). Concordance between the datasets is evident in the scarcity of pixels having combinations of red and green halves. Analysis of Pearson correlation coefficients showed good agreement between RNA levels measured by disclosed RNA profiling method vs. qRT-PCR for a given tissue (Figure 3b). Correlations were also observed between related tissues (e.g. colon and small bowel or ovary and testicle). Comparisons to data from other platforms are presented in Figure 5. Measurements showed good consistency across the various tested platforms. A similar range of pairwise correlation coefficients was found when comparing the disclosed RNA profiling method to four orthogonal platforms as was found when comparing those orthogonal platforms to each other (Figure 5). It was also possible to determine absolute rather than relative concentrations by co-amplifying a sample containing known, equimolar amounts of all synthetic miRNAs as a quantitative reference standard (Figure 6). Based on this analysis, the assay was found to be able to measure miRNAs over a concentration range of at least 4-5 orders of magnitude.

Figure 3 shows data to validate the quantitative performance of the method with RNA from human tissues and reference samples. Panel A shows a heat map with divided pixels compares levels of 90 miRNAs measured as three technical replicates from 20 normal human tissues to published qRT-PCR measurements. Both data sets were standardized. Panel B shows a heat map of correlation coefficients of miRNA levels measured by the disclosed RNA profiling method vs. qRT-PCR from the same tissue (diagonal) or between different tissues (off-diagonal). Color scheme and order of tissues is the same as in a. Panel C shows pair-wise correlation of fold-difference of mRNA levels in MAQC reference samples as measured by the disclosed RNA profiling method (in quadruplicate) vs. three other platforms. 30 mRNAs common to all platforms were tested. Linear regression fits are shown. UHR = Universal Human Reference RNA; HBR = Human Brain Reference RNA. Panel D shows a box plot of relative accuracy (for the same 30 genes), defined as the % difference between measured levels of an mRNA in MAQC samples C and D compared to levels predicted based on measurements of samples A and B¹². Predicted levels were calculated as C' = 0.75A + 0.25B and D' = 0.25A + 0.75B. Horizontal line = median; box = interquartile range; whiskers = 10^{th} - 90^{th} percentile; dots = outliers.

Figure 5 provides data to compare several miRNA profiling platforms. Panel A shows that miRNA levels were measured in total RNA derived from normal human brain and liver using five orthogonal platforms, including the disclosed RNA profiling method. Data from the other four platforms were reported by independent laboratories. Values for log₂ (fold-difference) between miRNA levels in brain vs. liver as measured by different platforms are displayed in the heat map. Analysis was restricted to miRNAs that were common to all assay panels. An miRNA was excluded if its level was below the limit of detection in both samples for a given platform. Reported values are means of 3 technical replicates. Panel B shows pairwise correlation of fold-difference of miRNA levels between brain and liver as measured by the disclosed RNA profiling method vs. four orthogonal platforms. Panel C shows pairwise R² values for all platform combinations. External data sets were downloaded from mirnabodymap.org for Taqman qRT-PCR⁸ and from Gene Expression Omnibus for Illumina RNA-Seq (GSE49816), for Affymetrix array (GSE49661) and for NanoString (GSE49600). D.M. = Disclosed RNA profiling Method.

Figure 6 shows absolute quantitation of miRNAs in human tissues. By normalizing relative to a co-amplified quantitative reference standard sample containing ∼15,000 synthetic copies of each miRNA species, the absolute miRNA concentration could be estimated. Total RNA input was 50 ng per tissue sample. Values were derived from the mean of 3 replicate RT reactions, which were pooled for single-plex PCR. Hsa-mir-381 was excluded from the analysis because it amplified poorly. A shade scale indicates miRNA abundance, and an embedded histogram indicates the frequency distribution of these values on the same scale.

### Measurement of mRNAs in reference standards:

Adapting the method to quantify mRNAs was straightforward. The absence of target length constraints allowed RT to be performed at higher temperature using longer gene-specific primers (Table 5). Other minor modifications are detailed in the Methods section. To provide a validation benchmark, 30 genes were targeted whose expression was measured at consistent levels using three distinct quantitative platforms as part of the MicroArray Quality Control (MAQC) consortium project. Assays were performed in quadruplicate using 100 ng of total RNA from the four MAQC reference samples, which consisted of (A) Stratagene Universal Human RNA, (B) Ambion Human Brain RNA, and mixtures of these two samples at ratios of (C) 3:1 and (D) 1:3. Expression levels were normalized relative to mean levels of *ACTB* and *POLR2A.* To evaluate the correlation of fold-change measurements between the disclosed RNA profiling method and each of the three quantitative MAQC platforms, pairwise regression analyses were performed of fold differences between samples A and B (Figure 3c). For the common set of 30 genes, the respective slope and R² for the disclosed RNA profiling method versus TaqMan were 1.02 and 0.89; versus StaRT-PCR, 0.97 and 0.91; and versus QuantiGene, 0.92 and 0.88. Since samples C and D are composed of defined ratios of samples A and B, the relative accuracy (RA) of the assay could be assessed by comparing observed expression levels for C and D to predicted levels calculated from measurements of A and B. The RA score for a gene was defined as Δ*C* = (*C-C'*)/*C'* and Δ*D* = (*D-D'*/*D',* where *C* and *D* are measured levels of the gene, and *C'* and *D'* are predicted levels. Box plots of RA scores for the panel of 30 mRNAs show that values are distributed closely around zero (Figure 3d).

### High-throughput assessment of radiation exposure:

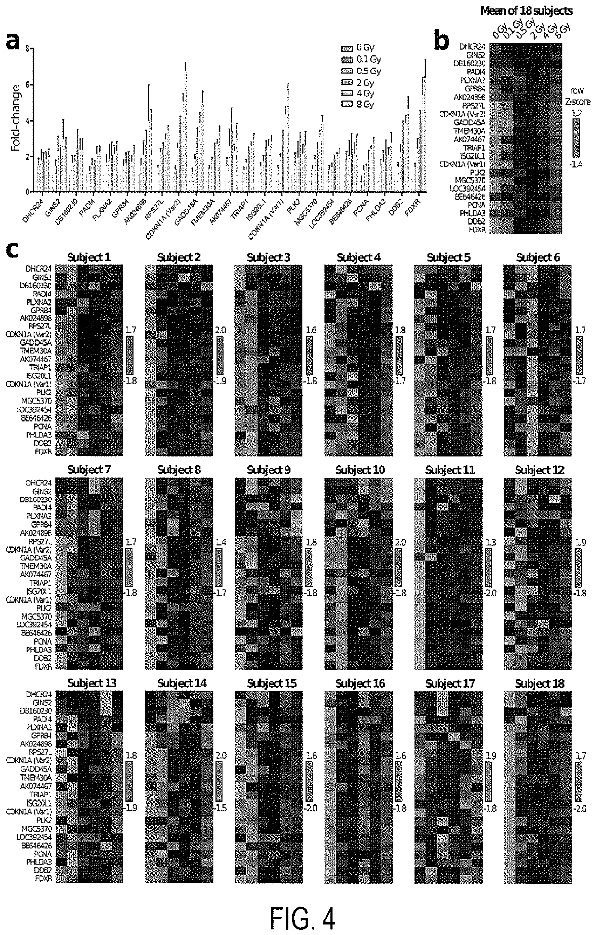

Finally, to evaluate the utility of the disclosed RNA profiling method on clinical samples, radiation-induced gene expression changes were measured in human blood. This has been proposed as an approach to estimate the dose of total-body radiation exposure following a large-scale nuclear disaster; but optimization of sample throughput would be needed to enable triage of thousands of potentially exposed individuals. To explore the feasibility of using the disclosed RNA profiling method for this purpose, an assay was developed to quantify expression changes in a panel of 23 previously identified radiation-responsive transcripts. This assay was used to perform parallel analysis of 108 *ex vivo* irradiated blood samples from 18 individuals (six dose levels each). Input consisted of 400 ng of total RNA derived from peripheral blood mononuclear cells that were isolated 24 hours after irradiation of whole blood. As expected, a dose-dependent increase in expression was observed for all genes in the panel when the signal was averaged across all 18 individuals (Figure 4, a and b). The expression pattern for each individual also exhibited good consistency with this overall trend (Figure 4c).

Figure 4 shows results of high-throughput measurements of radiation-induced gene expression changes in human blood. Expression level changes in a panel of previously identified radiation-responsive genes were measured 24 hours after *ex vivo* irradiation of 108 blood samples from 18 individuals. All samples were processed and measured in parallel in two replicate RNA profiling experiments. Panel A shows mean fold-induction of gene expression at various radiation doses, relative to a mock-irradiated sample. Error bars indicate SEM. Panel B shows a heat map of standardized gene expression values at different doses averaged over 18 subjects, each of whose values are shown separately in panel C. Mean centering and autoscaling were performed separately across samples from each subject.

### Example 2

This example describes methods and systems that are directed to sensitive and efficient measurement of low-abundance variant sequences within complex nucleic acid mixtures. We refer to the method described in this example as "lineage-traced PCR" (LT-PCR). The goal of LT-PCR is to assign molecule-specific tags (called molecular lineage tags or MLTs) to template DNA molecules during the first few cycles of PCR to make it possible to distinguish true template-derived mutations from sequencer or PCR errors. This example describes analysis of DNA from blood samples obtained from patients with cancer, but the method can also be more generally applied to samples from other sources such as tumor tissue, cells, urine, etc. The method can be applied to single-stranded or double-stranded DNA templates and also to complementary DNA (cDNA) generated by reverse-transcription of RNA.

### Materials and Methods:

### Collection and processing of patient plasma samples

Blood was collected by venipuncture into a vacuum tube containing potassium-EDTA. Various tube sizes were used, typically between 3 mL and 10 mL. Blood was inverted in the tube several times at the time of collection to ensure even mixing of the K₂-EDTA. Samples were stored temporarily and transported at room temperature (20-25° C) prior to separation of plasma. Plasma was separated and frozen as soon as possible after blood collection, preferably within three or four hours. The collection tubes were centrifuged at 1000 x g for 10 minutes in a clinical centrifuge with a swinging bucket rotor with slow acceleration and deceleration (brake off). Plasma was removed from the red blood cells and buffy coat using a 1 mL pipette, being careful not to disturb the cells at the bottom of the tube (to avoid aspirating white blood cells which would lead to increased background wild-type DNA levels). The plasma was dispensed into 1.5 mL cryovials in 0.5 to 1 mL aliquots. The plasma was then frozen at -80° C until needed for further processing.

### Extraction and purification of DNA from plasma

Plasma was removed from the -80° C freezer and was thawed at room temperature for 15 to 30 minutes before proceeding with DNA extraction. Thawed plasma was then centrifuged at 6800 x g for 3 minutes to remove any cryoprecipitate. The supernatant was transferred to a fresh tube for further processing.
The QiaAmp® MinElute® Virus Vacuum Kit (Qiagen) was used for extraction of DNA from plasma volumes up to 1 mL (elution volume as low as 20 µL). For larger volumes of plasma up to 5 mL, the QiaAmp® Circulating Nucleic Acid Kit was used for DNA purification (elution volume as low as 20 µL). All kits were used according to the manufacturer's instructions, generally eluting the DNA into the lowest recommended volume (preferably 20 µL). To process 1 mL of plasma using the QiaAmp® MinElute® Virus Vacuum Kit, 5 micrograms of carrier RNA (cRNA; Qiagen) were added per mL, and the user-developed protocol found on the Qiagen website was followed.

### Synthesis of universal primers and MLT-containing gene-specific primers having blocked 3'-ends:

Oligonucleotide primers were designed to target specific mutation-prone regions of genomic DNA for amplification via PCR. Primers were synthesized on an automated DNA oligonucleotide synthesizer (Dr. Oligo 192) using standard phosphoramidite chemistry in the 3' to 5' direction at 200 nanomole scale on Universal Polystyrene Support III (Glen Research). The design of the primers is schematized in Figures 7 and 8. Gene-specific primers have gene-specific sequences at their 3'-ends, they contain seven degenerate positions comprising the MLT, and they contain a portion of the universal primer sequence. Universal primers contained LNA modifications in order to raise their melting temperature. Primer sequences are listed in Table 10, below. Primers were either gel purified or cartridge purified. To verify that the method is able to simultaneously analyze multiple targets, primers were designed to target eight genomic regions that are often mutated in cancer: 1 region of *KRAS,* 1 region of *BRAF,* 1 region of *PPP2R1A,* two regions of *PIK3CA,* and three regions of *EGFR.* Although in this example, eight genomic regions were targeted in this example, the method can readily be expanded to include tens or hundreds or possibly thousands of target amplicons. **Table 10.** List of primers used in Lineage-Traced PCR and in compartmentalized PCR experiments

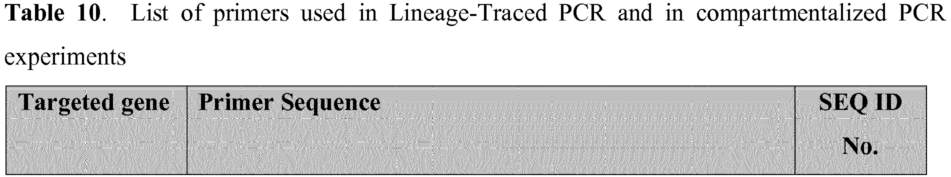

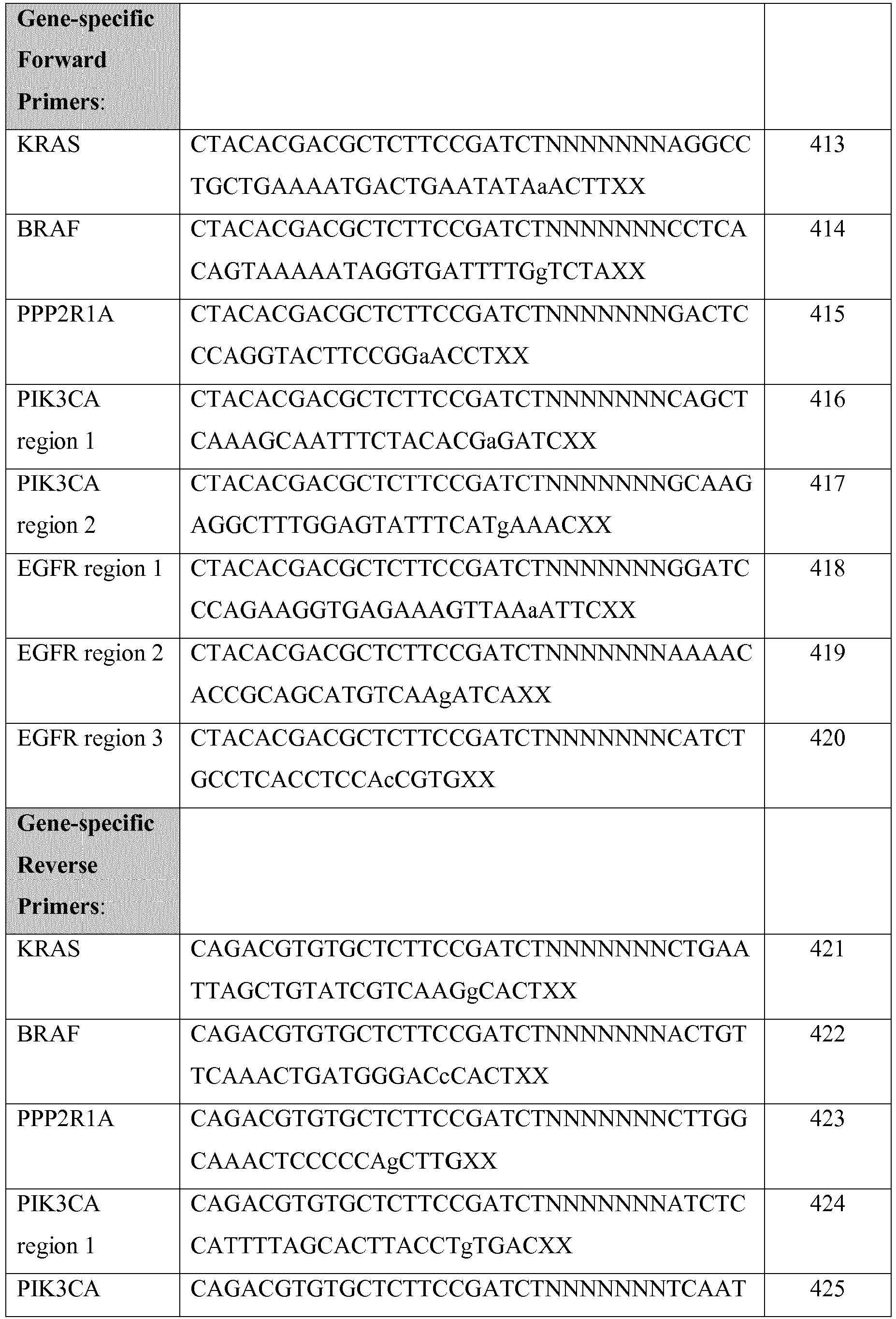

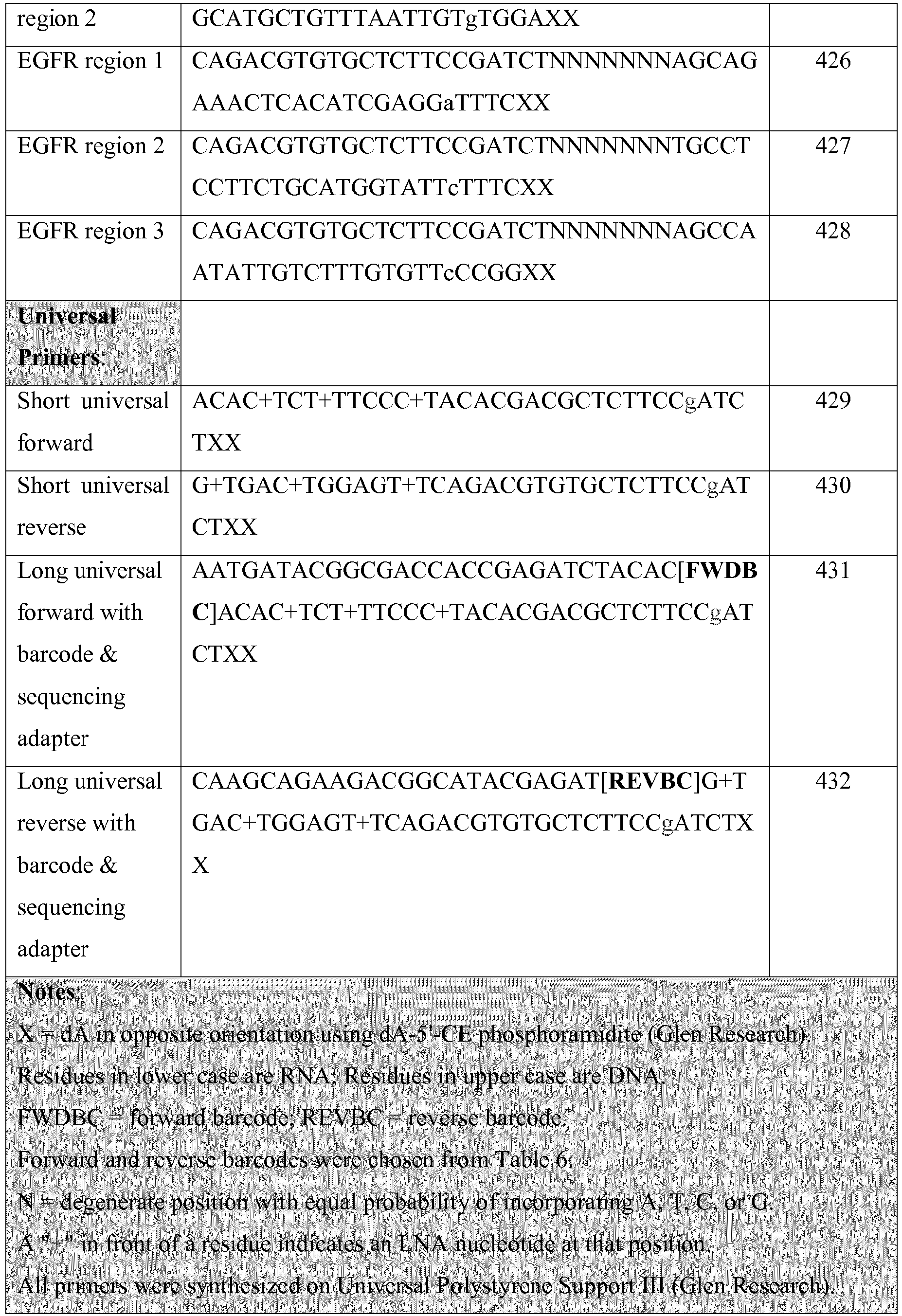

### Lineage-traced PCR tagging and amplification:

A modified polymerase chain reaction (PCR) was performed in a single reaction tube for each DNA template sample using the conditions outlined below:

### Lineage-traced PCR setup (20 µL reaction):

| | |
|---|---|
| Purified template DNA (may contain co-eluted carrier RNA [cRNA]) | 10 µL (or less) |
| 5 x concentrated Phusion HF Buffer (Thermo) | 4 µL |
| Mix of 16 gene-specific primers (stock has 200 nM each) | 2 µL |
| Mix of Universal Forward and Reverse primers with sample-specific barcode and sequencing adapter (stock has 5 µM each) | 2 µL |
| Mix of 4 dNTPs (stock 10 mM each) | 0.4 µL |
| Phusion Hot Start II DNA Polymerase (Thermo) (2 U/µL stock) | 0.2 µL |
| RNAse H2 (Integrated DNA Technologies) (20mU/µL stock) | 1 µL |
| Water | (to make final volume of 20 uL) |

For some reactions, the shorter universal primers (without a barcode and sequencing adapter [Table 10]) were added at a final concentration of 200 nM each, in addition to the longer universal primers. Inclusion of shorter universal primers with faster hybridization kinetics was intended to promote more efficient initial amplification of MLT-labeled copies.

### Temperature cycling conditions:

a. 98° C for 30 sec
b. 98° C for 10 sec
c. 70° C slowly decreased to 60° C at rate of 1° C per 10 sec
d. 60° C for 1 min
e. 72° C for 30 sec
f. repeat steps b-e for 2 more cycles (total 3 cycles)
g. 98° C for 10 sec
h. 72° C for 60 sec
i. repeat steps g-h for 34 more cycles (total 35 cycles)
g. hold at 4° C

Upon completion of thermal cycling, 2 µL of 100 mM EDTA-containing buffer was added to each reaction volume to inactivate polymerase activity. Approximately 10 µL of the amplification products from each sample were then pooled into a single tube for subsequent purification of the amplified DNA.

### Preparation of DNA for next-generation sequencing:

The pooled PCR reaction products were purified on a 2% agarose gel with ethidium bromide and 1x TBE buffer. Since all PCR products were of a similar final length, the pooled products appeared on the gel as a somewhat diffuse band. This diffuse band was excised from the gel using a fresh scalpel blade, ensuring that the gel was cut a few millimeters above and below the visible band to include any low-intensity bands that may have run faster or slower and were not well-visualized. Using a QIAquick® Gel Extraction kit (Qiagen) according to the manufacturer's instructions, the DNA was isolated from the gel slice. The DNA was eluted into 50 µL of elution buffer, EB.

### Next-generation sequencing

To prepare the sample for loading onto an Illumina HiSeq flow cell, the concentration of the DNA was measured using an Agilent Bioanalyzer®, and the DNA was diluted to the concentration recommended by Illumina. Cluster formation was carried out on the flow cell according to Illumina's protocol. The sample was loaded onto a single lane of a flow cell. The sequencing was performed on a HiSeq® 2000 instrument in multiplexed paired-end mode, with a read length of 75 base pairs in each direction. In additional experiments, sequencing has also been performed on an Illumina MiSeq instrument, and paired-end read lengths of 100, 150, 200, or 250 base pairs in each direction have also been utilized. Two index reads were also performed, and the length of the index read was increased from the standard seven cycles up to nine cycles so that our longer barcode (index) sequences could be appropriately read.

### Example 3

Similar to Example 2, Example 3 describes methods and systems that are directed to sensitive and efficient measurement of low-abundance variant sequences within complex nucleic acid mixtures. This example incorporates "lineage-traced PCR" (LT-PCR) as described in Example 2, but uses a compartmentalization strategy to further improve upon analytical sensitivity. The PCR was divided into many small reaction volumes such that there was a very low probability of having more than 1 copy of a particular targeted DNA fragment in a given reaction volume. A tagging strategy was used which made it possible to confirm that amplified copies of a variant sequence arose from both stands of a double-stranded template DNA fragment within a given reaction compartment. This example describes analysis of DNA from blood samples obtained from patients with cancer, but the method can also be more generally applied to samples from other sources such as tumor tissue, cells, urine, etc. The method can also be applied to single-stranded DNA templates and also to complementary DNA (cDNA) generated by reverse-transcription of RNA, but with a compromise in the robustness of error suppression.

### Materials and Methods

### Collection and processing of patient plasma samples

Blood was collected using the same methods as described in Example 2.

### Extraction and purification of DNA from plasma

DNA was extracted from patient plasma samples using the same methods as described in Example 2.

### Synthesis of universal primers and MLT-containing gene-specific primers having blocked 3'-ends

The same primers synthesized in Example 2 (Table 10) were used in this example, with the exception of the long forward universal primer (which contains a barcode and sequencing adapter). Primer synthesis was carried out using the same methods as described in Example 2.

### Split-and-pool synthesis of oligonucleotides containing bead-specific barcodes on magnetic beads

Magnetic micro-beads were used to deliver barcoded forward universal primers to different PCR micro-compartments (such as droplets or micro-wells). Each bead was designed to have many primer copies all having the same bead-specific barcode (BSBC). The sequence of the desired forward universal primer sequence is as follows:
5'-Biotin- 
AATGATACGGCGACCACCGAGATCTACAC[BSBC]ACACTCTTTCCCTACACGACG
CTCTTCC-3' (SEQ ID No: 433)
 To create millions of magnetic micro-beads having ∼1 million bead-specific barcodes, oligonucleotide synthesis was performed directly on the surface of the beads using a split-and-pool approach to generate the barcode sequence. Surface-activated super-paramagnetic 2.8 µm beads having amine modifications (Dynabeads M-270 Amine [Thermo Scientific]) were used as solid supports for oligonucleotide synthesis. For each batch of synthesis, 50 µL of bead slurry was used as provided by the manufacturer (∼100 million beads). Because the beads were too small to be retained in the synthesis column by a frit, a donut-shaped neodymium magnet was placed around the column to hold the magnetic beads in place on the sides of the column. A spacer 9 phosphoramidite (Glen Research) directly reacted with the amine-modified beads to create a phosphoramidate bond, which would not be cleaved during standard deprotection in ammonium hydroxide/methylamine (AMA). Additional phosphoramidites were linked to this spacer to grow the desired oligonucleotide chain. The synthesized oligonucleotides remained attached to the beads upon completion of synthesis. The following sequence was synthesized on the surface of the beads:
5'-Sacer 9 - TTTTTTTTTT - spacer C3 - 
GGAAGAGCGTCGTGTAGGGAAAGAGTGT[BSBC]GTGTAGATCTCGGTGGTCGCC
GTATCATT-3' (SEQ ID No: 434)
 To synthesize the oligonucleotide in the 5' to 3' direction, 5'-CE phosphoramidites were used (Glen Research). The oligonucleotide sequence contained 10 dT residues to introduce additional space from the surface of the bead. The bead-specific barcode (BSBC) consisted of 10 residues that were synthesized using split-and-pool synthesis. For phosphoramidite coupling at each of these 10 positions, the synthesis was paused and the magnetic beads were pooled and then split into four columns. The four different columns received the 4 different phosphoramidites (5'-dA, 5'-dT, 5'-dC, and 5'-dG). Synthesis was paused between each of the 10 coupling cycles, to allow the beads to be pooled and equally redistributed to four columns. After synthesis was complete, the bead-bound oligonucleotides were deprotected in AMA at 65° C for 10 minutes. The beads were then washed with deionized water and then re-suspended in 10 mM Tris pH 7.6 buffer.

To synthesize heat-releasable complementary barcoded primers on the surface of the micro-beads, the following primer was annealed to the bead-bound oligonucleotide, and was extended using Klenow Fragment (Exo-) (New England Biolabs).
5'-Biotin-AATGATACGGCGACCACCGAGATC-3' (SEQ ID No: 435)
The beads were re-suspended in 50 µL of NEB buffer 2 (1x concentration) supplemented with 0.2 mM dNTPs. The primer extension reaction was carried out according to the manufacturer's directions, incubating the reaction at 37° C for 30 minutes after adding Klenow polymerase. Beads were then washed and resuspended in buffer containing 50 mM NaCl and 10 mM Tris pH 7.6.

### Bead-free method for delivering clonally tagged primers to compartments.

In some experiments, instead of using beads, an alternative approach was used to introduce compartment specific tags to the PCR products within the compartments. Like with bead-based delivery, the goal was to deliver the following primer sequence to different compartments:
5'-Biotin- 
AATGATACGGCGACCACCGAGATCTACAC[CSBC]ACACTCTTTCCCTACACGACG
CTCTTCC-3' (SEQ ID No: 436)
 In a given compartment, multiple copies of this primer were introduced, with the clonal copies containing one or a few compartment-specific barcodes (CSBCs). To produce such primers, very dilute template DNA was added to the PCR cocktail prior to compartmentalization at a concentration that would allow an average of ∼2 to ∼3 amplifiable copies (molecules) to be distributed into each compartment (according to a Poisson distribution). The template DNA consisted of the following sequence:
DegenTemplate:
5'- 
AATGATACGGCGACCACCGAGATCTACACNNNNNNNNNNNNACACTCTTTCCCTAC
ACGACGCTCTTCC-3'  (SEQ ID No: 437)
 The following primers were also added to the cocktail:
Bio-ShortFWD:
   5'-Biotin-AA+TG+AT+ACGGCGACCACCGAGaTCTAXX-3' (SEQ ID No: 438) (Added in 100 nM final concentration)
ShortREV:
   5'-GGA+AGAGCG+TCG+TGTAGGGAAaGAGTXX-3' (SEQ ID No. 439) (Added in 20 nM final concentration)
X = dA in opposite orientation using dA-5'-CE phosphoramidite (Glen Research).
Residues in lower case are RNA; Residues in upper case are DNA.
N = degenerate position with equal probability of incorporating A, T, C, or G.
A "+" in front of a residue indicates an LNA nucleotide at that position.

As the micro-compartments were subjected to thermal cycling, the few tagged template molecules were clonally amplified, creating many copies of the desired primers containing compartment-specific tags. Because the biotinylated short forward primer was added in 5'-fold excess compared to the short reverse primer, more copies of the forward strand were made than of the reverse strand (via asymmetric PCR). Thus, the excess copies of the forward strand were then able to be further extended by hybridizing to co-amplified gene-specific PCR products in the same compartment. In this way, the gene-specific PCR products in a compartment were labeled with compartment-specific tags. This approach is schematized in Figure 12.

### PCR cocktail

The PCR cocktail used in this example depended on whether micro-beads were used to deliver compartment-specific primers or whether a bead-free approach was used.

For the bead-based approach, the following PCR cocktail was used:

| | |
|---|---|
| Purified template DNA (may contain co-eluted carrier RNA [cRNA]) | 10 µL (or less) |
| 5 x concentrated Phusion HF Buffer (Thermo) | 4 µL |
| Mix of 16 gene-specific primers (stock has 200 nM each) | 2 µL |
| Short Universal Forward and Reverse primers (Stock 10 µM each) | 1 µL |
| Long Universal Reverse primer with sample-specific barcode and sequencing adapter (10 µM stock) | 1 µL |
| Mix of 4 dNTPs (stock 10 mM each) | 0.4 µL |
| Phusion Hot Start II DNA Polymerase (Thermo) (2 U/µL stock) | 0.2 µL |
| RNAse H2 (Integrated DNA Technologies) (20mU/µL stock) | 1 µL |
| Water | (to make final volume of 20 uL) |

(Primer sequences are listed in Table 10)

Beads carrying tagged primers were added to the cocktail just prior to compartmentalization, and were mixed well to promote even distribution of the beads into the compartments. The number of beads was adjusted so that an average of ∼2 to ∼3 beads would be distributed into a micro-compartment.

When the bead-free approach was used to introduce clonal primers containing compartment-specific tags, the following PCR cocktail was used:

| | |
|---|---|
| Purified template DNA (may contain co-eluted carrier RNA [cRNA]) | 8 µL (or less) |
| 5 x concentrated Phusion HF Buffer (Thermo) | 4 µL |
| Mix of 16 gene-specific primers (stock has 200 nM each) | 2 µL |
| Mix of Short Universal Forward (Stock 5 µM) and Short Universal Reverse primers (Stock 10 µM) | 1 µL |
| Long Universal Reverse primer with sample-specific barcode and sequencing adapter (10 µM stock) | 1 µL |
| DegenTemplate (stock concentration adjusted as described below) | 1 µL |
| Mix of Bio-ShortFWD (1 µM stock) and Short REV (0.2 µM stock) | 1 µL |
| Mix of 4 dNTPs (stock 10 mM each) | 0.4 µL |
| Phusion Hot Start II DNA Polymerase (Thermo) (2 U/µL stock) | 0.2 µL |
| RNAse H2 (Integrated DNA Technologies) (20mU/µL stock) | 1 µL |
| Water | (to make final volume of 20 uL) |

The concentration of the stock solution of the "DegenTemplate" primer was adjusted so that an average of ∼2 to ∼3 amplifiable molecules would be distributed into each compartment. Digital PCR experiments were conducted using serial dilutions of this template to accurately determine the concentration of amplifiable molecules.

### Microfluidic compartmentalization of PCR

Two different approaches have been used to compartmentalize the PCR cocktail into microscopic reaction volumes prior to thermal cycling. One approach was to produce microfluidic droplets of aqueous PCR cocktail (optionally containing micro-beads) in oil. A second approach was to divide the PCR cocktail (optionally containing micro-beads) into micro-wells on a microfluidic device. In both approaches, approximately 20,000 separate microscopic reaction volumes of approximately 1 nanoliter each were created from a 20 microliter PCR cocktail. The total number and size of compartments could be adjusted in future experiments depending on the number of genome equivalents being analyzed. The compartmentalization scheme used in this example was based on an estimate of approximately 8-10 ng of genomic template DNA (∼3000 genome equivalents).

To compartmentalize the PCR cocktail into aqueous droplets in oil, a BioRad QX100 droplet generator was used with some modifications to the manufacturer's instructions. One modification was that the above PCR cocktail (with or without microbeads) was used instead of the manufacturer's recommended PCR super mix. Droplet Generation Oil for EvaGreen was used. Thermal cycling was carried out in 0.2 mL thin-walled PCR tubes.

To compartmentalize the PCR cocktail into micro-wells, we used a custom microfabricated clear slide onto which polydimethylsiloxane (PDMS) had been patterned to create 20,000 microwells, each holding ∼1 nL volume. The PDMS surface had been treated to make it hydrophilic to encourage even distribution of the PCR cocktail into the micro-wells. A coverslip was added to sandwich the PDMS pattern, thus sealing the micro-wells for thermal cycling.

### Thermal cycling

A thermal cycling protocol was used that was similar to the protocol used in example 2, except that the final two cycles had a lower annealing temperature to promote hybridization and extension of biotin-labeled primers containing compartment-specific tags.

### Temperature cycling conditions:

a. 98° C for 30 sec
b. 98° C for 10 sec
c. 70° C slowly decreased to 60° C at rate of 1° C per 10 sec
d. 60° C for 1 min
e. 72° C for 30 sec
f. repeat steps b-e for 2 more cycles (total 3 cycles)
g. 98° C for 10 sec
h. 72° C for 60 sec
i. repeat steps g-h for 34 more cycles (total 35 cycles)
j. 98°C for 10 sec
k. 60° C for 60 sec
l. repeat steps i-k for 1 more cycle (total 2 cycles)
m. hold at 4° C

### Combining tagged products from all compartments

Upon completion of thermal cycling, compartmentalized reaction volumes were combined and EDTA-containing buffer was added to the combined volume (∼10 mM final concentration) to inactivate polymerase activity. To coalesce droplets in oil, chloroform was added and the emulsion was agitated on a vortexer and then centrifuged at high speed according to Bio-Rad's recommended protocol. To combine the PCR products from micro-wells, the cover slip was removed and the micro-wells were washed with ∼200 µL of EDTA-containing buffer. If magnetic beads had been added to the cocktail, these were removed from the solution using a magnet.

### Preparation of DNA for next-generation sequencing:

Pooled PCR reaction products were purified on a 2% agarose gel with ethidium bromide and 1x TBE buffer. A band of the expected size (based on size makers run in an adjacent lane) was excised from the gel using a fresh scalpel blade. Using a QIAquick® Gel Extraction kit (Qiagen) according to the manufacturer's instructions, the DNA was isolated from the gel slice. The DNA was eluted into 50 µL of elution buffer, EB (Qiagen).

In some experiments, high-capacity streptavidin-agarose resin slurry (5 µL) (Thermo Scientific) was added to each reaction volume to capture biotin-labeled reaction products. The beads were then washed in 10 mM Tris pH 7.6, and then the DNA strands complementary to the biotinylated strands were eluted from the bead surface by heat-denaturation in 50 µL of elution buffer EB (Qiagen).

### Next-generation sequencing

To prepare the sample for loading onto an Illumina HiSeq flow cell, the concentration of the DNA was measured using an Agilent Bioanalyzer®, and the DNA was diluted to the concentration recommended by Illumina. Sequencing was performed as described in Example 2.

### Outline of algorithm for sequence analysis

Computational analysis was performed on the resulting sequence data to identify and quantify mutant double-stranded DNA fragments that produced matching mutant sequences from both strands. The underlying logic used for this analysis is described in the "Methods" section.

The present invention is defined by the enclosed claims. It is appreciated that the previous description of the disclosed embodiments is provided to enable any person skilled in the art to make or use the present disclosure. Thus, the present disclosure is not intended to be limited to the embodiments shown herein but is to be accorded the widest scope consistent with the claims.

## Claims

1. A method of identifying sequences that are derived from paired strands of a double-stranded nucleic acid fragment, the method comprising:
dissolving a plurality of double-stranded, biologically-derived nucleic acid fragments into an aqueous solution;
dissolving into the same aqueous solution a plurality of synthetic dilute template oligonucleotide (DTO) molecules, wherein DTO molecules comprise a degenerate tag sequence incorporated within a primer sequence;
distributing the solution into a plurality of compartments, wherein a compartment is unlikely to contain two or more double-stranded, biologically-derived nucleic acid fragments whose amplification products align to the same genomic reference sequence;
copying and amplifying both strands of the compartmentalized double-stranded, biologically-derived nucleic acid fragments by performing PCR, producing biologically-derived DNA copies;
simultaneously copying and amplifying the compartmentalized DTO molecules by PCR, producing within each compartment a plurality of clonal DTO copies comprising compartment-specific tags;
attaching one or more compartment-specific DNA sequence tags to the amplified biologically-derived DNA copies by PCR using the clonal DTO copies as primers, resulting in the same tag or set of tags being attached to copies of both strands of a double-stranded, biologically-derived nucleic acid fragment;
combining the compartments containing amplified tagged DNA copies; sequencing all or a subset of the amplified tagged DNA copies; and
identifying sequences that are derived from paired strands of a double-stranded, biologically-derived nucleic acid fragment based on sharing of a common compartment-specific DNA sequence tag or set of tags.

2. The method of claim 1, wherein comparison of sequences derived from paired strands of a double-stranded nucleic acid fragment enables reduction of errors in determining the sequence of the double-stranded nucleic acid fragment.

3. The method of claim 2, wherein error reduction in determining nucleic acid sequences is used to identify low-abundance sequence variants within a mixture of nucleic acid sequences.

4. The method of claim 1, wherein the double-stranded nucleic acid fragments are RNA.

5. The method of claim 1, wherein the double-stranded nucleic acid fragments are DNA.

6. The method of claim 1, wherein the double-stranded nucleic acid fragments are genomic DNA.

7. The method of claim 1, wherein the double-stranded nucleic acid fragments are genomic cell-free DNA derived from blood.

8. The method of claim 1, wherein the double-stranded nucleic acid fragments are genomic DNA derived from tumor tissue.

9. The method of claim 1, wherein the double-stranded nucleic acid fragments are genomic DNA derived from formalin-fixed, paraffin-embedded tumor tissue.

10. The method of claim 1, wherein the double-stranded nucleic acid fragments comprise genomic DNA to which synthetic adapter molecules have been ligated.

11. The method of claim 10, wherein a synthetic adapter molecule comprises at least one of the following components: DNA, RNA, modified bases, and oligonucleotide modifications not found in naturally occurring nucleic acids.

12. The method of claim 1, wherein the aqueous solution is compartmentalized into aqueous droplets within oil.

13. The method of claim 1, wherein the probability of finding contain two or more double-stranded nucleic acid fragments whose amplification products align to the same genomic reference sequence in the same compartment is ∼1/100..

14. The method of claim 1, wherein the compartmentalized double-stranded nucleic acid fragments are amplified by PCR using one or more primer pairs that target specific genomic sequences.

15. The method of claim 1, wherein the compartmentalized double-stranded nucleic acid fragments are amplified by PCR using one or more primer pairs that target ligated adapter sequences.

16. The method of claim 1, wherein a compartment-specific tag or compartment-specific set of tags can be used to distinguish DNA copies that are amplified within different compartments.

17. The method of claim 1, wherein compartments contain a mean of 2 to 3 compartment-specific tags per compartment.

18. The method of claim 1, wherein a subset of amplified, tagged DNA copies are selected for sequencing by target enrichment methods such as hybrid capture or in-solution capture.

## Patentansprüche

1. Verfahren zur Ermittlung von Sequenzen aus gepaarten Strängen eines Doppelstrang-Nukleinsäurefragments, wobei das Verfahren Folgendes umfasst:
Auflösen einer Vielzahl biologisch abgeleiteter Doppelstrang-Nukleinsäurefragmente in einer wässrigen Lösung;
Auflösen einer Vielzahl synthetischer Dilute-Template-Oligonukleotid(DTO-) -Moleküle in derselben wässrigen Lösung, wobei die DTO-Moleküle eine degenerierte Tag-Sequenz umfassen, die in einer Primer-Sequenz inkorporiert ist;
Verteilen der Lösung auf eine Vielzahl von Gefäßen, wobei ein Gefäß wahrscheinlich keine zwei oder mehr biologisch abgeleitete Doppelstrang-Nukleinsäurefragmente enthält, deren Amplifikationsprodukte auf dieselbe genomische Referenzfolge ausgerichtet sind;
Kopieren und Amplifizieren beider Stränge der aufgeteilten, biologisch abgeleiteten Doppelstrang-Nukleinsäurefragmente durch Ausführen einer PCR, wodurch biologisch abgeleitete DNA-Kopien erstellt werden;
gleichzeitiges Kopieren und Amplifizieren der aufgeteilten DTO-Moleküle durch die PCR, wodurch in jedem Gefäß eine Vielzahl klonaler DTO-Kopien mit den gefäßspezifischen Tags produziert werden;
Zuordnen einer oder mehrerer gefäßspezifischer DNA-Sequenz-Tags zu den amplifizierten, biologisch abgeleiteten DNA-Kopien durch die PCR unter Verwendung der klonalen DTO-Kopien als Primer, was zu demselben Tag oder demselben Tag-Paar führt, der/das den Kopien der beiden Stränge eines biologisch abgeleiteten Doppelstrang-Nukleinsäurefragments zugeordnet ist;
Kombinieren der Gefäße mit den amplifizierten, getaggten DNA-Kopien; Sequenzieren aller oder einer Teilmenge der amplifizierten, getaggten DNA-Kopien; und
Ermitteln von Sequenzen aus den gepaarten Strängen eines biologisch abgeleiteten Doppelstrang-Nukleinsäurefragments auf der Grundlage der gemeinsamen Nutzung eines üblichen gefäßspezifischen DNA-Sequenz-Tags oder eines Tag-Paares.

2. Verfahren nach Anspruch 1, wobei der Vergleich von Sequenzen aus den gepaarten Strängen eines biologisch abgeleiteten Doppelstrang-Nukleinsäurefragments die Fehlerreduzierung bei der Bestimmung der Sequenz des Doppelstrang-Nukleinsäurefragments ermöglicht.

3. Verfahren nach Anspruch 2, wobei die Fehlerreduzierung bei der Bestimmung der Nukleinsäuresequenzen verwendet wird, um geringe Mengen von Sequenzvarianten aus einer Mischung von Nukleinsäuresequenzen zu ermitteln.

4. Verfahren nach Anspruch 1, wobei die Doppelstrang-Nukleinsäurefragmente RNA sind.

5. Verfahren nach Anspruch 1, wobei die Doppelstrang-Nukleinsäurefragmente DNA sind.

6. Verfahren nach Anspruch 1, wobei die Doppelstrang-Nukleinsäurefragmente genomische DNA sind.

7. Verfahren nach Anspruch 1, wobei die Doppelstrang-Nukleinsäurefragmente genomische, zellfreie, aus Blut gewonnene DNA sind.

8. Verfahren nach Anspruch 1, wobei die Doppelstrang-Nukleinsäurefragmente genomische, aus Tumorgewebe gewonnene DNA sind.

9. Verfahren nach Anspruch 1, wobei die Doppelstrang-Nukleinsäurefragmente genomische, aus formalinfixiertem, paraffineingebettetem Tumorgewebe gewonnenen DNA sind.

10. Verfahren nach Anspruch 1, wobei die Doppelstrang-Nukleinsäurefragmente genomische DNA umfassen, auf die synthetische Adaptermoleküle ligiert wurden.

11. Verfahren nach Anspruch 10, wobei ein synthetisches Adaptermolekül mindestens eine der folgenden Komponenten umfasst: DNA, RNA, modifizierte Basen und Oligonukleotidmodifikationen, die nicht in natürlich vorkommenden Nukleinsäuren zu finden sind.

12. Verfahren nach Anspruch 1, wobei die wässrige Lösung in wässrige Tropfen in Öl aufgeteilt ist.

13. Verfahren nach Anspruch 1, wobei die Wahrscheinlichkeit, zwei oder mehr Doppelstrang-Nukleinsäurefragmente zu finden, deren Amplifikationsprodukte auf dieselbe genomische Referenzsequenz in demselben Gefäß ausgerichtet sind, ungefähr 1/100 beträgt.

14. Verfahren nach Anspruch 1, wobei die aufgeteilten Doppelstrang-Nukleinsäurefragmente unter Verwendung von einem oder mehreren Primerpaaren, die auf die spezifischen genomischen Sequenzen abzielen, durch die PCR amplifiziert sind.

15. Verfahren nach Anspruch 1, wobei die aufgeteilten Doppelstrang-Nukleinsäurefragmente unter Verwendung von einem oder mehreren Primerpaaren, die auf die ligierten Adaptersequenzen abzielen, durch die PCR amplifiziert sind.

16. Verfahren nach Anspruch 1, wobei ein gefäßspezifischer Tag oder ein gefäßspezifisches Tag-Paar verwendet werden kann, um DNA-Kopien zu unterscheiden, die in den verschiedenen Gefäßen amplifiziert sind.

17. Verfahren nach Anspruch 1, wobei die Gefäße einen Mittelwert von 2 bis 3 gefäßspezifischen Tags je Gefäß enthalten.

18. Verfahren nach Anspruch 1, wobei eine Teilmenge von amplifizierten, getaggten DNA-Kopien zum Sequenzieren von Target-Enrichment-Verfahren wie Hybrid Capture oder In-Solution-Capture ausgewählt sind.

## Revendications

1. Procédé d'identification de séquences qui sont dérivées de brins appariés d'un fragment d'acide nucléique double brin, le procédé comprenant les étapes consistant à :
dissoudre une pluralité de fragments d'acide nucléique dérivés biologiquement, double brin dans une solution aqueuse ;
dissoudre dans la même solution aqueuse une pluralité de molécules d'oligonucléotide de matrice diluée (DTO) synthétiques, les molécules DTO comprenant une séquence d'étiquette dégénérée incorporée dans une séquence d'amorce ;
distribuer la solution dans une pluralité de compartiments, un compartiment étant peu susceptible de contenir deux ou plus de deux fragments d'acide nucléique dérivés biologiquement double brin dont les produits d'amplification s'alignent à la même séquence génomique de référence ;
copier et amplifier les deux brins des fragments d'acide nucléique dérivés biologiquement, double brin compartimentalisés en effectuant une PCR, produisant des copies d'ADN dérivé biologiquement ;
simultanément copier et amplifier les molécules de DTO compartimentalisées par PCR, produisant dans chaque compartiment une pluralité de copies de DTO clonales comprenant des étiquettes spécifiques de compartiment ;
attacher une ou plusieurs étiquette(s) de séquence d'ADN spécifiques de compartiment aux copies d'ADN dérivées biologiquement amplifiées par PCR en utilisant les copies de DTO clonales comme amorces, résultant dans la même étiquette ou le même ensemble d'étiquettes étant attachées aux copies des deux brins d'un fragment d'acide nucléique dérivé biologiquement double brin ;
combiner les compartiments contenant les copies d'ADN étiqueté amplifiées ;
séquencer l'ensemble ou un sous-ensemble des copies d'ADN étiqueté amplifiées ; et
identifier les séquences qui sont dérivées de brins appariés d'un fragment d'acide nucléique dérivé biologiquement double brin, sur la base du partage d'une étiquette ou d'un ensemble d'étiquettes de séquence d'ADN spécifique de compartiment commun(e).

2. Procédé selon la revendication 1, selon lequel la comparaison de séquences dérivées des brins appariés d'un fragment d'acide nucléique double brin permet la réduction d'erreurs dans la détermination de la séquence du fragment d'acide nucléique double brin.

3. Procédé selon la revendication 2, selon lequel la réduction d'erreurs dans la détermination de séquences d'acide nucléique est utilisée pour identifier les variants de séquence de faible abondance dans un mélange de séquences d'acide nucléique.

4. Procédé selon la revendication 1, selon lequel les fragments d'acide nucléique double brin sont de l'ARN.

5. Procédé selon la revendication 1, selon lequel les fragments d'acide nucléique double brin sont de l'ADN.

6. Procédé selon la revendication 1, selon lequel les fragments d'acide nucléique double brin sont de l'ADN génomique.

7. Procédé selon la revendication 1, selon lequel les fragments d'acide nucléique double brin sont de l'ADN sans cellule génomique dérivé du sang.

8. Procédé selon la revendication 1, selon lequel les fragments d'acide nucléique double brin sont de l'ADN génomique dérivé d'un tissu tumoral.

9. Procédé selon la revendication 1, selon lequel les fragments d'acide nucléique double brin sont de l'ADN génomique dérivé d'un tissu tumoral inclus dans de la paraffine, fixé à la formaline.

10. Procédé selon la revendication 1, selon lequel les fragments d'acide nucléique double brin comprennent de l'ADN génomique auquel des molécules d'adaptateur synthétique ont été liguées.

11. Procédé selon la revendication 10, selon lequel une molécule d'adaptateur synthétique comprend au moins un des composants suivants : ADN, ARN, bases modifiées et modifications d'oligonucléotide non trouvées dans les acides nucléiques existant naturellement.

12. Procédé selon la revendication 1, selon lequel la solution aqueuse est compartimentalisée en gouttelettes aqueuses dans de l'huile.

13. Procédé selon la revendication 1, selon lequel la probabilité de trouver deux ou plus de deux fragments d'acide nucléique double brin dont les produits d'amplification s'alignent à la même séquence de référence génomique dans le même compartiment est -1/100.

14. Procédé selon la revendication 1, selon lequel les fragments d'acide nucléique double brin compartimentalisés sont amplifiés par PCR en utilisant une ou plusieurs paire(s) d'amorce qui cible(nt) des séquences génomiques spécifiques.

15. Procédé selon la revendication 1, selon lequel les fragments d'acide nucléique double brin compartimentalisés sont amplifiés par PCR en utilisant une ou plusieurs paire(s) d'amorce qui cible(nt) les séquences d'adaptateur liguées.

16. Procédé selon la revendication 1, selon lequel une étiquette spécifique de compartiment ou un ensemble d'étiquettes spécifiques de compartiment peut être utilisé(e) pour distinguer des copies d'ADN qui sont amplifiées dans différents compartiments.

17. Procédé selon la revendication 1, selon lequel les compartiments contiennent une moyenne de 2 à 3 étiquettes spécifiques de compartiment par compartiment.

18. Procédé selon la revendication 1, selon lequel un sous-ensemble de copies d'ADN étiqueté amplifiées est choisi pour le séquençage par des procédés d'enrichissement de cible tels que la capture d'hybride ou la capture en solution.
